(19) Europäisches Patentamt / European Patent Office / Office européen des brevets

(11) **EP 4 621 073 A1**

(12) **EUROPEAN PATENT APPLICATION**

(43) Date of publication:
**24.09.2025  Bulletin 2025/39**

(21) Application number: **24165583.6**

(22) Date of filing: **22.03.2024**

(51) International Patent Classification (IPC):
**C12Q 1/6886** (2018.01)

(52) Cooperative Patent Classification (CPC):
**C12Q 1/6886;** C12Q 2600/106; C12Q 2600/118;
C12Q 2600/158

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Istituto Europeo di Oncologia S.r.l.**
  **20121 Milano (IT)**
• **UNIVERSITA' DEGLI STUDI DI MILANO**
  **20122 Milano (IT)**
• **Università degli Studi di Torino**
  **10124 Torino (IT)**
• **Fondazione del Piemonte Per L'Oncologia**
  **10060 Candiolo (TO) (IT)**

(72) Inventors:
• **Di Fiore, Pier Paolo**
  **20121 c/o ISTITUTO EUROPEO DI ONCOLOGIA
  s.r.l.,
  Via Filodrammatici, 10 (IT)**

• **Confalonieri, Stefano**
  **20121 c/o ISTITUTO EUROPEO DI ONCOLOGIA
  s.r.l.,
  Via Filodrammatici, 10 (IT)**
• **Matoskova, Bronislava**
  **20121 c/o ISTITUTO EUROPEO DI ONCOLOGIA
  s.r.l.,
  Via Filodrammatici, 10 (IT)**
• **Lanzetti, Letizia**
  **10060 c/o Fondazione del Piemonte per
  l'Oncologia,
  Strada Provinciale 142, KM 3,95 (IT)**

(74) Representative: **Pace Napoleone, Maria et al
De Simone & Partners S.r.l.
Via Giulio Caccini, 1
00198 Roma (IT)**

(54) **METHOD FOR PROGNOSIS OF BREAST CANCER**

(57)    The present invention relates to a method of prognosis of a subject affected by breast cancer comprising determining the expression level of one or more genes in an isolated sample from said subject and to kits for performing said method.

**EP 4 621 073 A1**

**Description**

**FIELD OF THE INVENTION**

The present invention relates to the field of methods to predict the progression of a disease.

In particular, it is related to a method of prognosis of a subject affected by breast cancer, by determining the expression level of one or more genes in an isolated sample from said subject.

**BACKGROUND OF THE INVENTION**

Cancers meet their high anabolic and energetic demands by modulating their metabolism. Indeed, metabolic plasticity is a recognized hallmark of cancer that intersects cancer-intrinsic and extrinsic features, and contributes to tumor evolution [1]. The best characterized metabolic alteration in cancer is aerobic glycolysis, also known as the Warburg effect, a process characterized by the avid uptake of glucose by cancer cells and its conversion to lactate even in the presence of oxygen [2]. Despite having been discovered a century ago [3], the exact functional significance of the Warburg effect remains elusive. Traditionally interpreted as a strategy enacted by cancer cells to circumvent a deficit in energy production by the mitochondria [4], the Warburg effect is today thought to represent a way to increase glycolytic flux to heighten the availability of intermediates to feed anabolic pathways [2, 5]. *In vivo,* the Warburg effect can be indirectly studied using a variety of systems, the most common being 18F-fluorodeoxyglucose Positron Emission Tomography (FDG-PET). FDG-PET employs the uptake of the glucose-derivative tracer as a surrogate for enhanced glucose metabolism and is widely used in the clinics as it provides useful information in all phases of cancer management, including diagnosis, staging, re-staging after therapy and follow-up. FDG uptake is measured using a parameter called SUV (standardized uptake value), which corrects the uptake in a lesion by parameters indicative of the distribution volume of the tracer [6]. In the clinics, the most used parameter is the SUVmax which corresponds to the maximum SUV value measured in a given volume.

Breast cancer (BC) is the most frequently diagnosed cancer worldwide, accounting for ~12% of all cancer diagnosis and ~7% of cancer-related deaths yearly [7]. BCs are phenotypically and molecularly heterogenous, a feature that complicates the diagnostic itinerary and the stratification of patients for prognostic and therapeutic purposes [8, 9]. A widely used clinicopathological classification relies on the levels of expression of hormone receptors (HR), namely the estrogen receptor (ER) and the progesterone receptor (PGR), on the levels of the proliferation marker Ki67, and on the presence of gene amplification of the oncogene *HER2* (HUGO: *ERBB2*). BCs that are positive for HR, and negative for HER2 amplification (HR+, HER2-) are called Luminal BCs, which can be subclassified into Luminal A or B based on the levels of Ki67 expression. HER2 BCs display amplification of *HER2* and can be further subdivided into Luminal-HER2 (HR+, HER2+) and HER2 (HR-, HER2+). Finally, triple-negative BCs (TNBC) are negative for both HR and HER2 [10]. This classification system is clinically useful as it is prognostic for disease aggressiveness and clinical outcome, going in order from better to worse: Luminal A > Luminal B > Luminal-HER2 > HER2 > TNBC. More recently, multigene expression signatures have been developed that provide additional stratification tools which can influence therapeutic decision-making [11-15]. These signatures are proving advantageous especially in Luminal BCs which represent ~65% of all BC diagnoses. Patients with Luminal BCs have good overall prognosis, yet at least 20% of them will experience metastatic relapse within 10 years from surgery or even later [16].

Based on clinical utility as an imaging tool, PET/CT is useful for BC staging starting from clinical stage IIB, while it is not routinely used in early-stage BC due to the low sensitivity for lesions < 1.0 cm [17-20]. In principle, however, FDG-PET could be useful in all BC patients, as it provides information on the metabolic status of the tumor and could potentially inform prognosis and therapeutic decisions.

Therefore, there is still the need of a method of screening a subject suffering from breast cancer for predicting prognosis of the disease, in particular in the initial clinical stages, and in the Luminal subtype.

**SUMMARY OF THE INVENTION**

Inventors performed a transcriptomics analysis on a cohort of patients who underwent FDG-PET prior to any therapy, and derived a signature, the "PETsign", which was prognostic of outcome in three independent BC datasets originating from different institutes, different countries and using different technological platforms. PETsign was particularly effective in stratifying Luminal BCs and represented an *ab initio* signature being clearly detectable in *in situ* BCs. Furthermore, inventors found that genes of the PETsign are causal in the development of metabolic alterations connected with the Warburg effect in BCs. Thus, PETsign is a molecular surrogate for FDG-PET which can be derived from biological specimens without the need for additional invasive interventions. PETsign allows to gather information on the metabolic status of BCs, in the initial phases of the diagnostic/stratification process even in those patients in which FDG-PET is not indicated for imaging/diagnostic purposes.

The present invention therefore provides a genetic signature which can be easily and effectively used as a prognostic procedure for subjects affected by breast cancer.

It is an object of the invention an *in vitro* method of screening a subject suffering from breast cancer for predicting prognosis of the disease comprising at least the step of determining the expression level of at least one gene in an isolated biological sample from said subject, wherein said gene is selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4.

Said breast cancer can be selected from Luminal, such as Luminal A or Luminal B, Luminal-HER2, HER2 and triple-negative (TNBC) breast cancer, preferably it is Luminal or Luminal-HER2.

In an embodiment, said method comprises determining the expression level of at least 2, 3, 4, 5, 6, 7, 8, 9 or 10 genes from the above list.

In an embodiment, the expression level of all said genes or at least all said genes is determined. Expression level of further genes may also be determined.

In an embodiment, the method comprises determining the expression level of at least one gene selected from the group consisting of: CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, and CHI3L1, preferably of all said genes.

In another embodiment, the method comprises determining the expression level of at least one gene selected from the group consisting of: ADAMDEC1, ADM, AQP9, C5orf46, CDCA7, CDH3, CENPW, CP, CSF2RB, CXCL10, CXCL11, CXCL8, EGFR, EREG, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, and TMEM158, preferably of all said genes.

In another embodiment, the method comprises determining the expression level of at least one gene selected from the group consisting of: MCM10, SOX11, AQP9, CDCA7, S100A9, CXCL8, EGFR, PSAT1, ADM, CKB, CP, CXCL10, MFSD2A, CENPW, SPP1, ADAMDEC1, PIR, PLCH1, FOLH1, CXCL11, FYB2, WNK4, KRT14, ABCA10, IL33, PI15, ELOVL2, NOVA1, CCDC85A, NAV3, NTRK2, CYP2A7, PIEZO2, STC2, ERICH3, and TMEM26, preferably of all said genes.

In another embodiment, the method comprises determining the expression level of at least one gene selected from the group consisting of: CSF2RB, AQP9, S100A9, ADM, CXCL11, CXCL8, IFI27, CXCL10, IL33, CP, PIR, STC2, SPP1, EGFR, FOLH1, SLITRK5, NTRK2, SOX11, CDH3, PGR, KRT14, and SEC61G, preferably of all said genes.

In a preferred embodiment, said method comprises determining the expression level of at least 3 genes selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4, preferably it comprises determining the expression level of EGFR, CXCL8 and CP.

In a preferred embodiment, said method comprises determining the expression level of at least 3 genes, selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4, preferably it comprises determining the expression level of MCM10, SOX1 and AQP9.

In a preferred embodiment, said at least one gene or said at least three genes comprise any of:

- CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, and CHI3L 1;

- ADAMDEC1, ADM, AQP9, C5orf46, CDCA7, CDH3, CENPW, CP, CSF2RB, CXCL10, CXCL11, CXCL8, EGFR, EREG, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, and TMEM158;

- MCM10, SOX11, AQP9, CDCA7, S100A9, CXCL8, EGFR, PSAT1, ADM, CKB, CP, CXCL10, MFSD2A, CENPW, SPP1, ADAMDEC1, PIR, PLCH1, FOLH1, CXCL11, FYB2, WNK4, KRT14, ABCA10, IL33, PI15, ELOVL2, NOVA1, CCDC85A, NAV3, NTRK2, CYP2A7, PIEZO2, STC2, ERICH3, and TMEM26;

- CSF2RB, AQP9, S100A9, ADM, CXCL11, CXCL8, IFI27, CXCL10, IL33, CP, PIR, STC2, SPP1, EGFR, FOLH1, SLITRK5, NTRK2, SOX11, CDH3, PGR, KRT14, and SEC61 G;

- EGFR, CXCL8 and CP; or

- MCM10, SOX1 and AQP9.

In a preferred embodiment, said at least one gene or said at least three genes consist of:

- CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, and CHI3L 1;

- ADAMDEC1, ADM, AQP9, C5orf46, CDCA7, CDH3, CENPW, CP, CSF2RB, CXCL10, CXCL11, CXCL8, EGFR, EREG, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, and TMEM158;

- MCM10, SOX11, AQP9, CDCA7, S100A9, CXCL8, EGFR, PSAT1, ADM, CKB, CP, CXCL10, MFSD2A, CENPW, SPP1, ADAMDEC1, PIR, PLCH1, FOLH1, CXCL11, FYB2, WNK4, KRT14, ABCA10, IL33, PI15, ELOVL2, NOVA1, CCDC85A, NAV3, NTRK2, CYP2A7, PIEZO2, STC2, ERICH3, and TMEM26;

- CSF2RB, AQP9, S100A9, ADM, CXCL11, CXCL8, IFI27, CXCL10, IL33, CP, PIR, STC2, SPP1, EGFR, FOLH1, SLITRK5, NTRK2, SOX11, CDH3, PGR, KRT14, and SEC61G;

- EGFR, CXCL8 and CP; or

- MCM10, SOX1 and AQP9.

The method of the invention preferably comprises a further step b) wherein based on the expression of said at least one gene a risk score is calculated.
In a preferred embodiment, said risk score is calculated according to the following formula:

$$\text{Risk score} = \Sigma i \ (\beta i * Cq_{normalized}),$$

wherein i is the summation index for the at least one gene; $\beta$ is the ridge penalized Cox model coefficient for each gene; and $Cq_{normalized}$ is the normalized average level of expression for each gene.
Preferably the method further comprises a step c) of determining a prognosis by means of said risk score calculation.
In an embodiment, determining a prognosis includes classifying said subject in one of at least two classes corresponding to predicted levels of progression of the disease. Said classes may be better prognosis, intermediate prognosis and worse prognosis.
In an embodiment, determining a prognosis comprises stratifying the subject into a worse prognosis class if said calculated risk score is greater than a predetermined cut-off value or into a better prognosis class if the calculated risk score is lower than said predetermined cut-off value.
Said predetermined cut-off can be determined for example retrospectively correlating individual risk scores for a group of patients with disease outcome (e.g.: death related to breast cancer or overall survival, or disease-free interval) and then determining a cut-off separating better from worse prognosis patients (2-class risk score).
In another embodiment, determining a prognosis comprises stratifying the subject into a worse prognosis class if said calculated risk score is greater than a predetermined first cut-off value or into an intermediate prognosis class if the calculated risk score is lower than said predetermined first cut-off value but higher than a second predetermined cut-off value or into a better prognosis class if said calculated risk score is lower than said predetermined second cut-off value, wherein said first cut-off value is higher than said second cut-off value.
Said predetermined first and second cut-off can be determined for example retrospectively correlating individual risk scores for a group of patients with disease outcome (e.g.: death related to breast cancer or overall survival, or disease-free interval) and then determining two cut-offs separating better from intermediate from worse prognosis patients (3-class risk score).
Calculation of said cut-offs and consequent stratification into prognosis classes is within the common general knowledge and abilities of the skilled person.
A kit for performing the method of the invention is a further object of the invention, said kit comprising at least means for determining in an isolated biological sample from a subject the expression level of at least one gene as above defined. Said kit preferably further comprises means to calculate the risk score and optionally means for providing prognostic information.

The method of the invention can be implemented in a data processing device, such as a computer. Any suitable data processing device can be used for implementing the method of the invention.

A computer program comprising instructions which, when the program is executed by a computer, cause the computer to carry out the method of the invention is a further object of the invention.

A data processing device comprising means adapted for carrying out the method of the invention is a further object of the invention. Said data processing device preferably comprises:

i) means for receiving data representing the expression level of at least one gene as above defined in an isolated biological sample from a subject;

ii) means for calculating a risk score; and

iii) means for providing a prognosis of the disease based on said risk score.

## DETAILED DESCRIPTION OF THE INVENTION

### Figures

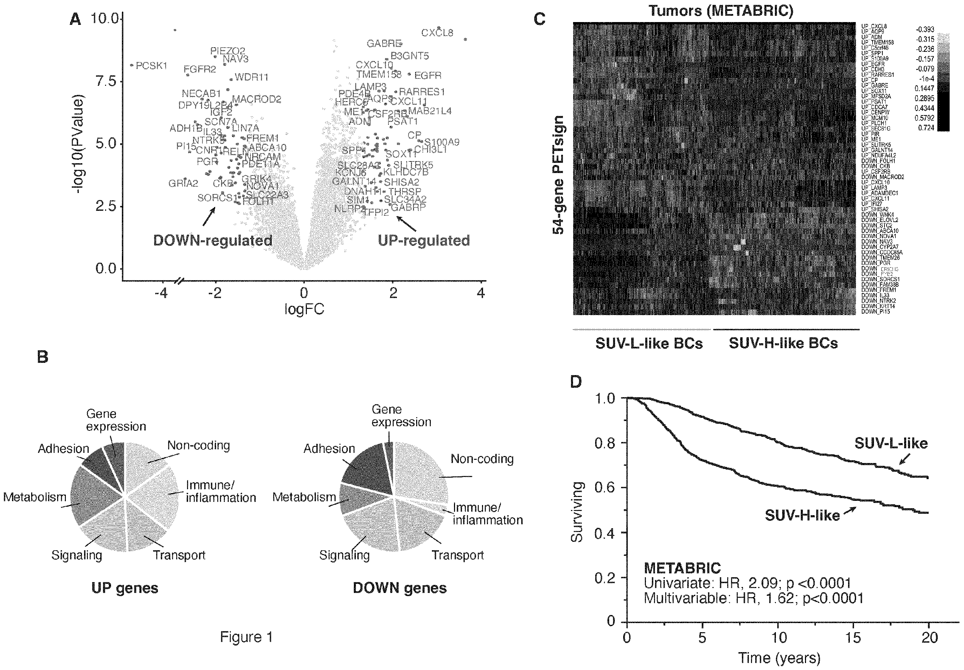

**Figure 1. Identification of PETsign. A.** Volcano plot of the genes differentially expressed between SUV-H and SUV-L BCs. Only the genes significantly regulated ($p < 0.05$) are shown with darker dots, in some cases accompanied by the name of the gene. Up-regulated genes are on the right, down-regulated genes are on the left. **B.** Pie charts of the functions of the upregulated and downregulated genes (SUV-H vs. SUV-L). The functional categories were attributed manually by interrogating the NCBI "Gene" database, followed by inspection of extant literature; (see details in Table 6). **C.** The 54 genes of the PETsign were used for hierarchical clustering of the METABRIC dataset leading to the identification of SUV-H-like and SUV-L-like BCs. The columns correspond to tumor samples, the rows are the genes. A grey-scale of the absolute levels on intensities is shown on the right. **D.** The two categories of SUV-H-like and SUV-L-like tumors identified in C were subjected to Kaplan-Meier analysis for time to DRBC (Death related to BC) in the METABRIC cohort. The results of the univariate and multivariable analyses are shown (parameters used for the multivariable analyses for all cohorts in this study are shown in Table 2). Hazard ratios (HR) and p-values (p) were calculated with the Cox proportional hazards model using JMP.

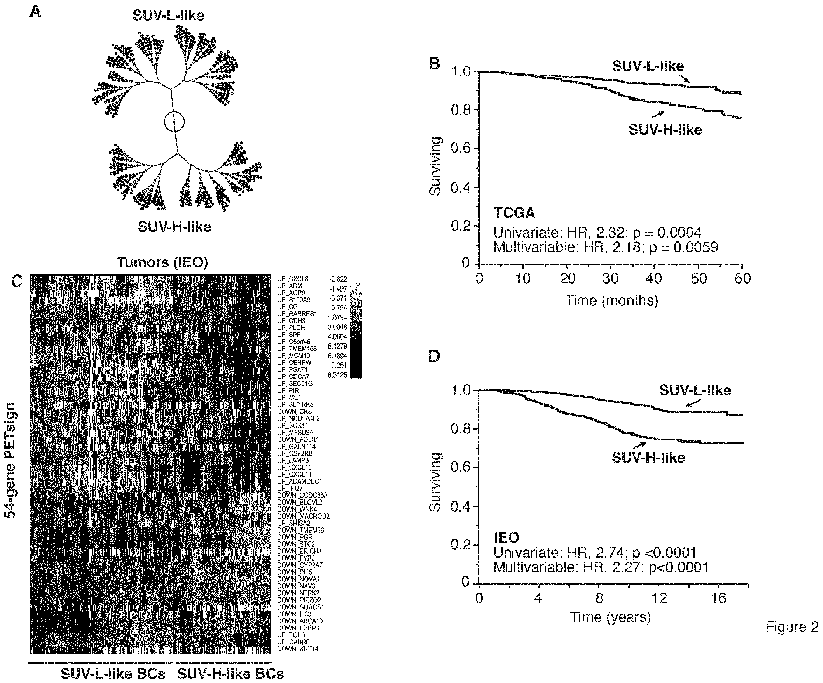

**Figure 2. Independent validation of PETsign. A.** Dendrogram analysis of the clustering of the TCGA dataset by the 54 genes of PETsign; top, SUV-H-like; bottom, SUV-L-like). **B.** Kaplan-Meier analysis of overall survival in the SUV-H-like and SUV-L-like tumor groups in the TCGA dataset. The results of the univariate and multivariable analyses are shown. **C.** Hierarchical clustering of the 970-IEO sub-cohort with the 54 genes of PETsign. The columns correspond to tumor samples, the rows are the genes. A grey-scale of the absolute levels on intensities is shown on the right. **D.** Kaplan-Meier analysis of time to DRBC (death related to BC) in the SUV-H-like and SUV-L-like tumor groups of the 970-IEO sub-cohort. The results of the univariate and multivariable analyses are shown. In (B) and (D), hazard ratios (HR) and p-values (p) were assessed by the Cox proportional hazards model using JMP. See also legend to Table 9 for the variables used in the multivariable analysis.

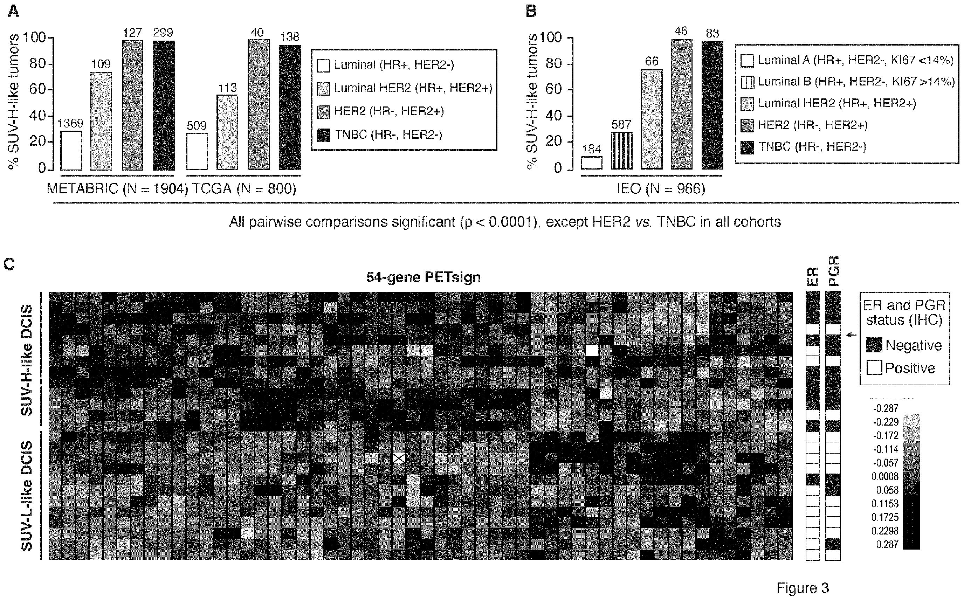

**Figure 3. Distribution of the SUV-H-like phenotype between BC molecular subtypes and DCIS. A, B.** The percentage of BCs displaying a SUV-H-like phenotype according to the BC molecular subtype is shown for the METABRIC and TCGA cohorts **A.** and for the 970-IEO sub-cohort **B.** The absolute numbers are indicated on the top of each bar. Due to the lack of complete clinicopathological information, 96 cases of the TCGA cohort (N=896) and 4 cases of the 970-IEO cohort were not assessed. In the IEO cohort, the availability of Ki-67 staining allowed the stratification of Luminal BCs into Luminal-A and Luminal-B, which was not possible in the other two cohorts. In (A) and (B), p-values were assessed by the chi-square tests of significance using JMP. **C.** Twenty-five DCIS (rows) where clustered using the genes of the PETsign (columns). The HR (ER/PGR) status of each DCIS is shown on the right. A grey-scale of the absolute levels on intensities is shown further on the right.

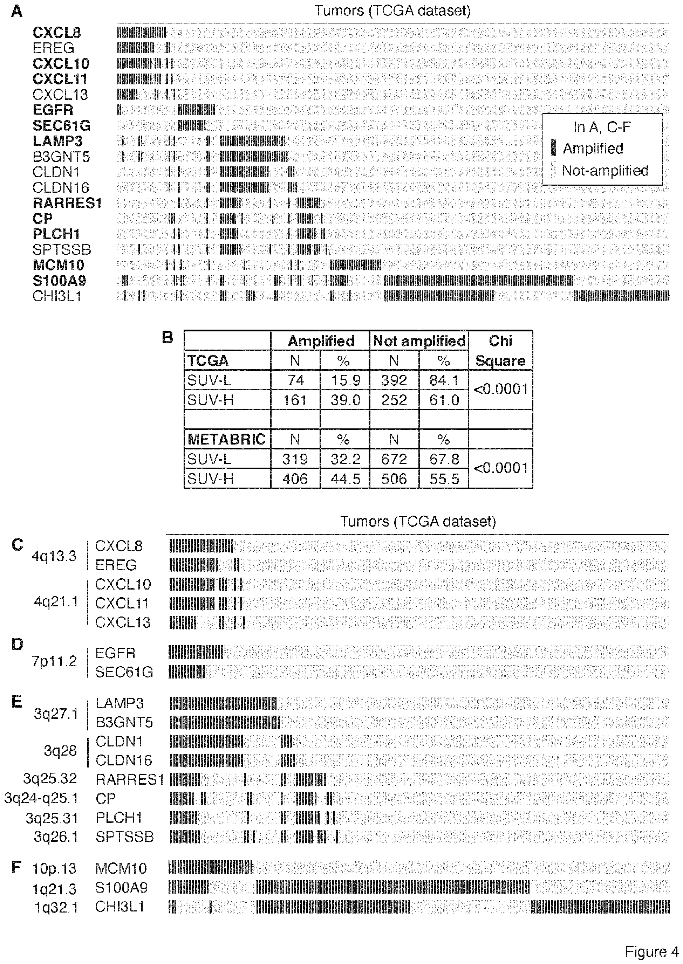

**Figure 4. Co-amplification of genes from PETsign and the 135-gene signature in BC. A.** Amplification and co-amplification of the 54 PETsign genes and the remaining upregulated genes from the original 135-gene signature was examined in the TCGA dataset. In total, 235 BCs from (26%) of the TGCA database harbored amplification/co-amplification of the PETsign/135-signature genes (indicated by black bars). We considered only genes that were amplified in at least 1.5% of cases and showed a pattern of co-occurrence with another amplified gene with a q-value <0.001. PETsign genes are in bold, while the other genes belong to the 135-gene signature. Graphic representations were obtained on the cBioPortal [21, 22]. **B.** Percentage of SUV-H-like and SUV-L-like tumors from the TCGA and METABRIC datasets harboring amplification of one or more of the 18 genes. P-values were calculated by the chi-square test in Excel. **C-F.** Pattern of co-amplification of the four group of genes in the TCGA dataset. The groups were identified when the highest stringency criterion (q-value for co-occurrence < 0.001) was applied to the 18 genes. The locus of each gene is indicated on the left.

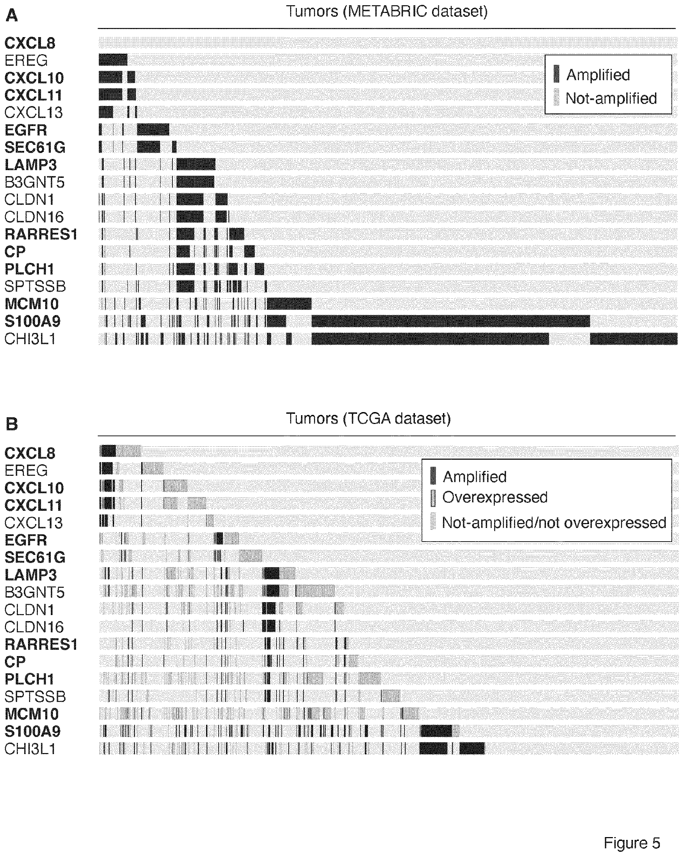

**Figure 5. Co-amplification and overexpression of genes from PETsign. A.** The pattern of amplification/co-amplification of the 18 genes uncovered in the mining of the TCGA dataset (Fig. 4A) was investigated in the METABRIC dataset. In total, 725 BCs 38% of the METABRIC database harbored co-amplification of the 18 genes (indicated by black bars). PETsign genes are in bold, while the other genes belong to the 135-gene signature. Note that no amplification data were retrievable for CXCL8. **B.** Amplification and overexpression of the 18 genes in the TCGA dataset. The graph shows the result of the combined analysis of amplification and mRNA overexpression in the TCGA dataset: 504 BC cases (~56%) showed one or both alterations. Amplification, black, overexpression, dark grey. For overexpression, we selected the function "mRNA expression z-scores relative to diploid samples (RNA Seq V2 RSEM)" with a threshold z-score of 2.0.

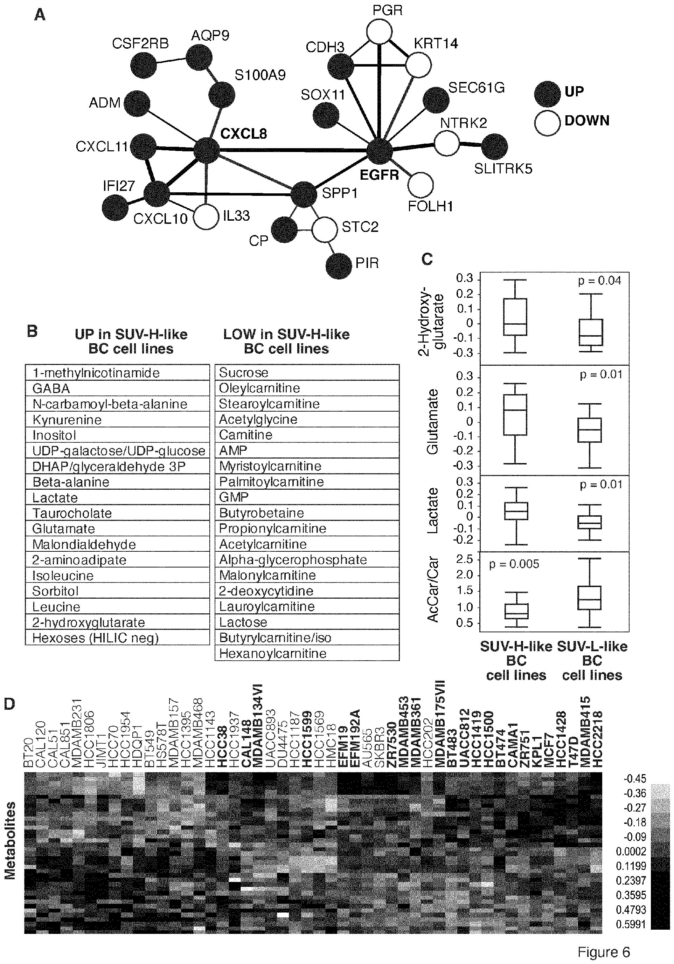

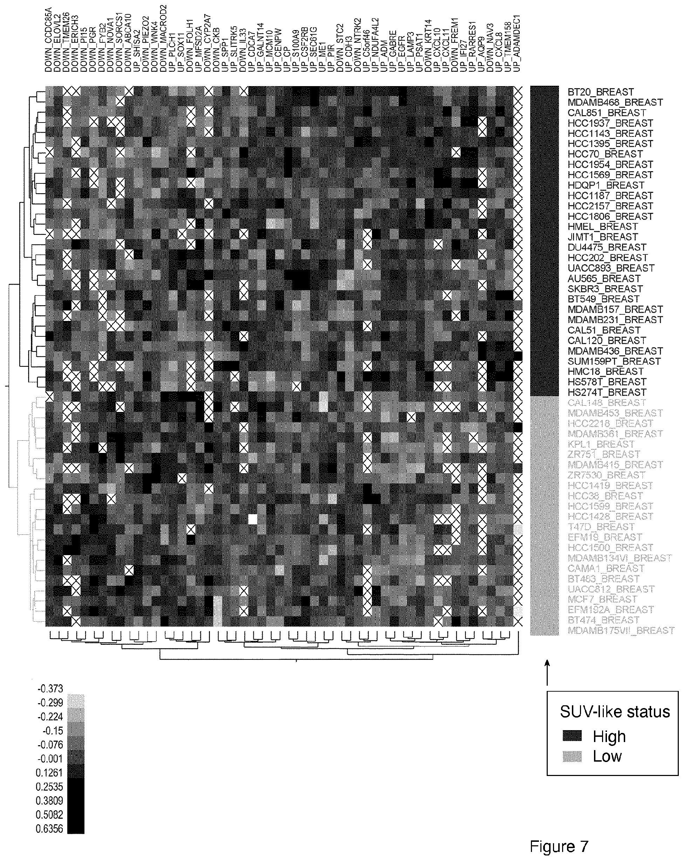

**Figure 6. PETsign genes predict metabolic features of BC cell lines. A.** STRING analysis of PETsign proteins. The clustering with STRING was performed with default parameters (full string network; meaning of network edges; evidence; medium confidence). Non-connected nodes or poorly connected ones (1 edge) were removed to yield the shown network. The thickness of the edges corresponds to the strength of the interaction. The color code indicates the gene regulation (filled circles, up; empty circles, down) in PETsign. The two major hubs (CXCL8 and EGFR) are in bold. **B, C.** Forty-eight BC cell lines for which transcriptomic [23] and metabolomic [24] data are available, were analyzed and metabolites with significantly different levels between SUV-H-like vs. SUV-L-like cell lines were identified. The list of significant differentially present metabolites is shown in (**B**) (see also Table 11). Boxplots of the levels of selected oncometabolites are shown in (**C**). For the analysis of acetylcarnitine vs. carnitine, in each cell line, the ratio between acetylcarnitine/carnitine abundance was calculated and then the differences in the mean ratio in SUV-H-like vs. SUV-L-like groups was assessed. All p-values were obtained by the non-parametric Wilcoxon test using JMP. (**D**) The metabolites (rows), identified in B, were used for unsupervised hierarchical clustering of the 48 cell lines (columns), which yielded a grouping largely superimposable with the SUV-H-like and SUV-L-like phenotypes obtained by transcriptomics (also shown in Fig. 7), indicated by regular font and bold font cell line names, respectively.

**Figure 7. Hierarchical clustering of BC cell lines.** The 54 genes of the PETsign were used for hierarchical clustering of the dataset of RNAseq of 53 BC cell lines available from the from the CCLE (Cancer Cell Line Encyclopedia) collection [23] (https://sites.broadinstitute.org/ccle/datasets). Cell lines are on the rows; genes on the columns. For 48 of these cell lines, metabolomics data were also available [24] and were used for the clustering shown in Fig. 6D. A grey-scale of the absolute levels on intensities is shown at the bottom on the left.

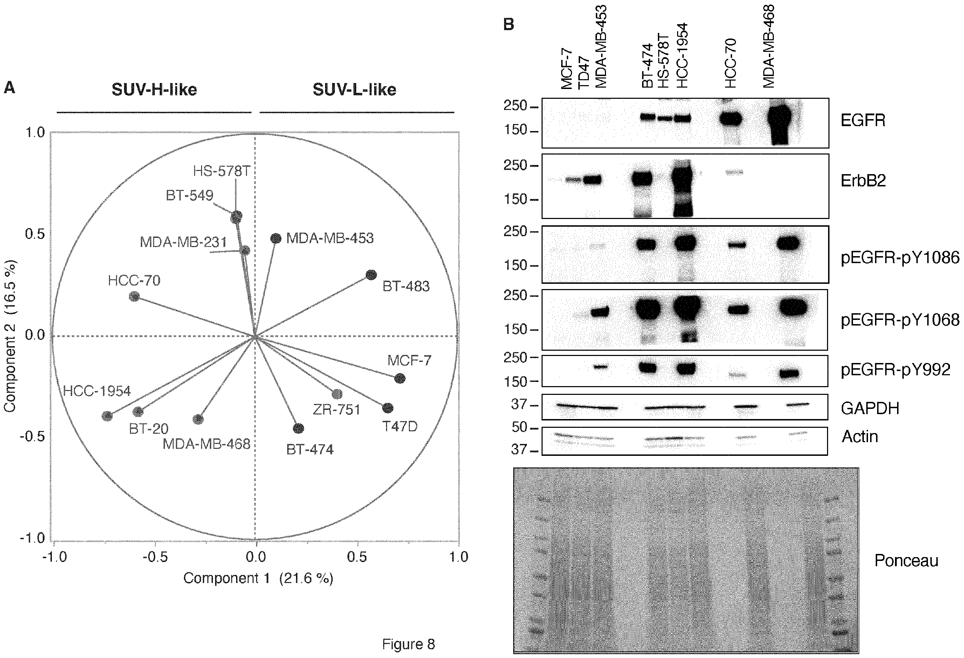

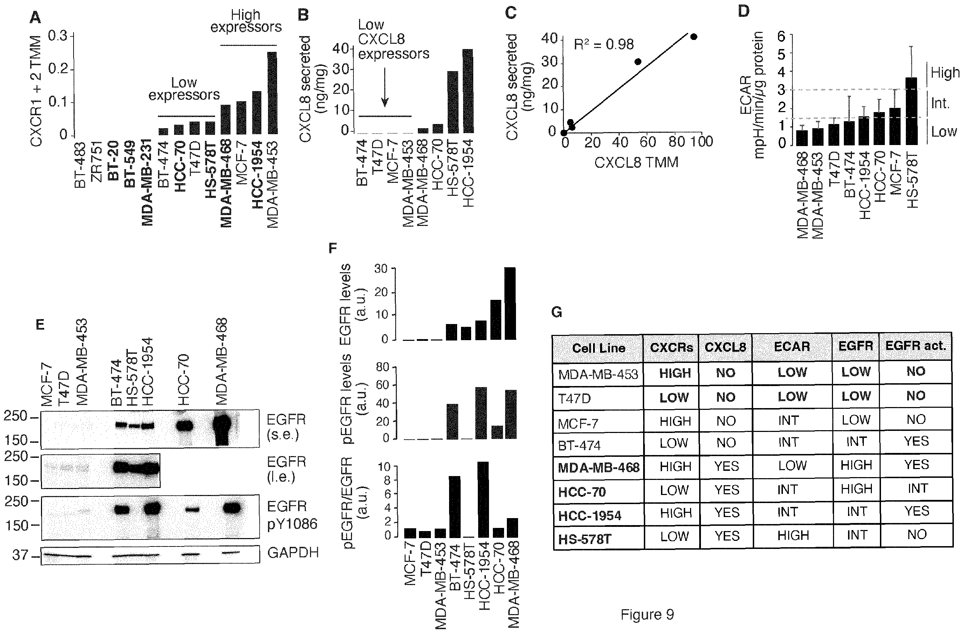

**Figure 8. Characterization of 13 BC cell lines. A.** Principal component analysis (PCA) analysis of the 13 BC cell lines stratified by PETsign. SUV-H-like lines and SUV-L-like lines are indicated on top. **B.** The immunoblot in Fig. 9E is shown again here with additional antibody staining for different EGFR phosphosites (pY992 and pY1068 in addition to pY1086). EGFR, total EGFR; GAPDH and actin, loading controls. Anti-ErbB2 was also performed to verify the HER2 status of the cell lines. MW markers (kDa) are shown. The Ponceau staining of the membrane is shown beneath the blots.

**Figure 9. Characterization of CXCL8 and EGFR signaling pathways in a panel of BC cell lines. A.** Expression analysis of the CXCL8 receptors, CXCR1 and CXCR2, by RNAseq in the indicated BC cell lines. CXCR1 and CXCR2 expression data are reported as combined (CXCR1 + CXCR2) Trimmed Mean of M-values (TMM). SUV-H-like and SUV-L-like BC lines (see Suppl. Fig. 3A) are shown in bold and reguar fonts, respectively **B.** CXCL8 secretion levels in the indicated BC cell lines. Results are the average of duplicate biological points. **C.** Regression analysis of CXCL8 transcription (TMM, extracted from the RNAseq dataset) vs. secretion (from panel B). **D.** Extracellular acidification rate (ECAR), measured as mpH/min/mg of protein, determined by Seahorse analysis. Data are expressed as the mean + SD of 3 independent experiments. Significance was calculated by the ANOVA one-way test using SigmaPlot 14.0. Two arbitrary thresholds of 1.5 and 3 were used to stratify the cell lines as into low, intermediate and high ECAR groups. **E.** Immunoblot (IB) of the indicated cell lines with anti-EGFR (s.e., short exposure; I.e., long exposure) and anti-phosphoEGFR (EGFR-pY1086). GAPDH, loading control. MW markers (kDa) are on the left. **F.** Densitometric quantitation of total EGFR and EGFR-pY1086 (pEGFR) levels in the IB in "E". The ratio of pEGFR to EGFR is also shown. Data are expressed as arbitrary units (a.u.) after normalization to GAPDH values. **G.** Summary of data in panels A-F. The SUV-like status of the cells lines is indicated (bold, SUV-H-like; regular font, SUV-L-like). MDA-MB-453 and T47D were chosen for subsequent experiments based on characteristics highlighted in bold.

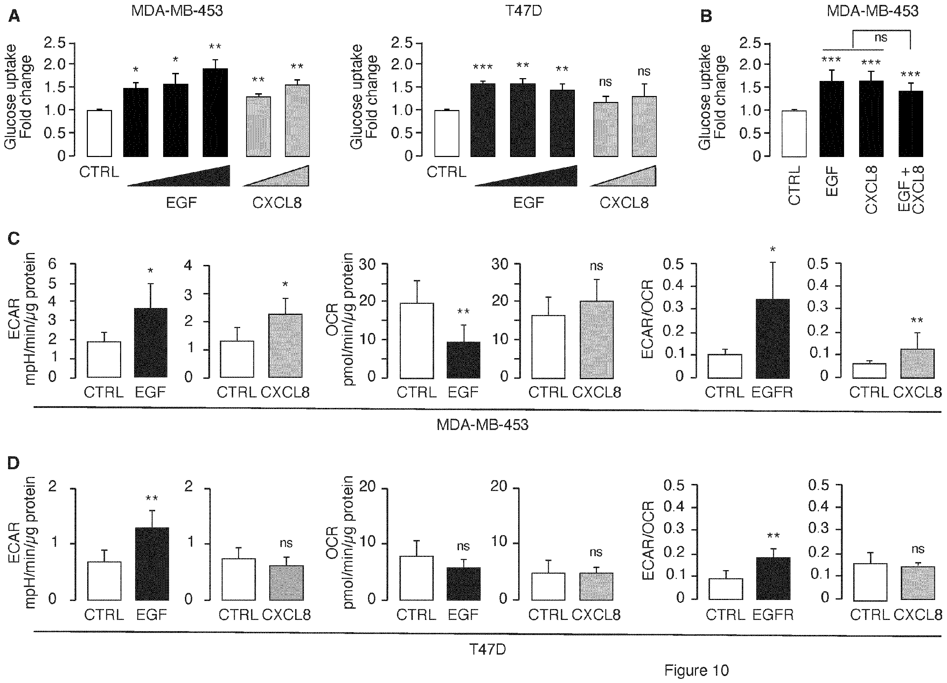

**Figure 10. EGF and CXCL8 stimulation induces metabolic alterations in MDA-MB-453 and T47D. A.** 2-deoxy-glucose (2-DG) uptake in MDA-MB-453 (left) and T47D (right) cells treated with EGF (1, 10 or 100 ng/mL for 1 h) or CXCL8 (10 or 100 ng/mL for 1 h) or mock treated (CTRL). Data are expressed as mean fold-increase over CTRL + SE (n = 4 independent biological replicas, with at least 4 technical replicates per experiment). Significance was calculated with the two-tailed unpaired t-test. **B.** 2-DG uptake in MDA-MB-453 cells treated with EGF or CXCL8 alone or together (10 ng/ml/each, 1 h). Data are expressed as mean fold-increase over CTRL + SE (n = 3 independent biological replicas, each in sextuplicate). Significance was calculated with the two-tailed unpaired t-test. Note that all treatments are significant vs. control, but EGFR+CXCL8 is not significant vs. single treatments. **C, D.** Seahorse analysis of MDA-MB-453 cells (C) and T47D cells (D), mock treated (CTRL) or treated with EGF or CXCL8 (100 ng/mL for 15 h). ECAR data (left) are expressed as mpH/min/mg of protein; OCR data (middle) are expressed as pmol/min/mg of protein. Results are the mean + SD of 3 independent experiments. The ECAR/OCR ratios are expressed as mpH/pmol and represent the mean + SD of ECAR/OCR values of 3 independent experiments. Significance was calculated vs. CTRL with the two-tailed unpaired *t*-test using SigmaPlot 14.0. In all panels: * $p < 0.05$, ** $p < 0.01$, *** $p < 0.001$, ns: not significant.

In the context of the present invention, for "breast cancer" is intended any kind of cancer affecting any part of the breast.

In the context of the present invention, for "prognosis" is intended the likely or expected development of a disease, in particular the more or less aggressiveness of the disease.

In the context of the present invention, "predicting prognosis" and "determining a prognosis" are used as synonyms.

In the context of the present invention, "gene" refers to a nucleic acid that is transcribed. In certain aspects, the gene includes regulatory sequences involved in transcription or message production. In particular embodiments, a gene comprises transcribed sequences that encode for a protein, polypeptide or peptide. As will be understood by those in the art, this functional term "gene" includes genomic sequences, RNA or cDNA sequences or smaller engineered nucleic acid segments, including nucleic acid segments of a non-transcribed part of a gene, including but not limited to the non-transcribed promoter or enhancer regions of a gene. Smaller engineered nucleic acid segments may express, or may be adapted to express proteins, polypeptides, polypeptide domains, peptides, fusion proteins, mutant polypeptides and/or the like.

The genes herein mentioned are preferably characterized by the sequences described by the following NCBI Gene ID (NCBI Database release version December 15, 2023):

| Gene Name | Approved Name | NCBI Gene ID |
|-----------|---------------|--------------|
| ADAMDEC1 | ADAM like decysin 1 | 27299 |
| ADM | adrenomedullin | 133 |
| AQP9 | aquaporin 9 | 366 |
| C5orf46 | chromosome 5 open reading frame 46 | 389336 |
| CDCA7 | cell division cycle associated 7 | 83879 |
| CDH3 | cadherin 3 | 1001 |
| CENPW | centromere protein W | 387103 |
| CP | ceruloplasmin | 1356 |
| CSF2RB | colony stimulating factor 2 receptor subunit beta | 1439 |
| CXCL10 | C-X-C motif chemokine ligand 10 | 3627 |
| CXCL11 | C-X-C motif chemokine ligand 11 | 6373 |
| CXCL8 | C-X-C motif chemokine ligand 8 | 3576 |
| EGFR | epidermal growth factor receptor | 1956 |
| GABRE | gamma-aminobutyric acid type A receptor subunit epsilon | 2564 |

(continued)

| Gene Name | Approved Name | NCBI Gene ID |
|---|---|---|
| GALNT14 | polypeptide N-acetylgalactosaminyltransferase 14 | 79623 |
| IFI27 | interferon alpha inducible protein 27 | 3429 |
| LAMP3 | lysosomal associated membrane protein 3 | 27074 |
| MCM10 | minichromosome maintenance 10 replication initiation factor | 55388 |
| ME1 | malic enzyme 1 | 4199 |
| MFSD2A | major facilitator superfamily domain containing 2A | 84879 |
| NDUFA4L2 | NDUFA4 mitochondrial complex associated like 2 | 56901 |
| PIR | pirin | 8544 |
| PLCH1 | phospholipase C eta 1 | 23007 |
| PSAT1 | phosphoserine aminotransferase 1 | 29968 |
| RARRES1 | retinoic acid receptor responder 1 | 5918 |
| S100A9 | S100 calcium binding protein A9 | 6280 |
| SEC61G | SEC61 translocon subunit gamma | 23480 |
| SHISA2 | shisa family member 2 | 387914 |
| SLITRK5 | SLIT and NTRK like family member 5 | 26050 |
| SOX11 | SRY-box transcription factor 11 | 6664 |
| SPP1 | secreted phosphoprotein 1 | 6696 |
| TMEM158 | transmembrane protein 158 | 25907 |
| ABCA10 | ATP binding cassette subfamily A member 10 | 10349 |
| CCDC85A | coiled-coil domain containing 85A | 114800 |
| CKB | creatine kinase B | 1152 |
| CYP2A7 | cytochrome P450 family 2 subfamily A member 7 | 1549 |
| ELOVL2 | ELOVL fatty acid elongase 2 | 54898 |
| ERICH3 | glutamate rich 3 | 127254 |
| FOLH1 | folate hydrolase 1 | 2346 |
| FREM1 | FRAS1 related extracellular matrix 1 | 158326 |
| FYB2 | FYN binding protein 2 | 199920 |
| IL33 | interleukin 33 | 90865 |
| KRT14 | keratin 14 | 3861 |
| MACROD2 | mono-ADP ribosylhydrolase 2 | 140733 |
| NAV3 | neuron navigator 3 | 89795 |
| NOVA1 | NOVA alternative splicing regulator 1 | 4857 |
| NTRK2 | neurotrophic receptor tyrosine kinase 2 | 4915 |
| PGR | progesterone receptor | 5241 |
| PI15 | peptidase inhibitor 15 | 51050 |
| PIEZO2 | piezo type mechanosensitive ion channel component 2 | 63895 |
| SORCS1 | sortilin related VPS10 domain containing receptor 1 | 114815 |
| STC2 | stanniocalcin 2 | 8614 |
| TMEM26 | transmembrane protein 26 | 219623 |

(continued)

| Gene Name | Approved Name | NCBI Gene ID |
|---|---|---|
| WNK4 | WNK lysine deficient protein kinase 4 | 65266 |

The biological sample is preferably a tissue sample. Preferably it is a tumoral tissue sample, i.e. a sample obtained by tumoral tissue, in particular by the breast tumoral tissue. Said tissue sample comprises at least one cancerous cell. The sample can be for example a tissue sample previously obtained by surgery, e.g. biopsy, from the subject. Methods for collecting biological samples, in particular tumor biological samples, are well known in the field. The sample can then be prepared for preservation and examination, for example using fixative solutions, according to common general knowledge. In an embodiment, the biological sample is a formalin-fixed paraffin-embedded (FFPE) sample.

In an exemplary embodiment of the invention, the sample is previously isolated from a subject affected by breast cancer, preferably Luminal breast cancer. Breast cancer can be of any kind or form. The subject may be at any stage of the disease, such as early stage or late stage. The subject can be a patient affected by ductal in situ carcinoma (DCIS), early stage breast cancer, Luminal A, Luminal B, Luminal-HER2, HER2 and triple-negative BC (TNBC).

For "luminal BC" is intended a breast cancer positive for HR, and negative for HER2 amplification (HR+, HER2-). Luminal BC can be subclassified into Luminal A or B based on the levels of Ki67 expression.

For "Luminal-HER2" is intended a breast cancer positive for HR and positive for HER2 (HR+, HER2+).

For "HER2" is intended a breast cancer negative for HR and positive for HER2 (HR-, HER2+).

For "triple-negative BC" or "TNBC" is intended a breast cancer negative for HR, negative for HER2 and negative for PR.

The breast cancer can be primary or metastatic.

The subject can be a subject diagnosed with breast cancer or a subject presenting with one or more symptoms of breast cancer or a subject having undiagnosed breast cancer. Preferably, is a subject diagnosed with breast cancer.

The sample may be prepared for subsequent RNA extraction, as known in the field.

Gene expression in the sample can be determined using any method known in the art. For example, the amount in the sample of gene expression products, such as RNA transcripts, mRNAs or proteins may be measured.

For example a PCR-based method, an array based method or a sequencing-based method might be used. All such methods are known in the art and can be carried out according to the common general knowledge in the field. Exemplary techniques to determine gene expression are analysis of single strand conformation polymorphism, capillary electrophoresis, denaturing high performance liquid chromatography, digital molecular barcoding technology, e.g., Nanostring's nCounter® system, direct sequencing, DNA mismatch-binding protein assays, dynamic allele-specific hybridization, Fluorescent in situ hybridization (FISH), high-density oligonucleotide SNP arrays, high-resolution melting analysis, microarray, next generation sequencing (NGS), e.g., using the Illumina Genome Analyzer, ABI Solid instrument, Roche 454 instrument, Heliscope instrument, Northern blot analysis, nuclease protection analysis, oligonucleotide ligase assays, polymerase chain reaction (PCR), primer extension assays, Quantigene analysis, quantitative nuclease-protection assay (qNPA), reporter gene detection, restriction fragment length polymorphism (RFLP) assays, reverse transcription and real-time quantitative polymerase chain reaction (RT- qPCR), reverse transcription-polymerase chain reaction (RT-PCR), RNA sequencing (RNA-seq), Serial analysis of gene expression (SAGE), Single Molecule Real Time (SMRT) DNA sequencing technology, SNPLex, Southern blot analysis, Sybr Green chemistry, TaqMan-based assays, temperature gradient gel electrophoresis (TGGE), Tiling array, Western blot analysis, and immunohistochemistry. In any of the above aspects or embodiments, gene expression may be determined using reverse transcription and real-time quantitative polymerase chain reaction (RT-qPCR) with primers and/or probes (e.g., TaqMan® probes) specific for each of said at least three genes. Alternately, the gene expression may be determined using microarray analysis with probes specific for an expression product of each gene.

In an embodiment, the level of RNA transcripts of the herein defined genes is measured in the sample using a RNA sequencing method, such as RNAseq. Methods of extracting RNA and determining the level of RNA transcripts of a particular gene in a sample are well-known to those skilled in the art.

Gene expression is typically normalized to the expression of at least one reference gene, such as an housekeeping gene. Housekeeping genes are well known in the field, reference can be made to [25]. For example, said reference gene can be selected from the group consisting of GAPDH, GUSB, HPRT1, and TBP.

According to a preferred embodiment, the method of the invention comprises a further step b) wherein based on the expression of said at least one gene a risk score is calculated.

In a preferred embodiment, said risk score is calculated according to the following formula:

$$\text{Risk score} = \Sigma i \ (\beta i * Cq_{normalized}),$$

wherein i is the summation index for the at least one gene; β is the ridge penalized Cox model coefficient for each gene; and $Cq_{normalized}$ is the normalized average level of expression for each gene.

Said calculation can be carried out by the skilled person according to common general knowledge in the field.

In preferred embodiments of the invention, the risk score is indicative of the prognosis of the disease.

In particular, the subject can be classified or stratified in better, intermediate or worse prognosis groups depending on said risk score. More in particular, cut-off values may be previously determined in a group of patients and then used to classify the subject, as above described.

For "worse prognosis" or "poor prognosis" is intended an aggressive likely or expected development of the disease.

For "better prognosis" is intended a not aggressive likely or expected development of the disease.

For "intermediate prognosis" is intended a moderately aggressive likely or expected development of the disease.

All such terms are well known in the field and herein have the meanings currently used in the field.

Stratification and classification are herein used as synonyms.

In some embodiments, the method of the invention can be advantageously used in combination with any other prognostic method useful in the prognosis and/or in the classification of a subject affected by breast cancer. Such prognostic methods are known in the art, such as for example the methods disclosed in WO2017220492, WO2005039382, WO2009158143, WO02103320 and patents or patent applications derived thereof, or disclosed in [11-15, 26, 27].

Kits for carrying out the method of the invention are also objects of the invention. In particular, said kit may comprise means for determining in an isolated biological sample the expression level of at least one gene as above defined, such as probes and/or primers for binding an expression gene product, such as a RNA transcript or a protein, of one or more genes disclosed herein or primers for polymerase chain reaction of one or more RNA transcripts of above mentioned genes. Such probes and primers can be synthetized according to common general knowledge in the field. The kit may further comprise instructions or a computer readable media to calculate the risk score and for providing prognostic information. For example, mathematical algorithms to calculate the risk score and estimate or quantify prognostic or predictive information, for example as above described, are properly potential components of the kit.

The method of the invention is also a method for stratifying subjects affected by breast cancer based on the predicted prognosis of the disease.

The method of the invention can also advantageously help in stratifying subjects for cancer treatments.

It is an object of the invention a method for determining a breast cancer treatment for a subject affected by breast cancer comprising the steps of a) determining the expression level of at least one gene in an isolated biological sample from said subject, wherein said gene is selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4; b) calculating a risk score based on the expression level of said at least one gene; c) determining a prognosis of said subject based on said risk score and d) determining a breast cancer treatment for said subject based on said prognosis.

In an embodiment, the expression level of all said genes or at least all said genes is determined. Expression level of further genes may also be determined.

In an embodiment, said method comprises determining the expression level of at least 2, 3, 4, 5, 6, 7, 8, 9 or 10 genes from the above list.

In an embodiment, said method comprises as step a) a step of determining the expression level of at least three genes in an isolated biological sample from said subject, wherein said at least three genes are selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4.

Said steps b) and c) may be carried out as above described.

Said breast cancer treatment may comprise surgery, radiation and/or any anti-cancer or chemotherapeutic agent. Said anti-cancer or chemotherapeutic agent may be selected from anthracycline agents, alkylating agents, nucleoside analogs, platinum agents, vinca agents, anti-estrogen drugs, aromatase inhibitors, ovarian suppression agents, endocrine/hormonal agents, bisphophonate therapy agents and targeted biological therapy agents (e.g., antibodies), cyclophosphamide, fluorouracil (or 5-fluorouracil or 5-FU), methotrexate, thiotepa, carboplatin, cisplatin, gemcitabine, anthracycline, taxanes, paclitaxel, protein-bound paclitaxel, doxorubicin, docetaxel, vinorelbine, tamoxifen, raloxifene, toremifene, fulvestrant, irinotecan, ixabepilone, temozolmide, topotecan, vincristine, vinblastine, eribulin, mutamycin, capecitabine, capecitabine, anastrozole, exemestane, letrozole, leuprolide, abarelix, buserlin, goserelin, megestrol acetate, risedronate, pamidronate, ibandronate, alendronate, denosumab, zoledronate, trastuzumab, tykerb or bevacizumab, inhibitors of EGFR, inhibitors of CXCL8, inhibitors of CXCR1, inhibitors of CXCR2, inhibitors of protein kinases, immunotherapy, immune checkpoint inhibitors, and combinations thereof.

The skilled person knows how to select one or more suitable cancer treatments based on the obtained prognosis of the disease.

It is also an object of the invention a method for treating a subject affected by breast cancer comprising a) determining the expression level of at least one gene in an isolated biological sample from said subject, wherein said gene is selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4; b) calculating a risk score based on the expression level of said at least one gene; c) determining a prognosis of said subject based on said risk score and d) administering to the subject a breast cancer treatment based on said prognosis.

In some embodiments, the subject classified as a subject with a worse or poor prognosis may be provided with a cancer treatment that is more aggressive than the cancer treatment provided to the subject classified as a subject with a better or intermediate prognosis.

For example, a "more aggressive" cancer treatment may comprise a higher dose of an anti-cancer or chemotherapeutic agent and/or more frequent dosing of an anti-cancer or chemotherapeutic agent and/or a more potent anti-cancer or chemotherapeutic agent and/or a plurality of anti-cancer or chemotherapeutic agents or of treatment modalities, e.g., anti-cancer or chemotherapeutic agents along with surgical intervention, anti-cancer or chemotherapeutic agents along with radiation, radiation along with surgical intervention, and anti-cancer or chemotherapeutic agents, surgical intervention, and radiation.

Said anti-cancer or chemotherapeutic agents for use for the treatment of a subject affected by breast cancer, wherein said subject has been stratified according to the method of the invention, is also an object of the invention.

## EXAMPLES

## MATERIALS AND METHODS

### Reagents and cell culture

DMEM, RPMI-1640, MEM, and Ham's F12 were from: Euroclone #ECM0103L, Thermo Fisher # 21875034, Lonza #LOBE12125F, and Thermo Fisher #31765027, respectively. FBS (fetal bovine serum) was of North American origin (Hyclone, CHA30088L) for MCF-7, T47D, BT-474, MDA-MB-431, HCC-1954, BT-483, HS-578T, HCC-70, MDA-MB-453, and BT-20 or of South America origin (Microtech # RM10432) for ZR-751, BT-549, MDA-MB-468. Supplements were: L-glutamine (Lonza #LOBE17605E), sodium pyruvate (Lonza #LOBE13115E), HEPES (Sigma Aldrich # H0887), Non-Essential Amino Acids (NEAA) (Lonza #LOBE13114E), insulin (Merck Life Science # 91077C). CXCL8 was from R&D system (Recombinant Human IL-8/CXCL8 Protein, 208-IL), EGF was from Preprotech (Animal-Free Recombinant Human EGF, AF-100-15).

MCF-7, T47D, BT-474, BT-549, MDA-MB-231, and MDA-MB-453 were cultured in DMEM + 10% FBS + 2 mM L-glutamine. ZR751, HCC-1954 and HCC-70 were cultured in RPMI-1640 + 10% FBS + 2 mM L-glutamine + 1 mM sodium pyruvate + 10 mM HEPES. MDA-MB-468 were cultured in DMEM/Ham's F12 (1:1) + 10% FBS + 2 mM L-glutamine. BT-20 were cultured in MEM with Earle's Salts + 10% FBS + 2 mM L-glutamine + 1 mM sodium pyruvate + 0.1 mM NEAA. BT-483 were cultured in RPMI-1640 + 20% FBS + 2 mM L-glutamine + 0.01 mg/mL insulin. HS-578T were cultured in DMEM + 10% FBS + 2 mM L-glutamine + 0.01 mg/mL insulin.

All cell lines, at each batch freezing, were authenticated by STR profiling (StemElite ID System, Promega) and tested for mycoplasma by PCR [28] and biochemical assay (Aurogene, REP-MYS-100).

### Experiments with BC cell lines

CXCL8 secretion was measured on the Luminex platform (Thermo Fisher) in biological duplicates on 24 h conditioned medium.

Immunoblotting was performed on 30 mg of protein from total cell lysates with the following antibodies: anti-EGFR (EGFR806, in-house, directed against the last 12 amino acids of the EGFR peptide), anti-ERBB2 (Cell Signaling #2165), anti-pEGFR-pY1086 (Cell Signaling #2220), anti-pEGFR-pY1068 (Cell Signaling #3777), anti-pEGFR-pY992 (Cell Signaling #2235), anti-GAPDH (Santa Cruz sc-32233), anti-actin (Merk A4700).

For glucose uptake assays, MDA-MB-453 and T47D cells were seeded in 96-well plates (5000 cells/well). After 72 h of culture, the medium was replaced with complete medium containing, or not, EGF (1, 10 and 100 ng/mL) or CXCL8 (10 and 100 ng/mL) for 1 h before measuring the uptake of 2-deoxy-glucose (2-DG) with the Glucose Uptake-Glo™ Assay kit (Promega). Briefly, cells were incubated in glucose free-medium supplemented with 1 mM 2-DG for 10 min at room temperature. Next cells were lysed and the amount of 2-DG quantified according to the manufacturer's instructions. Background was subtracted from measurements and data were normalized over control.

OCR and ECAR analyses were performed with a Seahorse XF24 Extracellular Flux Analyzer (Agilent) as previously described [29]. A titration with the uncoupler CCCP was performed to determine the CCCP concentration (1 $\mu$M) that maximally increases OCR. The results were normalized for the protein content. In some experiments, 24 h after plating, cells were mock-treated or treated with EGF (100 ng/mL) or with CXCL8 (100 ng/mL) in DMEM medium. After 15 h, medium was replaced with DMEM supplemented with 25 mM glucose, 1 mM sodium pyruvate, 30 mM NaCl, 5 mM HEPES, 1 mM L-glutamine, in the presence or absence of EGF or CXCL8, and measurements were performed.

**In-house clinical cohorts and the panel of 13 BC cell lines**

FFPE mammary tissue specimens were collected at the IEO (Milan, Italy). All tissues were collected via standard operating procedures approved by the Institutional Ethical Board, and informed consent was obtained for all tissue specimens linked with clinical data.

The 120-case PET cohort (Fig. 1A, Table 1) was collected at IEO (Milan, Italy). Medical records were screened for all patients with newly diagnosed BC who underwent 18F-FDG PET/CT before any treatment between December 2010 and March 2016. cT1-T3 BC patients were enrolled, excluding patients with multicentric disease. PET/CT scans were carried out with 3D PET/CT scanner and iterative reconstruction (GE Healthcare, Milwaukee, Winsconsin, USA) after the intravenous administration of 3.5 MBq/Kg of 18F-FDG. PET data were acquired from the skull base to the mid-thigh in three-dimensional mode and a scan time of 2.5-3 minutes per bed position. The tomographic raw data were corrected for attenuation using transmission data derived from low-dose non-contrast CT scans performed before emission imaging according to a standardized protocol with the following settings: 120 KVp, 80 mA, tube rotation 0.8 sec, table speed 15 mm/sec, pitch 1.5. For visual interpretation, images were displayed in the three orthogonal projections and as whole-body maximum-pixel-intensity projection (MIP) images. Visual analysis was performed on digital transaxial PET, CT, and fused images. Radiotracer uptake was considered abnormal if it was focal and appreciable in two or more slices, not explainable with physiological or benign/inflammatory processes and with higher intensity than surrounding tissues. Suspicion of malignancy was based on the combined analysis of PET, co-registered CT, and fused PET/CT images. For quantitative analysis, the slice with highest uptake was selected on axial images. The region of interest (ROI) was defined manually around the lesion and the single-pixel maximum standardized uptake value (SUVmax) was determined.

For the present study, 120 BCs were selected which displayed a SUVmax > 10 (SUV-H, N=57) or a SUVmax < 5 (SUV-L, N=63), and FFPE blocks were retrieved. After review of haematoxylin and eosin-stained slides, a pathologist identified and marked on the block, areas of high tumor cellularity (>70%), devoid of DCIS, immune infiltrate and/or necrosis. These areas were punched to extract a tissue core 1.5 mm in diameter and ~2 mm in length, which was then processed for RNA extraction.

The IEO clinical cohort of ~2000 consecutive BCs has been previously described [26] (see also Table 2); the 970-IEO sub-cohort used for RNAseq experiments is described in Table 3.

The 120-PET cohort and the 970-IEO sub-cohort, and the panel of 13 cell lines (see Cell lines and treatments) were subjected to transcriptomic profiling by RNAseq. Briefly, total cellular RNA was extracted and the quality was assessed using the Bioanalyzer 2100 (Agilent). For each sample, total RNA was depleted of ribosomal RNA and the RNAseq libraries were prepared with the Illumina TruSeq Stranded Total RNA kit following the manufacturer's protocol. Following adapter ligation, libraries were amplified by PCR. Amplified libraries were checked on a Bioanalyzer 2100 and quantified with picogreen reagent (Invitrogen) and sequenced for 100 bases in the paired-end mode with 50 million reads coverage on a Novaseq 6000 sequencer. Raw data were acquired for all datasets, and the human reference genome (hg38) was employed as the alignment template for mapping the reads through Bowtie2 (version 2.4.5) [30]. The estimation of gene expression abundance was carried out using RSEM (version 1.3.3) with default parameters [31].

**Publicly available datasets**

The TCGA BC dataset was downloaded from the cBioPortal (http://www.cbioportal.org/) [21, 22] (TCGA Breast Invasive Carcinoma. Source data from GDAC Firehose. Previously known as TCGA Provisional). Only 896 M0 patients (896 cases) were analyzed, available as RSEM upper quartile normalized counts.

The METABRIC dataset (1904 samples) was obtained through the cBioPortal (2019 freeze, available at https://github.com/cBioPortal/datahub/tree/master/public/brca_metabric) [32, 33]. Data were available as normalized log2 intensity values.

Raw RNASeq data for the DCIS dataset [34] were downloaded from the GEO database, accession number GSE69994.

BC cell lines RNASeq and metabolomics data were obtained from the Cancer Cell Line Encyclopedia (CCLE) collection (https://sites.broadinstitute.org/ccle/datasets) [23]. Cell line metabolomics data were available as log10 transformed data.

When raw RNASeq data were available, they were processed with RSEM (version 1.3.3) using Bowtie2 (version 2.4.5) as aligner and the human genome (hg38) as reference.

**Data analysis and statistical methods**

*Differential expression analysis between SUV-H and SUV-L tumors*

Following RNAseq, RNA counts were measured using the RESM software (version 1.3.3), and the unprocessed data were brought into the EdgeR package within the R software (version 3.40.2) [35]. Using default parameters, after filtering for not expressed or low expressed genes, library sizes were normalized and statistical analyses between groups were performed with the quasi-likelihood F-tests (QLF). Differentially expressed genes were obtained and the p-value adjusted with the Benjamini and Hochberg methodology to obtain the FDR (False Discovery Rate). Only genes with an FDR p-value $< 0.05$ and a fold-change $\geq 2.5$ were considered significant.

Three genes, encoding ribosomal RNAs were excluded, as they probably represented contaminants, to yield the 135 initial gene list.

*Data normalization, hierarchical clustering and survival analysis*

For hierarchical clustering and generation of heatmaps, when raw read counts were available, data (RNAseq data) were processed for TMM normalization with the EdgeR R package (version 3.40.2) and the Z-Score was normalized using JMP software version 14.3 (version used for all analyses performed in JMP; SAS Institute Inc., Cary, NC, 1989-2023). Data that had previously been normalized (specifically, cBioPortal TCGA data) were log transformed and mean-centered normalized. Metabolomics data, represented as log10 intensity values, or METABRIC gene expression data, represented as normalized log2 intensity, were solely subjected to mean-centering. All Distance-based Dendrograms were created using the Ward's method in cluster analysis within JMP. Each sample cluster was categorized as either SUV-H-like or SUV-L-like based on the gene expression pattern of the 54 genes of PETsign.

Kaplan-Meier analyses, univariate and multivariable survival analyses were performed within JMP, employing the Survival platform and the Cox proportional hazards model, as appropriate.

For the analyses involving the Cox proportional hazards regression model of the entire METABRIC dataset, shown in Table 4, the expression of each gene was categorized as HIGH or LOW with respect to the mean expression across all samples. Following this categorization, data were analyzed with the 'survival' package in R, version 3.5-5. The 'coxph' function was employed to determine HR (hazard ratio) and p-values for the univariate analysis (see also the legend of Table 4 for further details).

*Other statistical analyses*

For Seahorse measurements, statistics were calculated by using SigmaPlot 14.0. All results are representative of at least 3 independent experiments and are presented as the mean + SD. Significance was calculated by the ANOVA (one-way or two-way) test. P-values $< 0.05$ were considered statistically significant.

For the glucose uptake experiments, statistical analyses were performed using the two-tailed unpaired *t*-test. The results are expressed as mean $\pm$ standard error (SE).

**RESULTS**

**Example 1**

**A molecular signature that recapitulates the SUVmax status of BCs**

We analyzed patients who underwent FDG-PET at the European Institute of Oncology (IEO, Milan, Italy) prior to surgery. Patients were classified based on their SUVmax, which has been shown to reflect tumor aggressiveness in different types of cancer, and, in some cancers, to represent an independent prognostic factor [36-40]. Two groups of patients were selected: SUV-High (SUV-H, 57 patients) and SUV-low (SUV-L, 63 patients), displaying a SUVmax of $> 10$ and $< 5$, respectively. Collectively, we refer to these patients as the 120-PET cohort (Table 1). Formalin-fixed paraffin-embedded (FFPE) specimens, obtained after surgery from the same patients, were subjected to RNAseq. Comparison of the transcriptomic profiles led to the identification of 135 differentially expressed genes of which 73 were upregulated (FDR < 0.05, p < 0.05, FC > 2.5) and 62 were downregulated (FDR < 0.05, p < 0.05, FC < 0.4) in SUV-H vs. SUV-L tumors (Fig. 1A and Table 5). Of the 135 genes, 28 represented ncRNAs and 107 were coding RNAs (Table 6). Surprisingly, only a fraction of the coding genes could be attributed with clear "metabolic functions". Instead, there was a preponderance of genes connected with signaling, adhesion, and membrane transport, while a sizable fraction of the upregulated genes was immune/inflammation-related (Fig. 1B). The expression of these genes was most likely ascribable to the epithelial component of the tumor, since bioinformatics analyses revealed very limited contamination of the samples with immune and/or stromal cells (Tables 7 and 8).

**Example 2**

**Identification of PETsign and its prognostic value in BC**

To explore the potential clinical utility of the identified genes, we used the publicly available METABRIC database, comprising ~2000 BC transcriptomic profiles (Table 2) [32, 33]. Of the 135 genes, 99 were present in the dataset (most of the absent genes were ncRNAs). Fifty-four of the 99 genes correlated with worse prognostic outcome in univariate analysis (32 upregulated, 22 downregulated, Table 4). We calculated whether there was significant enrichment of genes predicting prognosis in univariate analysis in the 99-gene list as compared to the entire METABRIC dataset. From the 24,368 "genes" listed in the dataset, at the time of the analysis, 6632 (27.22%) were predictive of prognosis in univariate analysis, compared to 54/99 in the PET list (54.55%), when a p of 0.05 was used. By adopting a more stringent p-value of 0.01, 4336 of 24,368 genes were predictive (17.38%), *vs.* 45 of 99 (45.45%) genes from the PET list. This represents a 2-fold and a 2.6-fold increase in prognostic genes in the PET *vs.* METABRIC gene list, respectively. In both cases, the difference in the frequency of prognostic genes between the two gene lists was highly significant: P < 0.00001 by Fischer's exact tes. Henceforth, we will refer to the 54 genes as the "PET signature" (PETsign).

The 54 genes of PETsign could readily separate the tumors of the METABRIC dataset into a SUV-H-like or a SUV-L-like molecular phenotype (Fig. 1C, Table 9). The SUV-H-like phenotype correlated with aggressive disease in univariate (HR, 2.09; p < 0.0001) and multivariable (HR, 1.62; p < 0.0001) analysis (Fig. 1D, Table 9, see Table 2 for the variables used in the multivariable analysis).

Thus, PETsign is a strong independent prognostic marker of worse disease outcome.

When PETsign was applied to individual molecular subgroups of BC, it correlated with poor prognosis in Luminal BCs (HR+, HER2-) (Table 9).

**Example 3**

**Independent validation of PETsign**

The definition of the 54-gene PETsign relied on the selection of genes (from the 99 genes present in the METABRIC database) that were individually prognostic in univariate analysis. Thus, the results obtained with the complete PETsign in the METABRIC database might be affected by overfitting. Therefore, to determine the robustness of PETsign as a prognostic predictor, we interrogated two independent BC cohorts.

Initially, we employed the TCGA BC dataset [41, 42]. This dataset, despite high quality molecular data, suffers from poor clinical follow-up (median follow-up, 3.58 years, Table 2). For this reason, we limited our analysis to a 5-year follow-up period. In this timeframe, in the TCGA dataset, PETsign was an independent predictor of prognosis in univariate (HR, 2.32; p=0.0004) and multivariable (HR, 2.18; p=0.0059) analysis (Fig. 2A-B; Table 9).

To obtain an independent validation on a higher quality clinical cohort, we used the IEO cohort: a consecutive cohort of ~2000 BC patients who underwent surgery at the IEO in the years 1997-2000 (Table 3) [26, 27, 43]. We selected 970 cases from this cohort representative (matched) of the entire cohort (Table 3) and performed transcriptomic profiling by RNAseq. PETsign could readily stratify this cohort into a SUV-H-like and a SUV-L-like molecular phenotypes (Fig. 2C); the SUV-H-like phenotype correlated with poor prognosis in univariate (HR. 2.74, p<0.0001) and multivariable (HR, 2.27, p<0.0001) analysis (Fig. 2D, Table 9). Similar to the METABRIC cohort, when PETsign was applied to individual BC molecular subgroups in the 970-IEO sub-cohort, it correlated with poor prognosis in Luminal BCs, in particular Luminal B BCs, which represented the most frequent type of BC (Table 9).

We concluded that PETsign is an independent prognostic indicator of poor clinical outcome across three independent case collections generated at different institutes, in different countries, and using different technological platforms for transcriptomic profiling analysis.

**Example 4**

**PETsign is an *ab initio* signature of aggressive disease course**

To gain insights into why PETsign was prognostic only in Luminal BCs, we analyzed the distribution of SUV-H-like tumors between the different BC molecular subtypes in the three clinical cohorts. The HER2 and TNBC subtypes that have a more aggressive disease course were essentially all SUV-H-like (Fig. 3A-B). Conversely, Luminal (HR+) tumors displayed variable composition, with a higher representation of the SUV-H-like phenotype in BCs with more aggressive clinical behavior; i.e., the percentage of SUV-H-like tumors was higher in Luminal-HER2 vs. Luminal subtype (Fig. 3A-B). In the IEO cohort, where the availability of Ki-67 staining (a proliferation marker) allowed further stratification of Luminal BCs, the percentage of the SUV-H-like phenotype correlated again with increased clinical aggressiveness: Luminal HER2 > Luminal B > Luminal A (Fig. 3B). We concluded that the SUV-H-like phenotype is a characterizing feature of aggressive BC subtypes (HER2, TNBC), while being present only in a fraction of Luminal (HR+) BCs, where it is associated with worse disease outcome.

These data raised the question of whether the SUV-H-like phenotype appears *ab initio* or whether it is acquired as BCs progress in their natural history. To gain insights into this issue, we investigated a published cohort of ductal *in situ* carcinomas (DCIS) [34]. DCIS are considered to be the earliest form of BC; they are non-invasive/pre-invasive BCs confined to the lumen of the duct which have not breached the basement membrane. The condition is not considered life-threatening, as many DCIS do not progress to invasive BCs, although some do [44]. In the analyzed cohort, PETsign readily stratified a SUV-H-like and a SUV-L-like phenotype (Fig. 3C). These data indicate that the SUV-H-like and SUV-L-like molecular phenotypes are present from the earliest stage of BC development (DCIS), arguing that PETsign is an *ab initio* signature of the metabolic state of BCs. In addition, most of the SUV-H-like DCIS were HR (ER/PGR)-negative, similarly to invasive BCs (Fig. 3C).

**Example 5**

**Genes of the PETsign are frequently co-amplified in BC**

In principle, the alterations in gene expression detected by PETsign could either be causal of the tumorigenic process or reflect a consequence of tumorigenesis, for instance distinct cellular targets of transformation. In the former case, one might find genetic alterations of PETsign genes in SUV-H-like vs. SUV-L-like BCs. By interrogating the TCGA and the METABRIC databases, we uncovered that the PETsign genes are rarely mutated in BC and no significant recurrent mutations were identified. However, analysis of the gene copy number in the TGCA database, revealed that 11 PETsign genes displayed an evident pattern of co-amplification in groups (Fig. 4A). For this analysis, we considered only genes that were amplified in at least 1.5% of cases, showed a pattern of co-occurrence with another amplified gene with a stringent q-value of <0.001, and displayed congruent upregulation of expression in PETsign (Table 10). An additional search of all the remaining upregulated genes identified in the original list of 135 genes differentially expressed in SUV-H and SUV-L tumors (shown in Tables 5 and 6), led to the identification of a further 7 genes showing patterns of co-amplification (Fig. 4A, Table 10). Similar amplification patterns were obtained for the 18 identified genes in the METABRIC database, with the exception of CXCL8 for which no amplification data was retrievable (Fig. 5A). Overall, the 18 identified genes were cumulatively amplified in 27-38% of all BCs (235/879 = 27%, in the TCGA, Fig. 4A-B; 725/1903 = 38% in the METABRIC, Fig. 4B, Fig. 5A). Interestingly, when the analysis was widened to include mRNA overexpression in addition to the amplification of the 18 genes, ~56% of BCs (504/896 in the TCGA) showed one or both alterations (Fig. 5B).

We next asked whether there was a correlation between the SUV-like molecular phenotype of BCs and the amplification of these 18 genes. We found that a higher percentage of SUV-H-like BCs display amplification of one or more of the 18 genes compared with SUV-L-like BCs (Fig. 4B). In detail, four groups of co-amplified genes could be distinguished when the highest stringency criterion (q-value for co-occurrence < 0.001) was employed (Fig. 4C-F and Table 10). Three of the four groups were comprised of genes localized on the same chromosome: 4q, 7p or 3q (Fig. 4C-E). The fourth group included genes from two different chromosomes (Fig. 4F).

The impact of these co-amplifications on breast tumorigenesis remains to be established. In particular, the question remains as to whether some of them represent "passenger" amplifications that witness the key amplification of one of the genes in the same amplicon. Regardless, the frequent amplification of genes of the PETsign in BCs argues for a role in the determination of the transformed phenotype, possibly linked to the high metabolic state of SUV-H-like BCs and to their increased clinical aggressiveness.

**Example 6**

**PETsign stratifies BCs by their metabolic state**

To gain insights into the biological basis of PETsign, we performed a network analysis using the STRING database [45]. Of the 54 PETsign proteins, 22 could be placed into a network centered around two major hubs, represented by CXCL8 (C-X-C motif chemokine ligand 8, also known as IL8) and EGFR (epidermal growth factor receptor) (Fig. 6A). The CXCL8 sub-network was enriched in proteins involved in chemokine signaling pathways (CSF2RB, CXCL8, CXCL10, CXCL11, IFI27, IL33), while the EGFR sub-network was enriched in proteins connected with signaling from the cell surface or hormonal response (FOLH1, EGFR, NTRK2, SLITRK5, CDH3, PGR). Thus, this network analysis highlighted an unexpected feature of PETsign, i.e., that its ability to stratify SUV-H-like and SUV-L-like BCs (and to be prognostic of disease outcome) seems to be correlated with signaling activation in BCs, rather than with direct alterations in the level of expression of metabolic genes, as also hinted by our initial analysis of the 135 genes differentially expressed between SUV-H and SUV-L BCs (Fig. 1B).

This finding raised the question of whether PETsign stratifies BCs by their metabolic state. To answer to this, we used a panel of BC cell lines for which transcriptomic and metabolomic data are available from public databases [23, 24]. PETsign was used to assign the cell lines to a SUV-H-like or SUV-L-like molecular phenotype (Fig. 7). By comparing the levels of metabolites in SUV-H-like vs. SUV-L-like cell lines, we identified a group of metabolites that were significantly associated with the SUV-H-like molecular phenotype (Fig. 6B and Table 11). For example, the typical oncometabolites, lactate, 2-hydroxyglutarate, and glutamate were increased in SUV-H-like *vs.* SUV-L-like BC cell lines (Fig. 6C). Of note, increased lactate production is a hallmark of the Warburg effect. In contrast, carnitine and several of its metabolites were decreased in SUV-H-like *vs.* SUV-L-like BC cell lines (Fig. 6B and Table 11), and the ratio acetylcarnitine/carnitine was significantly decreased in SUV-H-like cell lines, arguing that fatty acid oxidation is less active in these cell lines.

In essence, SUV-H-like cell lines appear to rely more heavily on aerobic glycolysis, while SUV-L-like lines might depend more on fatty acid oxidation, a point we will comment further on later. Accordingly, unsupervised clustering revealed that the differentially produced metabolites can cluster BC cell lines in concordance with their SUV-like status (Fig. 6D). Thus, PETsign directly correlates with the metabolic state of BC cell lines.

### Example 7

### Biological relevance of PETsign

We have shown that there is a link between PETsign and the metabolic state of BC cell lines (Fig. 6B-D), but not causation. To do that, it would be necessary to demonstrate that activation of the signaling pathways identified by PETsign (most notably CXCL8 and EGFR, see Fig. 6A) can convert cells to a glycolytic phenotype. This type of evidence can be more easily obtained in cell lines. Cell lines, however, are adapted to culture and do not necessarily recapitulate the conditions occurring in real cancers; therefore, we searched for an "ideal" model that should be represented by a SUV-L-like BC cell line, displaying low glycolytic metabolism, yet potentially responsive to activation of the CXCL8 or the EGFR signaling pathways to measure possible metabolic modifications, upon their activation. Since cell lines are known to vary inter-laboratory [46, 47], we did not rely on publicly available datasets but performed a through in-house characterization of a panel of 13 cell lines, representative of all molecular subtypes of BC.

We performed RNAseq of the entire panel and extracted relevant information. This allowed to classify the 13 cell lines according to their SUV-like phenotype (Fig. 8A).

We investigated the status of the CXCL8 and the EGFR pathways in the 13 BC cell lines. Eight lines displayed variable expression of the CXCL8 receptors, CXCR1 and/or CXCR2, and are thus potentially capable of responding to CXCL8 (Fig. 9A). When these 8 cell lines, were tested for autocrine CXCL8 production, we detected excellent concordance between secretion (Fig. 9B) and mRNA levels (Fig. 9C). Of note, 4 cell lines (BT-474, T47D, MCF-7, and MDA-MB-453) secreted negligible levels of CXCL8, while expressing the cognate receptors. These cell lines represent, therefore, suitable candidates to perform CXCL8 stimulation experiments in the absence of the confounding factor of endogenous CXCL8 secretion.

Next, we analyzed the metabolic status of the 8 cell lines by Seahorse. The extracellular acidification rate (ECAR) was used to identify the cell lines displaying lower glycolytic metabolism, i.e., MDA-MB-468, MDA-MB-453, T47D and BT-474 (Fig. 9D). Finally, we investigated the levels of expression and the state of endogenous activation of the EGFR. Receptor activation was evaluated by measuring the levels of phosphorylation with anti-phosphoEGFR specific antibodies (Fig. 9E and Fig. 8B). Three of the cell lines (MCF7, T47D, and MDA-MB-453) displayed low normal-like levels of total EGFR and low levels of constitutive activation (Fig. 9F), thus are suitable for EGFR stimulation experiments with EGF in the absence of endogenous EGFR activation.

Based on the cell line characterization, we selected MDA-MB-453 as the most suitable cell line to analyze the effects of CXCL8 and EGFR signaling activation on the metabolic status of cells. We also selected T47D cells, despite their low expression of CXCR1/2, as an alternative cell line (Fig. 9G, see figure legend for more details).

We measured the uptake of 2-deoxy-glucose in MDA-MB-453 and T47D cells upon stimulation with EGF or CXCL8. MDA-MB-453 responded to both ligands with a significant increase of 2-deoxy-glucose transport across the cellular membrane (Fig. 10A). T47D cells responded to EGF with increased uptake of 2-deoxy-glucose, while treatment with CXCL8 did not elicit significant effects (Fig. 10A), probably due to the low expression levels of the CXCR1/2 receptors (see Fig. 9A). In MDA-MB-453 cells, which responded to both CXCL8 and EGF, the effects were already evident at low concentrations of ligand (EGF 1 ng/ml, CXCL8,10 ng/ml) and were only marginally increased by 10-100-fold increases of ligands (Fig. 10A). This prompted us to investigate the possible cooperation (or lack thereof) of the two pathways. When MDA-MB-453 cells were triggered with both ligands (Fig. 10B), they did not show any additive effect vs. treatment with individual ligands. This argues for redundant effects of the CXCL8 and EGF signaling pathway on 2-deoxy-glucose uptake, possibly due to the fact that both CXCR1/2 and EGFR impinge on the same downstream pathways leading to the observed phenotype.

We next asked whether EGF and/or CXCL8 could stimulate aerobic glycolysis, the hallmark of the Warburg effect. Results mirrored those obtained with 2-deoxy-glucose uptake. EGF stimulation of both MDA-MB-453 and T47D increased ECAR (Fig. 10C-D). In addition, the oxygen consumption rate (OCR, indicative of OXPHOS) was decreased by EGF stimulation in MDA-MB-453 cells and left unchanged in T47D cells (Fig. 10C-D), resulting in an increased ECAR/OCR ratio, indicative of a shift towards glycolysis in both cell lines. CXCL8 increased ECAR in MDA-MB-453 cells, but showed no effect in T47D cells (Fig. 10C-D). There was no effect of CXCL8 on OCR in both lines (Fig. 10C-D). Thus, at least in MDA-MB-453 cells, CXCL8 caused a shift towards a more glycolytic metabolism.

The sum of these results is consistent with the idea that at least some of the genes present in the PETsign contribute, in a cancer-cell autonomous fashion, to the altered metabolic state of some BCs characterized by increased glycolysis

## DISCUSSION

Through a transcriptomic analysis of a cohort of BC patients who underwent FDG-PET prior to treatment, we derived the 54-gene PETsign that stratifies tumors by their SUVmax status (high or low). In three large independent BC cohorts, PETsign provided independent prognostic information in the most frequent and most heterogeneous subtype of BC, the Luminal subtype, for which improved stratification criteria are required [16].

Our findings are relevant to a number of biological and clinical issues. Firstly, does PETsign predict the metabolic status of BCs? The increased glucose uptake revealed by FDG-PET is assumed to be a proxy for enhanced glycolytic flux. This is a reasonable assumption and supported by several observations, which are, however, based on metabolic data obtained in cell lines or on genomic/transcriptomic data obtained in real BCs (reviewed in [48]). Recent efforts have been directed at obtaining multiomics representations of human BCs. For instance, Gong *et al.* demonstrated that transcriptomic analysis of TNBC could identify three distinct "metabolic" subtypes: glycolytic, lipogenic and mixed. These subtypes were validated by metabolomic profiling of a number of TNBCs [49].

While we did not perform orthogonal transcriptomic/metabolomic profiling of real BCs, we believe that a cogent case can be made that PETsign actually "reads" the metabolic status of BCs. In a sizable panel of BC cell lines, PETsign could readily identify SUV-H-like and SUV-L-like molecular phenotypes. These two phenotypes associated with distinct metabolic profiles. In particular, SUV-H-like cell lines displayed increased production of lactate (the hallmark of the Warburg effect) vs. SUV-L-like cell lines. Conversely, carnitine metabolism was decreased in SUV-H-like lines, arguing for reduced reliance on fatty acid oxidation in this group. In addition, activation of signaling pathways involving PETsign genes induced *in vitro* alterations indicative of enhanced glycolytic flux. The sum of these observations argues that PETsign can identify BCs displaying metabolic reprogramming to aerobic glycolysis.

Secondly, is the presence of PETsign linked to progressive adaptations of individual tumors to the changing metabolic demands imposed on the growing tumor mass (e.g., local conditions of hypoxia, depletion of nutrients and so on, reviewed in [48]) or does it characterize some BCs *ab initio*? In a cohort of DCIS, which represent the earliest form of BC detectable *in vivo,* PETsign could stratify the cohort into SUV-H-like and SUV-L-like groups, arguing for an *ab initio* origin of the alterations read by PETsign.

Linked to the previous question, do any of the PETsign genes have causal roles in determining aerobic glycolysis in BCs or are they simply regulated downstream of other alterations that are driving metabolic reprogramming? Our data are in support of causality, as activation of both CXCL8 or EGFR signaling pathways induced metabolic modifications characteristic of aerobic glycolysis in a cell-autonomous fashion. Moreover, clear patterns of co-amplification of groups of the PETsign genes could be evidenced, preferentially in SUV-H-like BCs, again arguing for causality as opposed to consequence. This, of course, does not imply that all genes in the PETsign are causal: the signature likely reflects a mixture of causal and "consequential" genes. Similarly, it does not imply that all genes confer cell-autonomous properties and/or advantages, as they might also be involved in the establishment of tumor-stroma interactions, which are rampant in cancer and in BC in particular [48].

An additional question concerns what we can learn from the gene composition of the PETsign.. We list here some of the papers analyzing individual genes (or small groups of genes) from the PETsign with no claim to comprehensiveness.

UP genes: ADM [50, 51], AQP9 [52], CDCA7 [53], CDH3 [54], CENPW [55], CP [56, 57], CSF2RB [58], CXCL10 [59, 60], CXCL11 [59, 61], CXCL8 [62-66], EGFR (see for instance [67-70] from a wealth of papers on the subject), GALNT14 [71], LAMP3 [72], MCM10 [73], ME1 [74, 75], NDUFA4L2 [76], PIR [77], PSAT1 [78-80], RARRES1 [81], S100A9 [82-85], SEC61G [86], SOX11 [87, 88], SPP1 [89-92].

DOWN genes: ABCA10 [93], ELOVL2 [94], FOLH1 [95], FREM1 [96], NAV3 [97], PIEZO2 [98], STC2 [99-101].

A noteworthy feature of PETsign was the abundance of signaling molecules or PM-located proteins, including cytokines, various PM receptors, adhesion molecules and PM-located carriers/transporters. This raises the intriguing possibility that key events connected with the establishment of aerobic glycolysis are initiated by signaling alterations, as also supported by our biological experiments.

PETsign was developed for application on FFPE specimens and by RNAseq, which is quickly becoming routine in cancer diagnosis. In addition, the signature appears robust, across institutes, countries of origin of patients, and technological platforms; thus, it might find application in several clinical settings. First, it could be used for prognostic stratification, either on its own or by integrating existing molecular tools [11, 12, 14]. PETsign might also aid in patient stratification for clinical trials with anti-glycolytic drugs. Although no such drug has been approved yet in BC, there are several in preclinical phases and clinical trials (see [102, 103] and references therein). PETsign could represent a useful tool, acting as an FDG-PET-surrogate, to gather information on the metabolic status of BCs, in the initial phases of the diagnostic/stratification process even in those patients in which FDG-PET is not indicated for imaging/diagnostic purposes.

## TABLES

**Table 1. Clinicopathological characteristics of the patients of the 120-PET cohort.**

| | N | SUV-Low (N) | SUV-High (N) | SUV-Low (%) | SUV-High (%) | $X^2$ p-Value |
|---|---|---|---|---|---|---|
| Samples | 120 | 63 | 57 | 52.50% | 47.50% | |
| Age <50 | 69 | 32 | 37 | 46.38% | 53.62% | 0.12 |
| Age ≥50 | 51 | 31 | 20 | 60.78% | 39.22% | |
| pT1-2 | 91 | 48 | 43 | 52.75% | 46.15% | 0.92 |
| pT3 | 29 | 15 | 14 | 51.72% | 48.28% | |
| M0 | 112 | 57 | 55 | 50.89% | 49.11% | 0.19 |
| M1 | 8 | 6 | 2 | 75.00% | 25.00% | |
| pN Neg | 21 | 6 | 15 | 28.57% | 71.43% | 0.13 |
| pN Pos | 98 | 57 | 41 | 58.16% | 41.84% | |
| HR Pos | 98 | 56 | 42 | 57.14% | 42.86% | 0.03 |
| HR Neg | 22 | 7 | 15 | 31.82% | 68.18% | |
| HER2 AMP | 22 | 10 | 12 | 45.45% | 54.55% | 0.47 |
| HER2 WT | 98 | 53 | 45 | 54.08% | 45.92% | |
| KI67 <15% | 9 | 7 | 2 | 77.78% | 22.22% | 0.11 |
| KI67 ≥15% | 111 | 56 | 55 | 50.45% | 49.55% | |

**Table 1.** Comparison of the clinicopathological characteristics of the patients of the 120-PET cohort. SUV-High, patients whose tumor displayed a SUVmax of > 10; SUV-Low, patients whose tumor displayed a SUVmax of < 5. P-values were assessed by the chi-square tests of significance with JMP.

**Table 2. Characteristics of the three clinical cohorts employed in the study.**

| Cohort | Cases (N) | Clinical and Pathological Parameters | Endpoint | Average Follow-up |
|---|---|---|---|---|
| METABRIC * | 1904 | Age, TNM, HR (ER/PGR), HER2, Grade | DRBC | 10.21 years |
| TCGA ** | 896 | Age, TNM, HR (ER/PGR), HER2 | OS | 3.58 years |
| IEO *** | 970 | Age, TNM, HR (ER/PGR), HER2, Grade, Ki-67 | DRBC | 12.45 years |

**Table 2.** The three cohorts of BC patients utilized in this study are shown. The METABRIC cohort corresponds to the 1904 cases present in the cBioPortal and downloadable from it. There is no information in this cohort about whether the patients were M0 or M1 at the time of diagnosis. The TCGA cohort corresponds to 896 cases out of 1108 present in the TCGA Firehose Legacy dataset and downloadable from the cBioPortal. The 896 cases represent the M0 cases from the cohort. The IEO cohort comprises 970 patients, all M0, from a larger cohort as described in Table 3. For all cohorts, the table shows the clinicopathological parameters available for multivariable analysis and the endpoint for prognostic analyses (DRBC, death related to BC; OS, overall survival).

\* The METABRIC case collection was assembled from tumor banks in the UK and Canada. [32]. RNAs were extracted from fresh-frozen breast samples. Transcriptomics analysis was performed on the Illumina HumanHT-12 v3.0 Gene Expression BeadChip.

\*\* The Cancer Genome Atlas (TCGA) was a joint effort of the National Cancer Institute (NCI) and the National Human Genome Research Institute (NHGRI), which are both part of the National Institutes of Health, U.S. Department of Health and Human Services [9]. Tissue samples were accrued from a number of institutions, mainly based in the US, but also in Russia, Poland and Vietnam. For Breast samples, RNAs were extracted from fresh-frozen tissues. Transcriptomics analysis was performed on the Illumina HiSeq 2000 System.

\*\*\* The IEO cohort is a single institution consecutive cohort collected at the European Institute of Oncology in Milan Italy between years 1997 and 2000 [26, 27]. A sub-cohort, matched to the entire cohort (see Table 3) was subjected to RNAse for the purpose of the present study. RNAs were extracted from FFPE specimens, as detailed in the main text. Transcriptomics analysis was performed on the Novaseq 6000 sequencer.

**Table 3. Clinicopathological characteristics of the complete IEO cohort and the 970-patient sub-cohort.**

| | Complete cohort | | 970-Patient sub-cohort | | |
|---|---|---|---|---|---|
| Category | Number | % | Number | % | $\chi^2$ p-Value |
| Age <50 | 911 | 39.34% | 368 | 37.94% | |
| Age ≥50 | 1405 | 60.67% | 602 | 62.06% | |
| Age Total | 2316 | 100.00% | 970 | 100.00% | 0.45 |
| T1 | 1493 | 64.47% | 616 | 63.51% | |
| T2 | 744 | 32.12% | 322 | 33.20% | |
| T3 | 61 | 2.63% | 22 | 2.27% | |
| T4 | 18 | 0.78% | 10 | 1.03% | |
| T Total | 2316 | 100.00% | 970 | 100.00% | 0.75 |
| pN NEG | 1124 | 49.71% | 462 | 48.63% | |
| pN POS | 1137 | 50.29% | 488 | 51.37% | |
| pN Total | 2261 | 100.00% | 950 | 100.00% | 0.74 |
| pN Missing | 55 | | 20 | | |
| Grade G1 | 427 | 18.88% | 182 | 19.16% | |
| Grade G2 | 1009 | 44.61% | 414 | 43.58% | |
| Grade G3 | 826 | 36.52% | 354 | 37.26% | |
| Grade Total | 2262 | 100.00% | 950 | 100.00% | 0.92 |
| Grade Missing | 54 | | 20 | | |
| PGR NEG | 647 | 27.94% | 281 | 28.97% | |
| PGR POS | 1669 | 72.06% | 689 | 71.03% | |
| PGR Total | 2316 | 100.00% | 970 | 100.00% | 0.55 |
| ER NEG | 345 | 14.90% | 146 | 15.05% | |
| ER POS | 1971 | 85.10% | 824 | 84.95% | |

(continued)

| Category | Complete cohort | | 970-Patient sub-cohort | | |
| --- | --- | --- | --- | --- | --- |
| | Number | % | Number | % | χ² p-Value |
| ER Total | 2316 | 100.00% | 970 | 100.00% | 0.91 |
| Ki67 <14% | 653 | 28.22% | 279 | 28.79% | |
| Ki67 >14% | 1661 | 71.78% | 690 | 71.21% | |
| Ki67 Total | 2314 | 100.00% | 969 | 100.00% | 0.74 |
| Ki67 Missing | 2 | | 2 | | |
| HER2 (IHC) NEG | 1826 | 87.83% | 790 | 87.58% | |
| HER2 (IHC) POS | 253 | 12.17% | 112 | 12.42% | |
| HER2 (IHC) Total | 2079 | 100.00% | 902 | 100.00% | 0.85 |
| HER2 (IHC) Missing | 237 | | 68 | | |
| LUMINALA | 432 | 18.82% | 184 | 19.05% | |
| LUMINAL B | 1414 | 61.59% | 587 | 60.77% | |
| LUMINAL B HER2 POS | 152 | 6.62% | 66 | 6.83% | |
| HER2 | 101 | 4.40% | 46 | 4.76% | |
| TNBC | 197 | 8.58% | 83 | 8.59% | |
| Subtype Total | 2296 | 100.00% | 966 | 100.00% | 0.99 |
| Subtype Missing | 20 | | 4 | | |
| Event Distant | 346 | 14.94% | 150 | 15.46% | |
| Event Locoregional | 182 | 7.86% | 81 | 8.35% | |
| No Event | 1421 | 61.36% | 567 | 58.45% | |
| Other | 367 | 15.85% | 172 | 17.73% | |
| Event Total | 2316 | 100.00% | 970 | 100.00% | 0.43 |
| Death Other | 44 | 1.90% | 23 | 2.37% | |
| Death Other Tumor | 49 | 2.12% | 26 | 2.68% | |
| Death ictus | 28 | 1.21% | 13 | 1.34% | |
| Death Primary Tumor | 351 | 15.16% | 154 | 15.88% | |
| Alive | 1844 | 79.62% | 754 | 77.73% | |
| Total | 2316 | 100.00% | 970 | 100.00% | 0.67 |

**Table 3.** The clinicopathological characteristics of the complete IEO cohort (N=2316) and the 970-patient IEO sub-cohort is reported. The complete IEO has been previously described [26, 27]. Transcriptomic profiling by RNAseq was performed on the 970-patient sub-cohort. Statistical analysis (chi-square tests with Excel) shows that the sub-cohort is matched to the entire consecutive cohort.

**Table 4. Definition of the PETsign.**

| Gene Name | Reg. | Approved Name | HR | P |
| --- | --- | --- | --- | --- |
| **ADAMDEC1** | UP | ADAM like decysin 1 | 1.36 | <0.001 |
| **ADM** | UP | adrenomedullin | 1.48 | <0.001 |
| **AQP9** | UP | aquaporin 9 | 1.71 | <0.001 |
| **C5orf46** | UP | chromosome 5 open reading frame 46 | 1.22 | 0.024 |
| **CDCA7** | UP | cell division cycle associated 7 | 1.64 | <0.001 |

(continued)

| Gene Name | Reg. | Approved Name | HR | P |
|---|---|---|---|---|
| CDH3 | UP | cadherin 3 | 1.29 | 0.004 |
| CENPW | UP | centromere protein W | 1.42 | <0.001 |
| CP | UP | ceruloplasmin | 1.43 | <0.001 |
| CSF2RB | UP | colony stimulating factor 2 receptor subunit beta | 1.22 | 0.012 |
| CXCL10 | UP | C-X-C motif chemokine ligand 10 | 1.43 | <0.001 |
| CXCL11 | UP | C-X-C motif chemokine ligand 11 | 1.24 | 0.020 |
| CXCL8 | UP | C-X-C motif chemokine ligand 8 | 1.55 | <0.001 |
| EGFR | UP | epidermal growth factor receptor | 1.55 | <0.001 |
| GABRE | UP | gamma-aminobutyric acid type A receptor subunit epsilon | 1.21 | 0.040 |
| GALNT14 | UP | polypeptide N-acetylgalactosaminyltransferase 14 | 1.29 | 0.002 |
| IFI27 | UP | interferon alpha inducible protein 27 | 1.27 | 0.004 |
| LAMP3 | UP | lysosomal associated membrane protein 3 | 1.26 | 0.009 |
| MCM10 | UP | minichromosome maintenance 10 replication initiation factor | 1.88 | <0.001 |
| ME1 | UP | malic enzyme 1 | 1.30 | 0.002 |
| MFSD2A | UP | major facilitator superfamily domain containing 2A | 1.43 | <0.001 |
| NDUFA4L2 | UP | NDUFA4 mitochondrial complex associated like 2 | 1.30 | 0.006 |
| PIR | UP | pirin | 1.36 | <0.001 |
| PLCH1 | UP | phospholipase C eta 1 | 1.34 | <0.001 |
| PSAT1 | UP | phosphoserine aminotransferase 1 | 1.49 | <0.001 |
| RARRES1 | UP | retinoic acid receptor responder 1 | 1.29 | 0.008 |
| S100A9 | UP | S100 calcium binding protein A9 | 1.56 | <0.001 |
| SEC61G | UP | SEC61 translocon subunit gamma | 1.29 | 0.002 |
| SHISA2 | UP | shisa family member 2 | 0.83 | 0.048 |
| SLITRK5 | UP | SLIT and NTRK like family member 5 | 1.27 | 0.006 |
| SOX11 | UP | SRY-box transcription factor 11 | 1.80 | <0.001 |
| SPP1 | UP | secreted phosphoprotein 1 | 1.37 | <0.001 |
| TMEM158 | UP | transmembrane protein 158 | 1.27 | 0.007 |
| ABCA10 | DOWN | ATP binding cassette subfamily A member 10 | 0.74 | <0.001 |
| CCDC85A | DOWN | coiled-coil domain containing 85A | 0.66 | <0.001 |
| CKB | DOWN | creatine kinase B | 1.44 | <0.001 |
| CYP2A7 | DOWN | cytochrome P450 family 2 subfamily A member 7 | 0.61 | 0.013 |
| ELOVL2 | DOWN | ELOVL fatty acid elongase 2 | 0.67 | <0.001 |
| ERICH3 | DOWN | glutamate rich 3 | 0.54 | <0.001 |
| FOLH1 | DOWN | folate hydrolase 1 | 1.34 | <0.001 |
| FREM1 | DOWN | FRAS1 related extracellular matrix 1 | 0.84 | 0.034 |
| FYB2 | DOWN | FYN binding protein 2 | 0.79 | 0.009 |
| IL33 | DOWN | interleukin 33 | 0.72 | <0.001 |
| KRT14 | DOWN | keratin 14 | 0.75 | 0.007 |
| MACROD2 | DOWN | mono-ADP ribosylhydrolase 2 | 1.22 | 0.014 |

(continued)

| Gene Name | Reg. | Approved Name | HR | P |
|---|---|---|---|---|
| **NAV3** | DOWN | neuron navigator 3 | 0.66 | 0.001 |
| **NOVA1** | DOWN | NOVA alternative splicing regulator 1 | 0.67 | <0.001 |
| **NTRK2** | DOWN | neurotrophic receptor tyrosine kinase 2 | 0.65 | <0.001 |
| **PGR** | DOWN | progesterone receptor | 0.59 | <0.001 |
| **PI15** | DOWN | peptidase inhibitor 15 | 0.71 | 0.006 |
| **PIEZO2** | DOWN | piezo type mechanosensitive ion channel component 2 | 0.61 | <0.001 |
| **SORCS1** | DOWN | sortilin related VPS10 domain containing receptor 1 | 0.80 | 0.026 |
| **STC2** | DOWN | stanniocalcin 2 | 0.56 | <0.001 |
| **TMEM26** | DOWN | transmembrane protein 26 | 0.47 | <0.001 |
| **WNK4** | DOWN | WNK lysine deficient protein kinase 4 | 0.77 | 0.004 |
| ATP8A2 | UP | ATPase phospholipid transporting 8A2 | 1.14 | 0.119 |
| B3GNT5 | UP | UDP-GlcNAc:betaGal beta-1,3-N-acetylglucosaminyltransferase 5 | 1.07 | 0.388 |
| CLDN1 | UP | claudin 1 | 1.16 | 0.102 |
| CXCL13 | UP | C-X-C motif chemokine ligand 13 | 0.87 | 0.180 |
| CYP1B1 | UP | cytochrome P450 family 1 subfamily B member 1 | 0.91 | 0.310 |
| GABRP | UP | gamma-aminobutyric acid type A receptor subunit pi | 1.16 | 0.180 |
| HPGD | UP | 15-hydroxyprostaglandin dehydrogenase | 0.89 | 0.355 |
| KCNJ6 | UP | potassium inwardly rectifying channel subfamily J member 6 | 0.98 | 0.851 |
| KLHDC7B | UP | kelch domain containing 7B | 1.13 | 0.140 |
| LOC1079861 70 | UP | claudin 16 | 1.01 | 0.864 |
| MOXD1 | UP | monooxygenase DBH like 1 | 0.95 | 0.522 |
| MMP1 | UP | matrix metallopeptidase 1 | 1.12 | 0.240 |
| NLRP2 | UP | NLR family pyrin domain containing 2 | 1.01 | 0.881 |
| PAK3 | UP | p21 (RAC1) activated kinase 3 | 0.87 | 0.094 |
| PDE4B | UP | phosphodiesterase 4B | 0.97 | 0.670 |
| PRR16 | UP | proline rich 16 | 0.94 | 0.493 |
| SIM1 | UP | SIM bHLH transcription factor 1 | 1.15 | 0.092 |
| SLC28A3 | UP | solute carrier family 28 member 3 | 0.89 | 0.247 |
| SLC34A2 | UP | solute carrier family 34 member 2 | 1.00 | 0.974 |
| SPTSSB | UP | serine palmitoyltransferase small subunit B | 1.03 | 0.772 |
| TFPI2 | UP | tissue factor pathway inhibitor 2 | 1.03 | 0.808 |
| THRSP | UP | thyroid hormone responsive | 0.86 | 0.130 |
| UBD | UP | ubiquitin D | 0.91 | 0.290 |
| CCDC158 | DOWN | coiled-coil domain containing 158 | 0.99 | 0.886 |
| CNR1 | DOWN | cannabinoid receptor 1 | 1.09 | 0.384 |
| COL25A1 | DOWN | collagen type XXV alpha 1 chain | 1.12 | 0.153 |
| DPY19L2P4 | DOWN | DPY19L2 pseudogene 4 | 0.94 | 0.526 |
| FGFR2 | DOWN | fibroblast growth factor receptor 2 | 0.99 | 0.893 |
| GRIA2 | DOWN | glutamate ionotropic receptor AMPA type subunit 2 | 0.86 | 0.170 |

(continued)

| Gene Name | Reg. | Approved Name | HR | P |
|---|---|---|---|---|
| GRIK4 | DOWN | glutamate ionotropic receptor kainate type subunit 4 | 0.88 | 0.122 |
| IGF2 | DOWN | insulin like growth factor 2 | 0.95 | 0.543 |
| KCND3 | DOWN | potassium voltage-gated channel subfamily D member 3 | 1.09 | 0.324 |
| KRT15 | DOWN | keratin 15 | 0.84 | 0.086 |
| LIN7A | DOWN | lin-7 homolog A. crumbs cell polarity complex component | 1.16 | 0.096 |
| NECAB1 | DOWN | N-terminal EF-hand calcium binding protein 1 | 0.84 | 0.195 |
| NRCAM | DOWN | neuronal cell adhesion molecule | 0.98 | 0.863 |
| NTRK3 | DOWN | neurotrophic receptor tyrosine kinase 3 | 0.95 | 0.521 |
| PCSK1 | DOWN | proprotein convertase subtilisin/kexin type 1 | 0.75 | 0.140 |
| PDE11A | DOWN | phosphodiesterase 11A | 0.97 | 0.669 |
| RBM24 | DOWN | RNA binding motif protein 24 | 0.98 | 0.837 |
| RELN | DOWN | reelin | 0.98 | 0.817 |
| SCN7A | DOWN | sodium voltage-gated channel alpha subunit 7 | 0.97 | 0.738 |
| SLC22A3 | DOWN | solute carrier family 22 member 3 | 1.08 | 0.360 |
| WDR11 | DOWN | WD repeat domain 11 | 0.85 | 0.060 |
| ZSCAN1 | DOWN | zinc finger and SCAN domain containing 1 | 1.06 | 0.498 |

**Table 4.** List of the 99 genes, from the original 135 differentially expressed genes between SUV-H vs. SUV-L BCs, for which expression data were present in the METABRIC database. For each gene, the gene name, mode of regulation (Reg.: up or down in the 135 gene list, see Table 5), approved name *in expense,* HR (hazard ratio) for death related to BC in univariate analysis, p-value (P) in univariate analysis. P-values and HR were calculated by Cox proportional hazards regression model analysis using 'survival' package in R, version 3.5-5. The 54 genes that significantly correlated with worse prognostic outcome in univariate analysis, which comprise the PETsign, are in bold characters.

**Table 5. List of the 135 genes differentially expressed between SUV-H vs. SUV-L BCs.**

| Gene Name | SUV-H vs. SUV-L | FC | p-value | FDR |
|---|---|---|---|---|
| LOC105371822 | UP | 12.47 | <0.001 | 0.001 |
| CXCL8 | UP | 8.22 | <0.001 | 0.001 |
| TGFB2-OT1 | UP | 6.57 | <0.001 | <0.001 |
| S100A9 | UP | 6.28 | <0.001 | 0.005 |
| CP | UP | 6.01 | <0.001 | <0.001 |
| CHI3L1 | UP | 5.29 | <0.001 | <0.001 |
| EGFR | UP | 5.18 | <0.001 | <0.001 |
| LOC105374557 | UP | 5.17 | <0.001 | <0.001 |
| MAB21L4 | UP | 4.99 | <0.001 | 0.001 |
| ATP8A2 | UP | 4.83 | <0.001 | 0.001 |
| GABRE | UP | 4.54 | <0.001 | 0.002 |
| MMP1 | UP | 4.38 | <0.001 | 0.001 |
| LOC112268106 | UP | 4.36 | <0.001 | 0.001 |
| RARRES1 | UP | 4.19 | <0.001 | 0.001 |
| TMEM158 | UP | 4.18 | <0.001 | <0.001 |

(continued)

| Gene Name | SUV-H *vs.* SUV-L | FC | p-value | FDR |
|---|---|---|---|---|
| CXCL10 | UP | 3.91 | <0.001 | 0.001 |
| PSAT1 | UP | 3.89 | <0.001 | 0.009 |
| CXCL11 | UP | 3.89 | <0.001 | 0.001 |
| GABRP | UP | 3.84 | <0.001 | 0.002 |
| SLITRK5 | UP | 3.75 | <0.001 | 0.006 |
| SOX11 | UP | 3.68 | <0.001 | 0.001 |
| EREG | UP | 3.67 | <0.001 | <0.001 |
| B3GNT5 | UP | 3.64 | <0.001 | <0.001 |
| CXCL13 | UP | 3.58 | <0.001 | <0.001 |
| AQP9 | UP | 3.57 | <0.001 | 0.001 |
| HPGD | UP | 3.53 | <0.001 | <0.001 |
| PAK3 | UP | 3.50 | <0.001 | 0.001 |
| THRSP | UP | 3.49 | <0.001 | 0.005 |
| NDUFA4L2 | UP | 3.46 | 0.001 | 0.016 |
| SLC34A2 | UP | 3.32 | <0.001 | <0.001 |
| UBD | UP | 3.31 | <0.001 | <0.001 |
| KLHDC7B | UP | 3.31 | <0.001 | 0.004 |
| SPTSSB | UP | 3.26 | <0.001 | 0.001 |
| LOC107986170 | UP | 3.24 | <0.001 | 0.001 |
| LAMP3 | UP | 3.22 | <0.001 | 0.005 |
| SLC28A3 | UP | 3.22 | <0.001 | <0.001 |
| PLCH1 | UP | 3.15 | 0.001 | 0.022 |
| DNAH11 | UP | 3.14 | <0.001 | 0.001 |
| EPOP | UP | 3.14 | <0.001 | 0.002 |
| ADAMDEC1 | UP | 3.13 | <0.001 | <0.001 |
| CLDN1 | UP | 3.12 | 0.003 | 0.034 |
| MFSD2A | UP | 3.11 | <0.001 | 0.011 |
| CDCA7 | UP | 3.11 | <0.001 | 0.007 |
| C5orf46 | UP | 3.09 | <0.001 | 0.010 |
| SHISA2 | UP | 3.07 | <0.001 | <0.001 |
| LOC105375172 | UP | 3.05 | <0.001 | 0.001 |
| LINC00673 | UP | 3.02 | <0.001 | 0.002 |
| LOC107987296 | UP | 3.02 | <0.001 | <0.001 |
| CENPW | UP | 3.02 | 0.002 | 0.031 |
| LOC105375170 | UP | 2.92 | <0.001 | 0.001 |
| CCDC144NL-AS1 | UP | 2.91 | <0.001 | 0.001 |
| IGHG4 | UP | 2.88 | <0.001 | 0.005 |
| LOC107986350 | UP | 2.86 | <0.001 | 0.009 |
| CSF2RB | UP | 2.77 | <0.001 | 0.007 |

(continued)

| Gene Name | SUV-H *vs.* SUV-L | FC | p-value | FDR |
|---|---|---|---|---|
| IFI27 | UP | 2.76 | 0.002 | 0.029 |
| ADM | UP | 2.76 | <0.001 | <0.001 |
| PDE4B | UP | 2.71 | <0.001 | 0.001 |
| SEC61G | UP | 2.71 | <0.001 | 0.001 |
| CDH3 | UP | 2.71 | <0.001 | 0.001 |
| LOC105376442 | UP | 2.70 | <0.001 | 0.001 |
| SIM1 | UP | 2.68 | <0.001 | 0.009 |
| PIR | UP | 2.65 | <0.001 | <0.001 |
| MOXD1 | UP | 2.65 | <0.001 | 0.005 |
| GALNT14 | UP | 2.59 | <0.001 | <0.001 |
| TFPI2 | UP | 2.55 | <0.001 | 0.001 |
| CYP1B1 | UP | 2.55 | 0.002 | 0.031 |
| HERC5 | UP | 2.55 | <0.001 | 0.001 |
| SPP1 | UP | 2.53 | <0.001 | 0.002 |
| NLRP2 | UP | 2.52 | <0.001 | 0.001 |
| MCM10 | UP | 2.52 | 0.002 | 0.032 |
| ME1 | UP | 2.52 | <0.001 | <0.001 |
| PRR16 | UP | 2.51 | <0.001 | <0.001 |
| KCNJ6 | UP | 2.50 | <0.001 | 0.005 |
| ABCA10 | DOWN | 0.40 | 0.001 | 0.020 |
| SLC22A3 | DOWN | 0.39 | <0.001 | 0.001 |
| GRIK4 | DOWN | 0.39 | <0.001 | <0.001 |
| ME3-DT | DOWN | 0.39 | <0.001 | 0.002 |
| FREM1 | DOWN | 0.39 | <0.001 | 0.003 |
| LOC105375896 | DOWN | 0.39 | <0.001 | 0.009 |
| LINC01016 | DOWN | 0.38 | <0.001 | 0.003 |
| CCDC158 | DOWN | 0.37 | <0.001 | <0.001 |
| CYP2A7 | DOWN | 0.36 | <0.001 | <0.001 |
| LOC105375387 | DOWN | 0.36 | <0.001 | <0.001 |
| FOLH1 | DOWN | 0.36 | <0.001 | <0.001 |
| ADIPOQ | DOWN | 0.36 | <0.001 | <0.001 |
| NOVA1 | DOWN | 0.36 | <0.001 | <0.001 |
| PDE11A | DOWN | 0.36 | <0.001 | 0.001 |
| NRCAM | DOWN | 0.36 | <0.001 | 0.001 |
| LOC105374020 | DOWN | 0.36 | <0.001 | 0.001 |
| TMEM26 | DOWN | 0.35 | <0.001 | 0.002 |
| CCDC85A | DOWN | 0.35 | <0.001 | <0.001 |
| RELN | DOWN | 0.35 | <0.001 | 0.001 |
| LOC105378747 | DOWN | 0.35 | <0.001 | <0.001 |

(continued)

| Gene Name | SUV-H *vs.* SUV-L | FC | p-value | FDR |
|---|---|---|---|---|
| LOC101929322 | DOWN | 0.34 | 0.003 | 0.040 |
| KRT15 | DOWN | 0.34 | 0.001 | 0.017 |
| CKB | DOWN | 0.34 | <0.001 | 0.002 |
| ZSCAN1 | DOWN | 0.33 | <0.001 | 0.011 |
| KCND3 | DOWN | 0.33 | <0.001 | 0.001 |
| COL25A1 | DOWN | 0.33 | <0.001 | <0.001 |
| WNK4 | DOWN | 0.33 | <0.001 | <0.001 |
| NTRK2 | DOWN | 0.32 | <0.001 | 0.003 |
| LOC107984400 | DOWN | 0.32 | <0.001 | <0.001 |
| LOC105375634 | DOWN | 0.32 | <0.001 | 0.003 |
| LOC105369869 | DOWN | 0.32 | <0.001 | 0.001 |
| ELOVL2 | DOWN | 0.31 | <0.001 | <0.001 |
| SCN7A | DOWN | 0.30 | <0.001 | <0.001 |
| WDR11 | DOWN | 0.30 | <0.001 | 0.001 |
| LOC102724484 | DOWN | 0.30 | <0.001 | 0.001 |
| MACROD2 | DOWN | 0.30 | <0.001 | <0.001 |
| LOC105372877 | DOWN | 0.29 | <0.001 | <0.001 |
| STC2 | DOWN | 0.29 | <0.001 | 0.007 |
| NAV3 | DOWN | 0.29 | <0.001 | 0.002 |
| LIN7A | DOWN | 0.29 | <0.001 | <0.001 |
| MACROD2-AS1 | DOWN | 0.28 | <0.001 | <0.001 |
| SORCS1 | DOWN | 0.28 | <0.001 | 0.005 |
| LOC101926959 | DOWN | 0.27 | <0.001 | 0.001 |
| FYB2 | DOWN | 0.27 | <0.001 | <0.001 |
| IGF2 | DOWN | 0.27 | <0.001 | <0.001 |
| IL33 | DOWN | 0.27 | 0.001 | 0.012 |
| KRT14 | DOWN | 0.27 | 0.002 | 0.028 |
| RBM24 | DOWN | 0.27 | 0.001 | 0.015 |
| NTRK3 | DOWN | 0.26 | <0.001 | 0.004 |
| PGR | DOWN | 0.25 | 0.002 | 0.027 |
| CNR1 | DOWN | 0.25 | <0.001 | 0.003 |
| PIEZO2 | DOWN | 0.25 | 0.001 | 0.017 |
| LOC105375886 | DOWN | 0.23 | <0.001 | 0.002 |
| ERICH3 | DOWN | 0.22 | <0.001 | 0.002 |
| DPY19L2P4 | DOWN | 0.22 | <0.001 | 0.006 |
| NECAB1 | DOWN | 0.20 | 0.004 | 0.043 |
| ADH1B | DOWN | 0.19 | <0.001 | <0.001 |
| PI15 | DOWN | 0.17 | <0.001 | <0.001 |
| FGFR2 | DOWN | 0.16 | <0.001 | 0.004 |

(continued)

| Gene Name | SUV-H *vs.* SUV-L | FC | p-value | FDR |
|---|---|---|---|---|
| GRIA2 | DOWN | 0.15 | <0.001 | 0.005 |
| LOC105375472 | DOWN | 0.13 | <0.001 | <0.001 |
| PCSK1 | DOWN | 0.04 | <0.001 | 0.005 |

**Table 5.** The list of 135 genes differentially regulated in the SUV-H vs. SUV-L BC of the 120-PET cohort is shown. The comparison between SUV-H and SUV-L BC groups was subjected to statistical analysis using the EdgeR package of R software (version 3.40.2). Quasi-likelihood F-tests (QLF) were employed to obtain p-values and FDR, and these values were further adjusted using the Benjamini-Hochberg correction procedure. UP, upregulated genes (FDR < 0.05, p < 0.05, FC > 2.5, 73 genes); DOWN, downregulated genes (FDR < 0.05, p < 0.05, FC < 0.4, 62 genes). FC, fold-change SUV-H *vs.* SUV-L.

**Table 6. Functions of the 135 genes differentially expressed between SUV-H vs. SUV-L BCs.**

| Category/ Function | Upregulated Genes (PETsign genes are in bold) | Downregulated Genes (PETsign genes are in bold) |
|---|---|---|
| ncRNA (11 UP, 17 DOWN) | CCDC144NL-AS1, LINC00673, LOC105371822, LOC105374557, LOC105375170, LOC105375172, LOC105376442, LOC107986350, LOC107987296, LOC112268106, TGFB2-OT1. | DPY19L2P4, LINC01016, LOC101929322, LOC101926959, LOC102724484, LOC105369869, LOC105372877, LOC105374020, LOC105375387, LOC105375472, LOC105375634, LOC105375886, LOC105375896, LOC105378747, LOC107984400, MACROD2-AS1, ME3-DT. |
| Immune/inflammation-related (15 UP, 2 DOWN) | **ADM, CSF2RB, CXCL8, CXCL10, CXCL11,** CXCL13, CHI3L1, HERC5, KLHDC7B, **LAMP3,** NLRP2, **IF127,** IGHG4, **S100A9, SPP1.** | **IL33, FREM1.** |
| Ion transport at the PM, other transporters and/or carrier (10 UP, 11 DOWN) | **AQP9,** ATP8A2, **CP, GABRE,** GABRP, KCNJ6, **MFSD2A, SEC61G,** SLC28A3, SLC34A2. | **ABCA10, FOLH1,** GRIA2, GRIK4, KCND3, LIN7A, **PIEZO2,** SCN7A, SLC22A3, **STC2, WNK4.** |
| Signal transduction, DNA replication/mitosis, traffic (13 UP, 13 DOWN) | **CDCA7, CENPW, EGFR,** EREG, MAB21L4, **MCM10,** PAK3, PDE4B, **PLCH1, SHISA2, SLITRK5,** TFPI2, **TM EM 158.** | CNR1, FGFR2, **FYB2,** IGF2, **MACROD2,** NECAB1, **NTRK2,** NTRK3, PCSK1, PDE11A, **PGR, SORCS1,** WDR11. |
| Metabolism, mitochondrial function, modifying enzymes (14 UP, 6 DOWN) | B3GNT5, **C5orf46,** CYP1B1, **GALNT14,** HPGD, **ME1,** MOXD1, **NDUFA4L2,** PRR16, **PSAT1, RARRES1,** SPTSSB, THRSP, UBD. | ADH1B, **CKB, CYP2A7, ELOVL2, PI15, TMEM26.** |
| Cell adhesion, shape and/or motility, including ciliary proteins (6 UP, 11 DOWN) | **ADAMDEC1, CDH3,** CLDN1, CLDN16, DNAH11, MMP1. | ADIPOQ, CCDC158, **CCDC85A,** COL25A1, **ERICH3, KRT14,** KRT15, **NAV3, NOVA1,** NRCAM, RELN. |
| Regulators of gene expression (4 UP, 2 DOWN) | EPOP, **PIR,** SIM1, **SOX11.** | RBM24, ZSCAN1. |

**Table 6.** The functional categories were attributed manually by interrogating the NCBI "Gene" database, followed by inspection of extant literature. Genes of the 54-gene PET signature (PETsign) are in bold.

**Table 7: The immune-related genes of PETsign are not a reflection of different immune infiltrates.**

| Gene | Cell Type | FC SUV-H/SUV-L | p-value | FDR |
|---|---|---|---|---|
| BLK | B cell | ND | | |
| CD19 | B cell | ND | | |
| MS4A1 | B cell | 0.68 | 0.17 | 0.45 |
| TNFRSF17 | B cell | ND | | |
| PTPRC | CD45 | 1.17 | 0.26 | 0.57 |
| CD8A | CD8 T cell | 0.93 | 0.66 | 0.87 |
| CD8B | CD8 T cell | 0.80 | 0.24 | 0.55 |
| CTSW | Cytotoxic cell | ND | | |
| GNLY | Cytotoxic cell | ND | | |
| GZMA | Cytotoxic cell | ND | | |
| GZMB | Cytotoxic cell | ND | | |
| GZMH | Cytotoxic cell | ND | | |
| KLRB1 | Cytotoxic cell | 1.01 | 0.91 | 0.97 |
| KLRD1 | Cytotoxic cell | 0.93 | 0.10 | 0.34 |
| KLRK1 | Cytotoxic cell | 0.76 | 0.14 | 0.42 |
| PRF1 | Cytotoxic cell | ND | | |
| CCL13 | Dendritic cell | ND | | |
| CD209 | Dendritic cell | 0.99 | 0.95 | 0.98 |
| HSD11B1 | Dendritic cell | ND | | |
| CD244 | Exhausted CD8 T cell | ND | | |
| EOMES | Exhausted CD8 T cell | 0.98 | 0.91 | 0.97 |
| LAG3 | Exhausted CD8 T cell | ND | | |
| CD163 | Macrophage | 1.25 | 0.14 | 0.40 |
| CD68 | Macrophage | 1.37 | 0.10 | 0.34 |
| CD84 | Macrophage | 1.13 | 0.16 | 0.44 |
| MS4A2 | Mast cell | 0.84 | 0.37 | 0.67 |
| TPSAB1 | Mast cell | 0.47 | 0.008 | 0.071 |
| CSF3R | Neutrophil | 1.08 | 0.74 | 0.91 |
| FCGR3A | Neutrophil | 1.46 | 0.0021 | 0.030 |
| S100A12 | Neutrophil | ND | | |
| IL21R | Natural killer CD56$^{dim}$ cell | 1.27 | 0.18 | 0.47 |
| KIR3DL1 | Natural killer CD56$^{dim}$ cell | ND | | |
| KIR3DL2 | Natural killer CD56$^{dim}$ cell | ND | | |
| KIR3DL3 | Natural killer CD56$^{dim}$ cell | ND | | |
| NCR1 | Natural killer cell | ND | | |
| XCL2 | Natural killer cell | ND | | |
| CD3D | T cell | ND | | |
| CD3E | T cell | 0.89 | 0.54 | 0.80 |

(continued)

| Gene | Cell Type | FC SUV-H/SUV-L | p-value | FDR |
|---|---|---|---|---|
| CD3G | T cell | 1.06 | 0.65 | 0.87 |
| CD6 | T cell | 0.79 | 0.26 | 0.57 |
| SH2D1A | T cell | 1.17 | 0.46 | 0.75 |
| TBX21 | Type 1 T helper cell | ND | | |
| FOXP3 | Regulatory T cell | 0.98 | 0.91 | 0.97 |

**Table 7.** Since several of the upregulated genes in SUV-H BCs are annotated as immune/inflammation-related, we performed several controls to establish whether their presence was due to increased immune infiltration in SUV-H tumors. The results shown in this table were generated starting from the "nCounter Human Pan-Cancer Immune Profiling Panel; NanoString Technologies" which is widely used to establish immune components in a tumor mass (see for instance [104]). From the genes listed in the panel, we extracted the genes labeled as "cell-specific" as they should specifically reflect the presence of immune cells. Several genes were not detected (ND) in BCs, regardless of their SUV status. With two exceptions (*TPSAB1* and *FCGR3A),* the detectable genes did not show significant differences between SUV-H and SUV-L BCs. In the case of *TPSAB1* and *FCGR3A,* it should be noted that other genes identifying the same immune cell type (mast cells and neutrophils, respectively) were not differentially expressed in the SUV-H *vs*. SUV-L comparison.

We also performed additional analyses using TIMER 2.0 [105, 106] which revealed minimal contamination of immune/stromal components in the analyzed BC samples (see Table 8). We concluded that the presence of transcripts belonging to genes annotated as immune/inflammatory is due to the epithelial component of the analyzed BCs.

Statistical analysis using the EdgeR package in R software (version 3.40.2) was employed to compute fold-change (FC), p-values and false discovery rates (FDRs); please refer to the legend to Table 5 for further details.

**Table 8: Analysis of immune infiltrates and stromal contamination in the analyzed BCs.**

| Cell Type | Algorithm | Median SUV-H BCs | Median SUV-L BCs |
|---|---|---|---|
| B cell memory | CIBERSORT-ABS | 0.01 | 0.02 |
| B cell naive | CIBERSORT-ABS | 0.00 | 0.00 |
| B cell plasma | CIBERSORT-ABS | 0.03 | 0.03 |
| Eosinophil | CIBERSORT-ABS | 0.00 | 0.00 |
| Macrophage M0 | CIBERSORT-ABS | 0.00 | 0.00 |
| Macrophage M1 | CIBERSORT-ABS | 0.06 | 0.03 |
| Macrophage M2 | CIBERSORT-ABS | 0.20 | 0.19 |
| Mast cell activated | CIBERSORT-ABS | 0.04 | 0.04 |
| Mast cell resting | CIBERSORT-ABS | 0.00 | 0.00 |
| Monocyte | CIBERSORT-ABS | 0.01 | 0.01 |
| Myeloid dendritic cell activated | CIBERSORT-ABS | 0.04 | 0.03 |
| Myeloid dendritic cell resting | CIBERSORT-ABS | 0.00 | 0.00 |
| Neutrophil | CIBERSORT-ABS | 0.00 | 0.00 |
| NK cell activated | CIBERSORT-ABS | 0.01 | 0.01 |
| NK cell resting | CIBERSORT-ABS | 0.00 | 0.00 |
| T cell CD4+ memory activated | CIBERSORT-ABS | 0.00 | 0.00 |
| T cell CD4+ memory resting | CIBERSORT-ABS | 0.18 | 0.13 |
| T cell CD4+ naive | CIBERSORT-ABS | 0.00 | 0.00 |
| T cell CD8+ | CIBERSORT-ABS | 0.03 | 0.04 |
| T cell follicular helper | CIBERSORT-ABS | 0.04 | 0.02 |
| T cell gamma delta | CIBERSORT-ABS | 0.00 | 0.00 |

(continued)

| Cell Type | Algorithm | Median SUV-H BCs | Median SUV-L BCs |
|---|---|---|---|
| T cell regulatory (Tregs) | CIBERSORT-ABS | 0.00 | 0.00 |
| B cell | EPIC | 0.00 | 0.00 |
| Cancer associated fibroblast | EPIC | 0.06 | 0.08 |
| Endothelial cell | EPIC | 0.01 | 0.01 |
| Macrophage | EPIC | 0.00 | 0.00 |
| NK cell | EPIC | 0.00 | 0.00 |
| T cell CD4+ | EPIC | 0.05 | 0.04 |
| T cell CD8+ | EPIC | 0.01 | 0.01 |
| Uncharacterized cell | EPIC | 0.85 | 0.83 |
| B cell | QUANTISEQ | 0.01 | 0.01 |
| Macrophage M1 | QUANTISEQ | 0.01 | 0.00 |
| Macrophage M2 | QUANTISEQ | 0.01 | 0.01 |
| Monocyte | QUANTISEQ | 0.00 | 0.00 |
| Myeloid dendritic cell | QUANTISEQ | 0.00 | 0.00 |
| Neutrophil | QUANTISEQ | 0.01 | 0.01 |
| NK cell | QUANTISEQ | 0.01 | 0.01 |
| T cell CD4+ (non-regulatory) | QUANTISEQ | 0.00 | 0.00 |
| T cell CD8+ | QUANTISEQ | 0.00 | 0.00 |
| T cell regulatory (Tregs) | QUANTISEQ | 0.01 | 0.01 |
| Uncharacterized cell | QUANTISEQ | 0.95 | 0.95 |

**Table 8.** We used the TIMER 2.0 webtool [105, 106], which integrates multiple state-of-the-art algorithms for the estimation of immune and non-immune cell infiltration on user-provided expression profiles, to compare SUV-H and SUV-L BCs of the 120-BC PET cohort. We report here the data obtained with the EPIC [107] and QUANTISEQ [108] algorithms that provide absolute scores representing cell fractions (therefore numbers in the table correspond to percentage of cells reported as a decimal), and with the CYBERSORT algorithm in the absolute mode (CYBERSORT-ABS) [109], which scales cellular fractions to a score (in arbitrary units) that reflects each cell type's absolute proportion, allowing comparison across both samples and cell types. The data show that the contamination by immune, endothelial and fibroblast components is minimal in the analyzed BCs, with the majority of the cells (as evidenced by the EPIC and QUANTISEQ algorithms) classified as "Uncharacterized cell" (in bold): presumably epithelial cells.

**Table 9. Prognostic stratification by PETsign in the METABRIC, TCGA, and IEO clinical cohorts.**

| Cohort | Subgroup and Type of Analysis | HR | P | 95% CI |
|---|---|---|---|---|
| METABRIC | ALL Univariate | 2.09 | <0.0001 | 1.77-2.45 |
| | ALL Multivariable | 1.62 | <0.0001 | 1.33-1.98 |
| | LUMINAL Univariate | 1.79 | <0.0001 | 1.47-2.18 |
| | LUMINAL Multivariable | 1.57 | <0.0001 | 1.28-1.92 |
| | LUMINAL-HER2 Univariate | 1.24 | 0.49 | 0.67-2.29 |
| | HER2 Univariate | 1.18 | 0.57 | 0.66-2.11 |
| | TNBC Univariate | 0.97 | 0.86 | 0.67-1.40 |
| TCGA* | ALL Univariate | 2.32 | 0.0004 | 1.46-3.71 |
| | ALL Multivariable | 2.18 | 0.0059 | 1.25-3.78 |

(continued)

| Cohort | Subgroup and Type of Analysis | HR | P | 95% CI |
|---|---|---|---|---|
| IEO | ALL Univariate | 2.74 | <.0001 | 1.99-3.78 |
| | ALL Multivariable | 2.27 | <.0001 | 1.56-3.32 |
| | LUMINAL-A Univariate | Classes not assigned** | | |
| | LUMINAL-B Univariate | 2.38 | <0.0001 | 1.56-3.62 |
| | LUMINAL-B Multivariable | 1.66 | 0.026 | 1.06-2.58 |
| | LUMINAL (A+B) Univariate | 1.82 | <0.0001 | 1.49-2.22 |
| | LUMINAL (A+B) Multivariable | 1.60 | <0.0001 | 1.30-1.96 |
| | LUM-HER2 Univariate | 1.91 | 0.22 | 0.68-5.37 |
| | HER2 Univariate | Classes not assigned*** | | |
| | TNBC Univariate | 1.25 | 0.71 | 0.39-3.97 |

**Table 9.** The performance of PETsign in the three clinical cohorts is shown. Hazard ratios (HR) and p-values (P) were calculated with the Cox proportional hazards model using JMP. The 95% confidence interval (CI) is also shown. Variables used for the multivariable analyses were as follows. METABRIC: age, tumor size, nodal status, HR (ER/PGR), HER2, and tumor grade. TCGA: age, tumor size, nodal status, HR (ER/PGR), and HER2. IEO: age, tumor size, nodal status, HR (ER/PGR), HER2, tumor grade and Ki67 status.

*, in the TCGA dataset at 5 years of follow up, there were only 63 events (deaths). This prevented meaningful analysis of the molecular subgroups, since too few events were present (26 for Luminal, 12 for Luminal-HER2, 4 for HER2, 21 for TNBC).

**, Classes could not be assigned because only 4 events (DRBC) were present, all SUV-High.

***, Hierarchical clustering applied to HER2-positive samples did not result in two classes of tumors, resembling SUV-H-like or SUV-L-like, because almost all tumors were SUV-H-like.

**Table 10. Co-occurrence of amplification of PETsign and 135-signature genes in the TGCA cohort.**

| A | B | Neither | A Not B | B Not A | Both | Log2 Odds Ratio | q-Value | Tendency |
|---|---|---|---|---|---|---|---|---|
| LAMP3 | B3GNT5 | 843 | 0 | 1 | 35 | >3 | <0.001 | Co-occurrence |
| CLDN1 | CLDN16 | 850 | 0 | 1 | 28 | >3 | <0.001 | Co-occurrence |
| CXCL10 | CXCL11 | 859 | 0 | 0 | 20 | >3 | <0.001 | Co-occurrence |
| RARRES 1 | SPTSSB | 851 | 1 | 5 | 22 | >3 | <0.001 | Co-occurrence |
| LAMP3 | CLDN1 | 840 | 11 | 4 | 24 | >3 | <0.001 | Co-occurrence |
| B3GNT5 | CLDN1 | 839 | 12 | 4 | 24 | >3 | <0.001 | Co-occurrence |
| LAMP3 | CLDN16 | 839 | 11 | 5 | 24 | >3 | <0.001 | Co-occurrence |
| B3GNT5 | CLDN16 | 838 | 12 | 5 | 24 | >3 | <0.001 | Co-occurrence |
| CXCL8 | CXCL10 | 856 | 3 | 2 | 18 | >3 | <0.001 | Co-occurrence |
| CXCL8 | CXCL11 | 856 | 3 | 2 | 18 | >3 | <0.001 | Co-occurrence |
| RARRES 1 | PLCH1 | 853 | 4 | 3 | 19 | >3 | <0.001 | Co-occurrence |
| CP | PLCH1 | 853 | 4 | 4 | 18 | >3 | <0.001 | Co-occurrence |
| PLCH1 | SPTSSB | 849 | 3 | 8 | 19 | >3 | <0.001 | Co-occurrence |
| S100A9 | CHI3L1 | 722 | 52 | 49 | 56 | >3 | <0.001 | Co-occurrence |
| EREG | CXCL10 | 857 | 2 | 4 | 16 | >3 | <0.001 | Co-occurrence |
| EREG | CXCL11 | 857 | 2 | 4 | 16 | >3 | <0.001 | Co-occurrence |
| CXCL8 | EREG | 856 | 5 | 2 | 16 | >3 | <0.001 | Co-occurrence |

(continued)

| A | B | Neither | A Not B | B Not A | Both | Log2 Odds Ratio | q-Value | Tendency |
|---|---|---|---|---|---|---|---|---|
| RARRES 1 | CP | 850 | 7 | 6 | 16 | >3 | **<0.001** | Co-occurrence |
| CP | SPTSSB | 846 | 6 | 11 | 16 | >3 | **<0.001** | Co-occurrence |
| EGFR | SEC61G | 859 | 8 | 0 | 12 | >3 | **<0.001** | Co-occurrence |
| CXCL10 | CXCL13 | 857 | 8 | 2 | 12 | >3 | **<0.001** | Co-occurrence |
| CXCL11 | CXCL13 | 857 | 8 | 2 | 12 | >3 | **<0.001** | Co-occurrence |
| CXCL8 | CXCL13 | 855 | 10 | 3 | 11 | >3 | **<0.001** | Co-occurrence |
| EREG | CXCL13 | 857 | 8 | 4 | 10 | >3 | **<0.001** | Co-occurrence |
| CLDN1 | RARRES 1 | 840 | 16 | 11 | 12 | >3 | **<0.001** | Co-occurrence |
| CLDN16 | RARRES 1 | 839 | 17 | 11 | 12 | >3 | **<0.001** | Co-occurrence |
| LAMP3 | SPTSSB | 830 | 22 | 14 | 13 | >3 | **<0.001** | Co-occurrence |
| CLDN1 | SPTSSB | 836 | 16 | 15 | 12 | >3 | **<0.001** | Co-occurrence |
| LAMP3 | CP | 834 | 23 | 10 | 12 | >3 | **<0.001** | Co-occurrence |
| B3GNT5 | SPTSSB | 829 | 23 | 14 | 13 | >3 | **<0.001** | Co-occurrence |
| B3GNT5 | CP | 833 | 24 | 10 | 12 | >3 | **<0.001** | Co-occurrence |
| CLDN16 | SPTSSB | 835 | 17 | 15 | 12 | >3 | **<0.001** | Co-occurrence |
| CLDN1 | CP | 840 | 17 | 11 | 11 | >3 | **<0.001** | Co-occurrence |
| CLDN1 | PLCH1 | 840 | 17 | 11 | 11 | >3 | **<0.001** | Co-occurrence |
| CLDN16 | CP | 839 | 18 | 11 | 11 | >3 | **<0.001** | Co-occurrence |
| CLDN16 | PLCH1 | 839 | 18 | 11 | 11 | >3 | **<0.001** | Co-occurrence |
| LAMP3 | RARRES 1 | 832 | 24 | 12 | 11 | >3 | **<0.001** | Co-occurrence |
| B3GNT5 | RARRES 1 | 831 | 25 | 12 | 11 | >3 | **<0.001** | Co-occurrence |
| LAMP3 | PLCH1 | 832 | 25 | 12 | 10 | >3 | **<0.001** | Co-occurrence |
| B3GNT5 | PLCH1 | 831 | 26 | 12 | 10 | >3 | **<0.001** | Co-occurrence |
| MCM10 | S100A9 | 755 | 16 | 94 | 14 | 2.813 | **<0.001** | Co-occurrence |

**Table 10.** The TCGA database was interrogated through the cBioPortal. Initially, the putative copy number alteration was evaluated by GISTIC 2.0 and only genes displaying amplification in at least 1.5% of cases were further considered. For these genes, the "mutual exclusivity function" of the cBioPortal was used to yield the numerical values shown in the Table. Only instances of highly significant co-occurrence (q-value < 0.001) are shown. In red, PETsign genes; in black, other upregulated genes from the list of 135 differentially expressed genes. In the table: columns A and B show the identity of gene A and gene B used in the pairwise comparison; Neither, number of samples with no amplification of A or B; A not B, number of samples with amplification of A but not B; B not A, number of samples with amplification of B but not A; Both, number of samples with amplification of both A and B; Log2 Odds Ratio, log2(odds of amplification of B given amplification of A)/(odds of amplification of B given lack of amplification of A); q-value, derived from two-sided Fisher's Exact Test and the Benjamini-Hochberg FDR correction procedure; Tendency; result of the analysis showing highly significant co-occurrence of amplification of A and of B.

Two genes, CCDC158 and ADIPOQ, were not included in our analysis, despite showing amplification in > 1.5% of BCs and significant co-occurrence with the other selected genes. This is because these two genes were downregulated in the PETsign. Since CCDC158 and ADIPOQ are located on 4q21.1 and 3q27.3, respectively, they are probably co-amplified in the amplicons reported in Fig. 4C and 4E.

**Table 11: Metabolite production in SUV-H-like and SUV-L-like BC cell lines.**

| Metabolite | FC SUV-H/SUV-L | p-value |
|---|---|---|
| 1-methylnicotinamide | 87.18 | <.0001 |
| GABA | 6.48 | 0.0023 |
| N-carbamoyl-beta-alanine | 3.65 | <.0001 |
| Kynurenine | 1.96 | 0.0371 |
| Inositol | 1.73 | 0.0001 |
| UDP-galactose/UDP-glucose | 1.69 | 0.0453 |
| DHAP/glyceraldehyde 3P | 1.64 | 0.0272 |
| Beta-alanine | 1.59 | 0.039 |
| Lactate | 1.57 | 0.0158 |
| Taurocholate | 1.55 | 0.0176 |
| Glutamate | 1.53 | 0.0141 |
| Malondialdehyde | 1.43 | 0.0057 |
| 2-aminoadipate | 1.39 | 0.0499 |
| Isoleucine | 1.38 | 0.0245 |
| Sorbitol | 1.35 | 0.0371 |
| Leucine | 1.30 | 0.0476 |
| 2-hydroxyglutarate | 1.28 | 0.039 |
| Hexoses (HILIC neg) | 1.26 | 0.0318 |
| Sucrose | 0.87 | 0.0149 |
| Oleylcarnitine | 0.78 | 0.0053 |
| Stearoylcarnitine | 0.71 | 0.0258 |
| Acetylglycine | 0.64 | 0.0335 |
| Carnitine | 0.61 | 0.0197 |
| AMP | 0.61 | 0.0083 |
| Myristoylcarnitine | 0.60 | 0.0001 |
| palmitoylcarnitine | 0.58 | 0.0007 |
| GMP | 0.50 | 0.0208 |
| Butyrobetaine | 0.49 | 0.0002 |
| Propionylcarnitine | 0.47 | 0.0099 |
| Acetylcarnitine | 0.44 | 0.0023 |
| Alpha-glycerophosphate | 0.39 | 0.0302 |
| Malonylcarnitine | 0.37 | 0.0005 |
| 2-deoxycytidine | 0.35 | 0.0232 |
| Lauroylcarnitine | 0.25 | <.0001 |
| Lactose | 0.24 | 0.0197 |
| Butyrylcarnitine/isobutyrylcarnitine | 0.23 | 0.0001 |
| Hexanoylcarnitine | 0.20 | <.0001 |

**Table 11.** Transcriptomic and metabolomic data for 48 BC lines were extracted from public databases [23, 24] (PMID). SUV-H-like and SUV-L-like status was assigned to the cell lines by unsupervised hierarchical clustering using the PETsign genes (the list of cell lines and their SUV-like status is shown in Fig. 7). Average production of the metabolites was then calculated separately for SUV-H-like and SUV-L-like cell lines and the fold-change (FC) SUV-H/SUV-L is shown. Only metabolites displaying significant differences are shown. P-values were determined using the non-parametric Wilcoxon test within JMP.

**REFERENCES**

1. Hanahan, D., Hallmarks of Cancer: New Dimensions. Cancer Discov, 2022. 12(1): p. 31-46.

2. DeBerardinis, R.J. and N.S. Chandel, We need to talk about the Warburg effect. Nat Metab, 2020. 2(2): p. 127-129.

3. Warburg, O., Versuche an überlebendem carcinom-gewebe (Methoden). Biochemische Zeitschrift, 1923. 142: p. 317-333.

4. Koppenol, W.H., P.L. Bounds, and C.V. Dang, Otto Warburg's contributions to current concepts of cancer metabolism. Nat Rev Cancer, 2011. 11(5): p. 325-37.

5. DeBerardinis, R.J. and N.S. Chandel, Fundamentals of cancer metabolism. Sci Adv, 2016. 2(5): p. e1600200.

6. Boellaard, R., Standards for PET image acquisition and quantitative data analysis. J Nucl Med, 2009. 50 Suppl 1: p. 11S-20S.

7. Sung, H., et al., Global Cancer Statistics 2020: GLOBOCAN Estimates of Incidence and Mortality Worldwide for 36 Cancers in 185 Countries. CA Cancer J Clin, 2021. 71(3): p. 209-249.

8. Allison, K.H., Molecular pathology of breast cancer: what a pathologist needs to know. Am J Clin Pathol, 2012. 138(6): p. 770-80.

9. Cancer Genome Atlas, N., Comprehensive molecular portraits of human breast tumours. Nature, 2012. 490(7418): p. 61-70.

10. Burstein, H.J., et al., Customizing local and systemic therapies for women with early breast cancer: the St. Gallen International Consensus Guidelines for treatment of early breast cancer 2021. Ann Oncol, 2021. 32(10): p. 1216-1235.

11. Coates, A.S., et al., Tailoring therapies--improving the management of early breast cancer: St Gallen International Expert Consensus on the Primary Therapy of Early Breast Cancer 2015. Ann Oncol, 2015. 26(8): p. 1533-46.

12. Harris, L.N., et al., Use of Biomarkers to Guide Decisions on Adjuvant Systemic Therapy for Women With Early-Stage Invasive Breast Cancer: American Society of Clinical Oncology Clinical Practice Guideline. J Clin Oncol, 2016. 34(10): p. 1134-50.

13. Paik, S., et al., A multigene assay to predict recurrence of tamoxifen-treated, node-negative breast cancer. N Engl J Med, 2004. 351(27): p. 2817-26.

14. Senkus, E., et al., Primary breast cancer: ESMO Clinical Practice Guidelines for diagnosis, treatment and follow-up. Ann Oncol, 2015. 26 Suppl 5: p. v8-30.

15. Sorlie, T., et al., Gene expression patterns of breast carcinomas distinguish tumor subclasses with clinical implications. Proc Natl Acad Sci USA, 2001. 98(19): p. 10869-74.

16. Pan, H., et al., 20-Year Risks of Breast-Cancer Recurrence after Stopping Endocrine Therapy at 5 Years. N Engl J Med, 2017. 377(19): p. 1836-1846.

17. Avril, N., et al., Glucose metabolism of breast cancer assessed by 18F-FDG PET: histologic and immunohistochemical tissue analysis. J Nucl Med, 2001. 42(1): p. 9-16.

18. Adejolu, M., et al., False-positive lesions mimicking breast cancer on FDG PET and PET/CT. AJR Am J Roentgenol, 2012. 198(3): p. W304-14.

19. Cecil, K., et al., Metabolic Positron Emission Tomography in Breast Cancer. PET Clin, 2023.

20. Groheux, D. and E. Hindie, Breast cancer: initial workup and staging with FDG PET/CT. Clin Transl Imaging, 2021. 9(3): p. 221-231.

21. Cerami, E., et al., The cBio cancer genomics portal: an open platform for exploring multidimensional cancer genomics data. Cancer Discov, 2012. 2(5): p. 401-4.

22. Gao, J., et al., Integrative analysis of complex cancer genomics and clinical profiles using the cBioPortal. Sci Signal, 2013. 6(269): p. pl1.

23. Ghandi, M., et al., Next-generation characterization of the Cancer Cell Line Encyclopedia. Nature, 2019. 569(7757): p. 503-508.

24. Li, H., et al., The landscape of cancer cell line metabolism. Nat Med, 2019. 25(5): p. 850-860.

25. Joshi, C.J., et al., What are housekeeping genes? PLoS Comput Biol, 2022. 18(7): p. e1010295.

26. Pece, S., et al., Identification and clinical validation of a multigene assay that interrogates the biology of cancer stem cells and predicts metastasis in breast cancer: A retrospective consecutive study. EBioMedicine, 2019. 42: p. 352-362.

27. Pece, S., et al., Comparison of StemPrintER with Oncotype DX Recurrence Score for predicting risk of breast cancer distant recurrence after endocrine therapy. Eur J Cancer, 2022. 164: p. 52-61.

28. Uphoff, C.C. and H.G. Drexler, Comparative PCR analysis for detection of mycoplasma infections in continuous cell lines. In Vitro Cell Dev Biol Anim, 2002. 38(2): p. 79-85.

29. De Mario, A., et al., Identification and functional validation of FDA-approved positive and negative modulators of the mitochondrial calcium uniporter. Cell Rep, 2021. 35(12): p. 109275.

30. Langmead, B., et al., Ultrafast and memory-efficient alignment of short DNA sequences to the human genome. Genome Biol, 2009. 10(3): p. R25.

31. Li, B. and C.N. Dewey, RSEM: accurate transcript quantification from RNA-Seq data with or without a reference genome. BMC Bioinformatics, 2011. 12: p. 323.

32. Curtis, C., et al., The genomic and transcriptomic architecture of 2,000 breast tumours reveals novel subgroups. Nature, 2012. 486(7403): p. 346-52.

33. Pereira, B., et al., The somatic mutation profiles of 2,433 breast cancers refines their genomic and transcriptomic landscapes. Nat Commun, 2016. 7: p. 11479.

34. Abba, M.C., et al., A Molecular Portrait of High-Grade Ductal Carcinoma In Situ. Cancer Res, 2015. 75(18): p. 3980-90.

35. Robinson, M.D., D.J. McCarthy, and G.K. Smyth, edgeR: a Bioconductor package for differential expression analysis of digital gene expression data. Bioinformatics, 2010. 26(1): p. 139-40.

36. Lee, M.I., et al., Prognostic value of SUVmax in breast cancer and comparative analyses of molecular subtypes: A systematic review and meta-analysis. Medicine (Baltimore), 2021. 100(31): p. e26745.

37. Kim, K.H., et al., Evaluating the tumor biology of lung adenocarcinoma: A multimodal analysis. Medicine (Baltimore), 2019. 98(29): p. e16313.

38. Watanabe, R., et al., SUVmax in FDG-PET at the biopsy site correlates with the proliferation potential of tumor cells in non-Hodgkin lymphoma. Leuk Lymphoma, 2010. 51(2): p. 279-83.

39. Li, D., et al., The Correlation between (18)F-FDG PET/CT Imaging SUVmax of Preoperative Colon Cancer Primary Lesions and Clinicopathological Factors. J Oncol, 2021. 2021: p. 4312296.

40. Kitajima, K., et al., Clinical significance of SUVmax in (18)F-FDG PET/CT scan for detecting nodal metastases in patients with oral squamous cell carcinoma. Springerplus, 2015. 4: p. 718.

41. Ciriello, G., et al., Comprehensive Molecular Portraits of Invasive Lobular Breast Cancer. Cell, 2015. 163(2): p. 506-19.

42. Liu, J., et al., An Integrated TCGA Pan-Cancer Clinical Data Resource to Drive High-Quality Survival Outcome Analytics. Cell, 2018. 173(2): p. 400-416 e11.

43. Schiano Lomoriello, I., et al., A self-sustaining endocytic-based loop promotes breast cancer plasticity leading to aggressiveness and pro-metastatic behavior. Nat Commun, 2020. 11(1): p. 3020.

44. Ryser, M.D., et al., Cancer Outcomes in DCIS Patients Without Locoregional Treatment. J Natl Cancer Inst, 2019. 111(9): p. 952-960.

45. Szklarczyk, D., et al., The STRING database in 2023: protein-protein association networks and functional enrichment analyses for any sequenced genome of interest. Nucleic Acids Res, 2023. 51(D1): p. D638-D646.

46. Ben-David, U., et al., Genetic and transcriptional evolution alters cancer cell line drug response. Nature, 2018. 560(7718): p. 325-330.

47. Liu, Y., et al., Multi-omic measurements of heterogeneity in HeLa cells across laboratories. Nat Biotechnol, 2019. 37(3): p. 314-322.

48. Martino, F., et al., Breast cancers as ecosystems: a metabolic perspective. Cell Mol Life Sci, 2023. 80(9): p. 244.

49. Gong, Y., et al., Metabolic-Pathway-Based Subtyping of Triple-Negative Breast Cancer Reveals Potential Therapeutic Targets. Cell Metab, 2021. 33(1): p. 51-64 e9.

50. Yang, H., et al., Extracellular ATP promotes breast cancer chemoresistance via HIF-1alpha signaling. Cell Death Dis, 2022. 13(3): p. 199.

51. Oehler, M.K., et al., Tissue and plasma expression of the angiogenic peptide adrenomedullin in breast cancer. Br J Cancer, 2003. 89(10): p. 1927-33.

52. Zhu, L., et al., Significant prognostic values of aquaporin mRNA expression in breast cancer. Cancer Manag Res, 2019. 11: p. 1503-1515.

53. Ye, L., et al., Overexpression of CDCA7 predicts poor prognosis and induces EZH2-mediated progression of triple-negative breast cancer. Int J Cancer, 2018. 143(10): p. 2602-2613.

54. Sridhar, S., et al., Increased expression of P-cadherin is an indicator of poor prognosis in breast cancer: a systematic review and meta-analysis. Breast Cancer Res Treat, 2020. 179(2): p. 301-313.

55. Wang, L., et al., Investigating CENPW as a Novel Biomarker Correlated With the Development and Poor Prognosis of Breast Carcinoma. Front Genet, 2022. 13: p. 900111.

56. Chen, F., et al., Ceruloplasmin correlates with immune infiltration and serves as a prognostic biomarker in breast cancer. Aging (Albany NY), 2021. 13(16): p. 20438-20467.

57. Chan, N., et al., Influencing the Tumor Microenvironment: A Phase II Study of Copper Depletion Using Tetrathiomolybdate in Patients with Breast Cancer at High Risk for Recurrence and in Preclinical Models of Lung Metastases. Clin Cancer Res, 2017. 23(3): p. 666-676.

58. Zhang, J., et al., Transcriptome-Based Network Analysis Unveils Eight Immune-Related Genes as Molecular Signatures in the Immunomodulatory Subtype of Triple-Negative Breast Cancer. Front Oncol, 2020. 10: p. 1787.

59. de Araujo, R.A., et al., The elusive Luminal B breast cancer and the mysterious chemokines. J Cancer Res Clin Oncol, 2023.

60. Tsutsumi, E., et al., Cxcl10 Chemokine Induces Migration of ING4-Deficient Breast Cancer Cells via a Novel Cross Talk Mechanism between the Cxcr3 and Egfr Receptors. Mol Cell Biol, 2022. 42(2): p. e0038221.

61. Li, L., Y.P. Gan, and H. Peng, RAMP2-AS1 inhibits CXCL11 expression to suppress malignant phenotype of breast cancer by recruiting DNMT1 and DNMT3B. Exp Cell Res, 2022. 416(2): p. 113139.

62. Motyka, J., et al., Plasma Levels of CXC Motif Chemokine 1 (CXCL1) and Chemokine 8 (CXCL8) as Diagnostic Biomarkers in Luminal A and B Breast Cancer. J Clin Med, 2022. 11(22).

63. Hozhabri, H., et al., A comprehensive bioinformatics analysis to identify potential prognostic biomarkers among CC and CXC chemokines in breast cancer. Sci Rep, 2022. 12(1): p. 10374.

64. Wang, R.X., et al., Value of CXCL8-CXCR1/2 axis in neoadjuvant chemotherapy for triple-negative breast cancer patients: a retrospective pilot study. Breast Cancer Res Treat, 2020. 181(3): p. 561-570.

65. Tiainen, L., et al., Low Plasma IL-8 Levels During Chemotherapy Are Predictive of Excellent Long-Term Survival in Metastatic Breast Cancer. Clin Breast Cancer, 2019. 19(4): p. e522-e533.

66. Fang, Q.I., et al., Increased CXCL8 Expression Is Negatively Correlated with the Overall Survival of Patients with ER-Negative Breast Cancer. Anticancer Res, 2017. 37(9): p. 4845-4852.

67. Sainsbury, J.R., et al., Epidermal-growth-factor receptor status as predictor of early recurrence of and death from breast cancer. Lancet, 1987. 1(8547): p. 1398-402.

68. Lehmann, B.D., et al., Identification of human triple-negative breast cancer subtypes and preclinical models for selection of targeted therapies. J Clin Invest, 2011. 121(7): p. 2750-67.

69. Masuda, H., et al., Role of epidermal growth factor receptor in breast cancer. Breast Cancer Res Treat, 2012. 136(2): p. 331-45.

70. Bhargava, R., et al., EGFR gene amplification in breast cancer: correlation with epidermal growth factor receptor mRNA and protein expression and HER-2 status and absence of EGFR-activating mutations. Mod Pathol, 2005. 18(8): p. 1027-33.

71. Song, K.H., et al., GALNT14 promotes lung-specific breast cancer metastasis by modulating self-renewal and interaction with the lung microenvironment. Nat Commun, 2016. 7: p. 13796.

72. Nagelkerke, A., et al., Hypoxic regulation and prognostic value of LAMP3 expression in breast cancer. Cancer, 2011. 117(16): p. 3670-81.

73. Jacquet, E., et al., Aberrant activation of five embryonic stem cell-specific genes robustly predicts a high risk of relapse in breast cancers. BMC Genomics, 2023. 24(1): p. 463.

74. Liu, C., et al., Malic Enzyme 1 Indicates Worse Prognosis in Breast Cancer and Promotes Metastasis by Manipulating Reactive Oxygen Species. Onco Targets Ther, 2020. 13: p. 8735-8747.

75. Liao, R., et al., ME1 promotes basal-like breast cancer progression and associates with poor prognosis. Sci Rep, 2018. 8(1): p. 16743.

76. Yuan, Y., et al., NDUFA4L2 promotes trastuzumab resistance in HER2-positive breast cancer. Ther Adv Med Oncol, 2021. 13: p. 17588359211027836.

77. Suleman, M., et al., PIR promotes tumorigenesis of breast cancer by upregulating cell cycle activator E2F1. Cell Cycle, 2019. 18(21): p. 2914-2927.

78. Choi, B.H., et al., Lineage-specific silencing of PSAT1 induces serine auxotrophy and sensitivity to dietary serine starvation in luminal breast tumors. Cell Rep, 2022. 38(3): p. 110278.

79. Barnabas, G.D., et al., Serine Biosynthesis Is a Metabolic Vulnerability in IDH2-Driven Breast Cancer Progression. Cancer Res, 2021. 81(6): p. 1443-1456.

80. De Marchi, T., et al., Phosphoserine aminotransferase 1 is associated to poor outcome on tamoxifen therapy in recurrent breast cancer. Sci Rep, 2017. 7(1): p. 2099.

81. Wang, X., et al., TIG1 promotes the development and progression of inflammatory breast cancer through activation of Axl kinase. Cancer Res, 2013. 73(21): p. 6516-25.

82. Zhang, Y.L., et al., Chromatin complexes subunit BAP18 promotes triple-negative breast cancer progression through transcriptional activation of oncogene S100A9. Cell Death Dis, 2022. 13(4): p. 408.

83. Li, Z., et al., ESR1 mutant breast cancers show elevated basal cytokeratins and immune activation. Nat Commun, 2022. 13(1): p. 2011.

84. Li, J., et al., S100A9-CXCL12 activation in BRCA1-mutant breast cancer promotes an immunosuppressive microenvironment associated with resistance to immunotherapy. Nat Commun, 2022. 13(1): p. 1481.

85. Goh, J.Y., et al., Chromosome 1q21.3 amplification is a trackable biomarker and actionable target for breast cancer recurrence. Nat Med, 2017. 23(11): p. 1319-1330.

86. Ma, J., et al., SEC61G promotes breast cancer development and metastasis via modulating glycolysis and is transcriptionally regulated by E2F1. Cell Death Dis, 2021. 12(6): p. 550.

87. Oliemuller, E., et al., SOX11 promotes epithelial/mesenchymal hybrid state and alters tropism of invasive breast cancer cells. Elife, 2020. 9.

88. Oliemuller, E., et al., SOX11 promotes invasive growth and ductal carcinoma in situ progression. J Pathol, 2017. 243(2): p. 193-207.

89. Gothlin Eremo, A., et al., Evaluation of SPP1/osteopontin expression as predictor of recurrence in tamoxifen treated breast cancer. Sci Rep, 2020. 10(1): p. 1451.

90. Walaszek, K., et al., Breast cancer risk in premalignant lesions: osteopontin splice variants indicate prognosis. Br J Cancer, 2018. 119(10): p. 1259-1266.

91. Insua-Rodriguez, J., et al., Stress signaling in breast cancer cells induces matrix components that promote chemoresistant metastasis. EMBO Mol Med, 2018. 10(10).

92. Sangaletti, S., et al., Osteopontin shapes immunosuppression in the metastatic niche. Cancer Res, 2014. 74(17): p. 4706-19.

93. Chu, P.Y., et al., Downregulation of ATP binding cassette subfamily a member 10 acts as a prognostic factor associated with immune infiltration in breast cancer. Aging (Albany NY), 2022. 14(5): p. 2252-2267.

94. Jeong, D., et al., ELOVL2: a novel tumor suppressor attenuating tamoxifen resistance in breast cancer. Am J Cancer Res, 2021. 11(6): p. 2568-2589.

95. Bradbury, R., W.G. Jiang, and Y.X. Cui, MDM2 and PSMA Play Inhibitory Roles in Metastatic Breast Cancer Cells Through Regulation of Matrix Metalloproteinases. Anticancer Res, 2016. 36(3): p. 1143-51.

96. Li, H.N., et al., Elevated expression of FREM1 in breast cancer indicates favorable prognosis and high-level immune infiltration status. Cancer Med, 2020. 9(24): p. 9554-9570.

97. Cohen-Dvashi, H., et al., Navigator-3, a modulator of cell migration, may act as a suppressor of breast cancer progression. EMBO Mol Med, 2015. 7(3): p. 299-314.

98. Lou, W., et al., Five miRNAs-mediated PIEZO2 downregulation, accompanied with activation of Hedgehog signaling pathway, predicts poor prognosis of breast cancer. Aging (Albany NY), 2019. 11(9): p. 2628-2652.

99. Di, S., et al., Long non-coding RNA MAFG-AS1 promotes proliferation and metastasis of breast cancer by modulating STC2 pathway. Cell Death Discov, 2022. 8(1): p. 249.

100. Niu, X., et al., Research progress of STC2 in breast cancer. Biophys Rep, 2021. 7(3): p. 185-192.

101. Parris, T.Z., et al., Clinical implications of gene dosage and gene expression patterns in diploid breast carcinoma. Clin Cancer Res, 2010. 16(15): p. 3860-74.

102. Lei, P., et al., Role of Glucose Metabolic Reprogramming in Breast Cancer Progression and Drug Resistance. Cancers (Basel), 2023. 15(13).

103. Abdel-Wahab, A.F., W. Mahmoud, and R.M. Al-Harizy, Targeting glucose metabolism to suppress cancer progression: prospective of anti-glycolytic cancer therapy. Pharmacol Res, 2019. 150: p. 104511.

104. Pich-Bavastro, C., et al., Activin A-mediated polarization of cancer-associated fibroblasts and macrophages confers resistance to checkpoint immunotherapy in skin cancer. Clin Cancer Res, 2023.

105. Li, B., et al., Comprehensive analyses of tumor immunity: implications for cancer immunotherapy. Genome Biol, 2016. 17(1): p. 174.

106. Li, T., et al., TIMER: A Web Server for Comprehensive Analysis of Tumor-Infiltrating Immune Cells. Cancer Res, 2017. 77(21): p. e108-e110.

107. Racle, J., et al., Simultaneous enumeration of cancer and immune cell types from bulk tumor gene expression data. Elife, 2017. 6.

108. Finotello, F., et al., Molecular and pharmacological modulators of the tumor immune contexture revealed by deconvolution of RNA-seq data. Genome Med, 2019. 11(1): p. 34.

109. Chen, B., et al., Profiling Tumor Infiltrating Immune Cells with CIBERSORT. Methods Mol Biol, 2018. 1711: p. 243-259.

## Claims

1. An *in vitro* method of screening a subject suffering from breast cancer for predicting prognosis of the disease comprising at least the step a) of determining the expression level of at least one gene in an isolated biological sample from said subject, wherein said gene is selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4, preferably the expression level of all said genes or of at least all said genes is determined.

2. The method according to claim 1 wherein said method comprises determining the expression level of at least three genes selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4.

3. The method according to claim 1 or 2 wherein said at least one gene or said at least three genes comprise any of:

   - CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, and CHI3L1;
   - ADAMDEC1, ADM, AQP9, C5orf46, CDCA7, CDH3, CENPW, CP, CSF2RB, CXCL10, CXCL11, CXCL8, EGFR, EREG, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, and TMEM158;
   - MCM10, SOX11, AQP9, CDCA7, S100A9, CXCL8, EGFR, PSAT1, ADM, CKB, CP, CXCL10, MFSD2A, CENPW, SPP1, ADAMDEC1, PIR, PLCH1, FOLH1, CXCL11, FYB2, WNK4, KRT14, ABCA10, IL33, PI15, ELOVL2, NOVA1, CCDC85A, NAV3, NTRK2, CYP2A7, PIEZO2, STC2, ERICH3, and TMEM26;
   - CSF2RB, AQP9, S100A9, ADM, CXCL11, CXCL8, IFI27, CXCL10, IL33, CP, PIR, STC2, SPP1, EGFR, FOLH1, SLITRK5, NTRK2, SOX11, CDH3, PGR, KRT14, and SEC61G;
   - EGFR, CXCL8 and CP; or
   - MCM10, SOX1 and AQP9.

4. The method according to claim 1 or 2 wherein said at least one gene or said at least three genes consist of any of:

   - CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, and CHI3L1;
   - ADAMDEC1, ADM, AQP9, C5orf46, CDCA7, CDH3, CENPW, CP, CSF2RB, CXCL10, CXCL11, CXCL8, EGFR, EREG, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, and TMEM158;
   - MCM10, SOX11, AQP9, CDCA7, S100A9, CXCL8, EGFR, PSAT1, ADM, CKB, CP, CXCL10, MFSD2A, CENPW, SPP1, ADAMDEC1, PIR, PLCH1, FOLH1, CXCL11, FYB2, WNK4, KRT14, ABCA10, IL33, PI15, ELOVL2, NOVA1, CCDC85A, NAV3, NTRK2, CYP2A7, PIEZO2, STC2, ERICH3, and TMEM26;
   - CSF2RB, AQP9, S100A9, ADM, CXCL11, CXCL8, IFI27, CXCL10, IL33, CP, PIR, STC2, SPP1, EGFR, FOLH1, SLITRK5, NTRK2, SOX11, CDH3, PGR, KRT14, and SEC61G;
   - EGFR, CXCL8 and CP; or
   - MCM10, SOX1 and AQP9.

5. The method according to any one of the preceding claims wherein it further comprises a step b) wherein based on the expression of said at least one gene or of said at least three genes a risk score is calculated, preferably said risk score being calculated according to the following formula:

$$\text{Risk score} = \Sigma i \, (\beta i * Cq_{normalized}),$$

wherein i is the summation index for the at least one or at least three genes; $\beta$ is the ridge penalized Cox model coefficient for each gene; and $Cq_{normalized}$ is the normalized average level of expression for each gene.

6. The method according to claim 5 further comprising a step c) of determining a prognosis by means of said risk-score calculation, preferably determining a prognosis includes classifying said subject in one of at least two classes corresponding to levels of progression of the disease, such as better prognosis, intermediate prognosis and worse prognosis.

7. The method according to claim 6 wherein determining a prognosis comprises stratifying the subject into a worse prognosis class if said calculated risk score is greater than a predetermined cut-off value or into a better prognosis class if the calculated risk score is lower than said predetermined cut-off value, or wherein determining a prognosis comprises stratifying the subject into a worse prognosis class if said calculated risk score is greater than a first predetermined cut-off value or into an intermediate prognosis class if the calculated risk score is lower than said first predetermined cut-off value but higher than a second predetermined cut-off value or into a better prognosis class if said calculated risk score is lower than said second predetermined cut-off value, wherein said first cut-off value is higher than said second cut-off value.

8. The method according to anyone of the preceding claims wherein said biological sample is a tumoral tissue sample.

9. The method according to any one of the preceding claims wherein said breast cancer is selected from Luminal and Luminal-HER2 breast cancer.

10. The method according to any one of the preceding claims wherein determining the gene expression in the sample comprises determining the amount of respective RNA transcript, mRNA or protein translation products in the sample.

11. A kit for performing the method of anyone of claims 1-10, said kit comprising at least means for determining in an isolated biological sample from a subject the expression level of at least one gene or of at least three genes as defined in any one of claims 1-4, said kit preferably further comprising means to calculate a risk score and optionally means for providing prognostic information.

12. The method according to any one of claims 1-10 or the kit according to claim 11 which is used in combination with a further prognostic method useful in the prognosis and/or in the classification of a subject affected by breast cancer.

13. A computer program comprising instructions which, when the program is executed by a computer, cause the computer to carry out the method of anyone of claims 1-10.

14. A data processing device comprising means adapted for carrying out the method of anyone of claims 1-10, said device preferably comprising:

   i) means for receiving data representing the expression level of at least one gene as defined in any one of claims 1-4 in an isolated biological sample from a subject;
   ii) means for calculating a risk score; and
   iii) means for providing a prognosis of the disease based on said risk score.

15. A method for determining a breast cancer treatment for a subject affected by breast cancer comprising the steps of a) determining the expression level of at least one gene in an isolated biological sample from said subject, wherein said gene is selected from the group consisting of ADAMDEC1, ADM, AQP9, C5orf46, CDCA7, CDH3, CENPW, CP, CSF2RB, CXCL10, CXCL11, CXCL8, EGFR, GABRE, GALNT14, IFI27, LAMP3, MCM10, ME1, MFSD2A, NDUFA4L2, PIR, PLCH1, PSAT1, RARRES1, S100A9, SEC61G, SHISA2, SLITRK5, SOX11, SPP1, TMEM158, ABCA10, CCDC85A, CKB, CYP2A7, ELOVL2, ERICH3, FOLH1, FREM1, FYB2, IL33, KRT14, MACROD2, NAV3, NOVA1, NTRK2, PGR, PI15, PIEZO2, SORCS1, STC2, TMEM26 and WNK4; b) calculating a risk score based on the expression level of said at least one gene; c) determining a prognosis of said subject based on said risk score and d) determining a breast cancer treatment for said subject based on said prognosis, preferably said breast cancer treatment comprising surgery, radiation and/or an anti-cancer or chemotherapeutic agent, said anti-cancer or chemotherapeutic agent preferably being selected from anthracycline agents, alkylating agents, nucleoside analogs, platinum agents, vinca agents, anti-estrogen drugs, aromatase inhibitors, ovarian suppression agents, endocrine/-hormonal agents, bisphophonate therapy agents and targeted biological therapy agents (e.g., antibodies), cyclophosphamide, fluorouracil (or 5-fluorouracil or 5-FU), methotrexate, thiotepa, carboplatin, cisplatin, gemcitabine, anthracycline, taxanes, paclitaxel, protein-bound paclitaxel, doxorubicin, docetaxel, vinorelbine, tamoxifen, raloxifene, toremifene, fulvestrant, irinotecan, ixabepilone, temozolmide, topotecan, vincristine, vinblastine, eribulin, mutamycin, capecitabine, capecitabine, anastrozole, exemestane, letrozole, leuprolide, abarelix, buserlin, goserelin, megestrol acetate, risedronate, pamidronate, ibandronate, alendronate, denosumab, zoledronate, trastuzumab, tykerb, bevacizumab, inhibitors of EGFR, inhibitors of CXCL8, inhibitors of CXCR1, inhibitors of CXCR2, inhibitors of protein kinases, immunotherapy, immune checkpoint inhibitors, and combinations thereof.

Figure 1

Figure 2

EP 4 621 073 A1

Figure 3

42

**A** Tumors (TCGA dataset)

CXCL8, EREG, CXCL10, CXCL11, CXCL13, EGFR, SEC61G, LAMP3, B3GNT5, CLDN1, CLDN16, RARRES1, CP, PLCH1, SPTSSB, MCM10, S100A9, CHI3L1

In A, C-F
▌ Amplified
▌ Not-amplified

**B**

|  | Amplified | | Not amplified | | Chi |
|---|---|---|---|---|---|
| **TCGA** | N | % | N | % | **Square** |
| SUV-L | 74 | 15.9 | 392 | 84.1 | <0.0001 |
| SUV-H | 161 | 39.0 | 252 | 61.0 |  |
|  |  |  |  |  |  |
| **METABRIC** | N | % | N | % |  |
| SUV-L | 319 | 32.2 | 672 | 67.8 | <0.0001 |
| SUV-H | 406 | 44.5 | 506 | 55.5 |  |

Tumors (TCGA dataset)

**C** 4q13.3 | CXCL8, EREG
4q21.1 | CXCL10, CXCL11, CXCL13

**D** 7p11.2 | EGFR, SEC61G

**E** 3q27.1 | LAMP3, B3GNT5
3q28 | CLDN1, CLDN16
3q25.32 RARRES1
3q24-q25.1 CP
3q25.31 PLCH1
3q26.1 SPTSSB

**F** 10p.13 MCM10
1q21.3 S100A9
1q32.1 CHI3L1

Figure 4

Figure 5

**A**

CSF2RB AQP9
S100A9
ADM
CXCL11 **CXCL8**
IFI27
CXCL10 IL33
CP
PIR STC2
SPP1

PGR
CDH3 KRT14
SOX11
SEC61G
NTRK2
**EGFR** SLITRK5
FOLH1

● **UP**
○ **DOWN**

**B**

| UP in SUV-H-like BC cell lines | LOW in SUV-H-like BC cell lines |
|---|---|
| 1-methylnicotinamide | Sucrose |
| GABA | Oleylcarnitine |
| N-carbamoyl-beta-alanine | Stearoylcarnitine |
| Kynurenine | Acetylglycine |
| Inositol | Carnitine |
| UDP-galactose/UDP-glucose | AMP |
| DHAP/glyceraldehyde 3P | Myristoylcarnitine |
| Beta-alanine | Palmitoylcarnitine |
| Lactate | GMP |
| Taurocholate | Butyrobetaine |
| Glutamate | Propionylcarnitine |
| Malondialdehyde | Acetylcarnitine |
| 2-aminoadipate | Alpha-glycerophosphate |
| Isoleucine | Malonylcarnitine |
| Sorbitol | 2-deoxycytidine |
| Leucine | Lauroylcarnitine |
| 2-hydroxyglutarate | Lactose |
| Hexoses (HILIC neg) | Butyrylcarnitine/iso |
| | Hexanoylcarnitine |

**C**

2-Hydroxy-glutarate    p = 0.04
Glutamate    p = 0.01
Lactate    p = 0.01
AcCar/Car    p = 0.005

SUV-H-like BC cell lines    SUV-L-like BC cell lines

**D**

BT20, CAL120, CAL51, CAL851, MDAMB231, HCC1806, JIMT1, HCC70, HCC1954, HDQP1, BT549, HS578T, MDAMB157, HCC1395, MDAMB468, HCC1143, **HCC38**, HCC1937, **CAL148**, **MDAMB134VI**, UACC893, DU4475, HCC1187, **HCC1599**, HCC1569, HMC18, **EFM19**, **EFM192A**, AU565, SKBR3, ZR7530, **MDAMB453**, **MDAMB361**, HCC202, **MDAMB175VII**, **BT483**, **UACC812**, **HCC1419**, **HCC1500**, BT474, **CAMA1**, ZR751, **KPL1**, MCF7, **HCC1428**, T47D, **MDAMB415**, **HCC2218**

Metabolites

-0.45
-0.36
-0.27
-0.18
-0.09
0.0002
0.1199
0.2397
0.3595
0.4793
0.5991

Figure 6

Figure 7

Figure 8

Figure 9

Figure 10

| | Europäisches Patentamt European Patent Office Office européen des brevets | **EUROPEAN SEARCH REPORT** | **Application Number** EP 24 16 5583 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEE MOON IL ET AL: "Prognostic value of SUVmax in breast cancer and comparative analyses of molecular subtypes : A systematic review and meta-analysis", MEDICINE, vol. 100, no. 31, 6 August 2021 (2021-08-06), page e26745, XP093192988, US ISSN: 0025-7974, DOI: 10.1097/MD.0000000000026745 * the whole document * | 1-10,15 | INV. C12Q1/6886 |
| A | AKTAS AYSEGUL ET AL: "Correlations of Primary Tumor SUVmax and Axillary Lymph Node SUVmax with Molecular Subtypes of Invasive Breast Cancer", INDIAN JOURNAL OF SURGERY, SPRINGER-VERLAG, INDIA, vol. 83, no. Suppl 2, 8 February 2021 (2021-02-08), pages 468-474, XP037649536, ISSN: 0972-2068, DOI: 10.1007/S12262-021-02770-W [retrieved on 2021-02-08] * the whole document * | 1-10,15 | |

**TECHNICAL FIELDS SEARCHED (IPC)**

C12Q

-/--

The present search report has been drawn up for all claims

| Place of search | Date of completion of the search | Examiner |
|---|---|---|
| The Hague | 7 August 2024 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

page 1 of 3

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GROHEUX DAVID ET AL: "18FDG-PET/CT for predicting the outcome in ER+/HER2- breast cancer patients: comparison of clinicopathological parameters and PET image-derived indices including tumor texture analysis", BREAST CANCER RESEARCH (ONLINE EDITION), vol. 19, no. 1, 5 January 2017 (2017-01-05), XP093193014, United Kingdom, Netherlands, United States ISSN: 1465-542X, DOI: 10.1186/s13058-016-0793-2 Retrieved from the Internet: URL:http://link.springer.com/content/pdf/10.1186/s13058-016-0793-2.pdf> * the whole document * | 1-10,15 | |
| X | MENG JIN ET AL: "Prognostic value of metabolic signature on 18F-FDG uptake in breast cancer patients after radiotherapy", MOLECULAR THERAPY - ONCOLYTICS, vol. 23, 1 December 2021 (2021-12-01), pages 412-419, XP093193030, ISSN: 2372-7705, DOI: 10.1016/j.omto.2021.10.008 * the whole document * & Meng Jing ET AL: "Prognostic value of metabolic signature on 18F-FDG uptake in breast cancer patients after radiotherapy - Supplemental Information", MOLECULAR THERAPY - ONCOLYTICS, 1 December 2021 (2021-12-01), pages 1-18, XP093193060, Retrieved from the Internet: URL:https://www.sciencedirect.com/science/article/pii/S2372770521001455?via%3Dihub | 1 | |

TECHNICAL FIELDS SEARCHED (IPC)

-/--

The present search report has been drawn up for all claims

| Place of search | Date of completion of the search | Examiner |
|---|---|---|
| The Hague | 7 August 2024 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 16 5583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/415434 A1 (PEROU CHARLES [US] ET AL) 29 December 2022 (2022-12-29) * the whole document * | 1-4, 6-10,15 | |
| A | MARTÍNEZ-ORDOÑEZ ANXO ET AL: "POU1F1 transcription factor induces metabolic reprogramming and breast cancer progression via LDHA regulation", ONCOGENE, NATURE PUBLISHING GROUP UK, LONDON, vol. 40, no. 15, 13 March 2021 (2021-03-13), pages 2725-2740, XP037425008, ISSN: 0950-9232, DOI: 10.1038/S41388-021-01740-6 [retrieved on 2021-03-13] * the whole document * | 1-10,15 | |
| A | PONNUSAMY LAVANYA ET AL: "Distinctive role of SIK1 and SIK3 isoforms in aerobic glycolysis and cell growth of breast cancer through the regulation of p53 and mTOR signaling pathways", BIOCHIMICA ET BIOPHYSICA ACTA, ELSEVIER SCIENCE PUBLISHERS, AMSTERDAM, NL, vol. 1868, no. 5, 2 February 2021 (2021-02-02), XP086513660, ISSN: 0167-4889, DOI: 10.1016/J.BBAMCR.2021.118975 [retrieved on 2021-02-02] * the whole document * | 1-10,15 | TECHNICAL FIELDS SEARCHED (IPC) |

The present search report has been drawn up for all claims

| Place of search | Date of completion of the search | Examiner |
|---|---|---|
| The Hague | 7 August 2024 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 24 16 5583

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2024

| Patent document cited in search report | Publication date | Patent family member(s) | Publication date |
|---|---|---|---|
| US 2022415434 A1 | 29-12-2022 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017220492 A
- WO 2005039382 A
- WO 2009158143 A
- WO 02103320 A

**Non-patent literature cited in the description**

- JMP. SAS Institute Inc, 1989
- **HANAHAN, D**. Hallmarks of Cancer: New Dimensions. *Cancer Discov*, 2022, vol. 12 (1), 31-46
- **DEBERARDINIS, R.J** ; **N.S. CHANDEL**. We need to talk about the Warburg effect.. *Nat Metab*, 2020, vol. 2 (2), 127-129
- **WARBURG, O.** Versuche an überlebendem carcinom-gewebe (Methoden). *Biochemische Zeitschrift*, 1923, vol. 142, 317-333
- **KOPPENOL, W.H.** ; **P.L. BOUNDS** ; **C.V. DANG**. Otto Warburg's contributions to current concepts of cancer metabolism.. *Nat Rev Cancer*, 2011, vol. 11 (5), 325-37
- **DEBERARDINIS, R.J.** ; **N.S. CHANDEL**. Fundamentals of cancer metabolism.. *Sci Adv*, 2016, vol. 2 (5), e1600200
- **BOELLAARD, R.** Standards for PET image acquisition and quantitative data analysis.. *J Nucl Med*, 2009, vol. 50 (1), 11S-20S
- **SUNG, H. et al.** Global Cancer Statistics 2020: GLOBOCAN Estimates of Incidence and Mortality Worldwide for 36 Cancers in 185 Countries.. *CA Cancer J Clin*, 2021, vol. 71 (3), 209-249
- **ALLISON, K.H.** Molecular pathology of breast cancer: what a pathologist needs to know.. *Am J Clin Pathol*, 2012, vol. 138 (6), 770-80
- Cancer Genome Atlas, N., Comprehensive molecular portraits of human breast tumours.. *Nature*, 2012, vol. 490 (7418), 61-70
- **BURSTEIN, H.J. et al.** Customizing local and systemic therapies for women with early breast cancer: the St. Gallen International Consensus Guidelines for treatment of early breast cancer 2021.. *Ann Oncol*, 2021, vol. 32 (10), 1216-1235
- **COATES, A.S. et al.** Tailoring therapies--improving the management of early breast cancer: St Gallen International Expert Consensus on the Primary Therapy of Early Breast Cancer 2015. *Ann Oncol*, 2015, vol. 26 (8), 1533-46
- **HARRIS, L.N. et al.** Use of Biomarkers to Guide Decisions on Adjuvant Systemic Therapy for Women With Early-Stage Invasive Breast Cancer: American Society of Clinical Oncology Clinical Practice Guideline.. *J Clin Oncol*, 2016, vol. 34 (10), 1134-50
- **PAIK, S. et al.** A multigene assay to predict recurrence of tamoxifen-treated, node-negative breast cancer.. *N Engl J Med*, 2004, vol. 351 (27), 2817-26
- **SENKUS, E. et al.** Primary breast cancer: ESMO Clinical Practice Guidelines for diagnosis, treatment and follow-up.. *Ann Oncol*, 2015, vol. 26 (5), v8-30
- **SORLIE, T. et al.** Gene expression patterns of breast carcinomas distinguish tumor subclasses with clinical implications.. *Proc Natl Acad Sci USA*, 2001, vol. 98 (19), 10869-74
- **PAN, H. et al.** 20-Year Risks of Breast-Cancer Recurrence after Stopping Endocrine Therapy at 5 Years.. *N Engl J Med*, 2017, vol. 377 (19), 1836-1846
- **AVRIL, N. et al.** Glucose metabolism of breast cancer assessed by 18F-FDG PET: histologic and immunohistochemical tissue analysis.. *J Nucl Med*, 2001, vol. 42 (1), 9-16
- **ADEJOLU, M. et al.** False-positive lesions mimicking breast cancer on FDG PET and PET/CT.. *AJR Am J Roentgenol*, 2012, vol. 198 (3), W304-14
- **CECIL, K. et al.** Metabolic Positron Emission Tomography in Breast Cancer.. *PET Clin*, 2023
- **GROHEUX, D.** ; **E. HINDIE**. Breast cancer: initial workup and staging with FDG PET/CT.. *Clin Transl Imaging*, 2021, vol. 9 (3), 221-231
- **CERAMI, E. et al.** The cBio cancer genomics portal: an open platform for exploring multidimensional cancer genomics data.. *Cancer Discov*, 2012, vol. 2 (5), 401-4
- **GAO, J. et al.** Integrative analysis of complex cancer genomics and clinical profiles using the cBioPortal.. *Sci Signal*, 2013, vol. 6 (269), 1

- **GHANDI, M. et al.** Next-generation characterization of the Cancer Cell Line Encyclopedia.. *Nature*, 2019, vol. 569 (7757), 503-508
- **LI, H. et al.** The landscape of cancer cell line metabolism.. *Nat Med*, 2019, vol. 25 (5), 850-860
- **JOSHI, C.J. et al.** What are housekeeping genes?. *PLoS Comput Biol*, 2022, vol. 18 (7), e1010295
- **PECE, S. et al.** Identification and clinical validation of a multigene assay that interrogates the biology of cancer stem cells and predicts metastasis in breast cancer: A retrospective consecutive study.. *EBioMedicine*, 2019, vol. 42, 352-362
- **PECE, S. et al.** Comparison of StemPrintER with Oncotype DX Recurrence Score for predicting risk of breast cancer distant recurrence after endocrine therapy.. *Eur J Cancer*, 2022, vol. 164, 52-61
- **UPHOFF, C.C.** ; **H.G. DREXLER**. Comparative PCR analysis for detection of mycoplasma infections in continuous cell lines.. *Cell Dev Biol Anim*, 2002, vol. 38 (2), 79-85
- **DE MARIO, A. et al.** Identification and functional validation of FDA-approved positive and negative modulators of the mitochondrial calcium uniporter.. *Cell Rep*, 2021, vol. 35 (12), 109275
- **LANGMEAD, B. et al.** Ultrafast and memory-efficient alignment of short DNA sequences to the human genome.. *Genome Biol*, 2009, vol. 10 (3), R25
- **LI, B.** ; **C.N. DEWEY**. RSEM: accurate transcript quantification from RNA-Seq data with or without a reference genome.. *BMC Bioinformatics*, 2011, vol. 12, 323
- **CURTIS, C. et al.** The genomic and transcriptomic architecture of 2,000 breast tumours reveals novel subgroups.. *Nature*, 2012, vol. 486 (7403), 346-52
- **PEREIRA, B. et al.** The somatic mutation profiles of 2,433 breast cancers refines their genomic and transcriptomic landscapes.. *Nat Commun*, 2016, vol. 7, 11479
- **ABBA, M.C. et al.** A Molecular Portrait of High-Grade Ductal Carcinoma In Situ.. *Cancer Res*, 2015, vol. 75 (18), 3980-90
- **ROBINSON, M.D.** ; **D.J. MCCARTHY** ; **G.K. SMYTH**. edgeR: a Bioconductor package for differential expression analysis of digital gene expression data.. *Bioinformatics*, 2010, vol. 26 (1), 139-40
- **LEE, M.I. et al.** Prognostic value of SUVmax in breast cancer and comparative analyses of molecular subtypes: A systematic review and meta-analysis.. *Medicine*, 2021, vol. 100 (31), e26745
- **KIM, K.H. et al.** Evaluating the tumor biology of lung adenocarcinoma: A multimodal analysis.. *Medicine*, 2019, vol. 98 (29), e16313
- **WATANABE, R. et al.** SUVmax in FDG-PET at the biopsy site correlates with the proliferation potential of tumor cells in non-Hodgkin lymphoma.. *Leuk Lymphoma*, 2010, vol. 51 (2), 279-83
- **LI, D. et al.** The Correlation between (18)F-FDG PET/CT Imaging SUVmax of Preoperative Colon Cancer Primary Lesions and Clinicopathological Factors.. *J Oncol*, 2021, vol. 2021, 4312296
- **KITAJIMA, K. et al.** Clinical significance of SUVmax in (18)F-FDG PET/CT scan for detecting nodal metastases in patients with oral squamous cell carcinoma.. *Springerplus*, 2015, vol. 4, 718
- **CIRIELLO, G. et al.** Comprehensive Molecular Portraits of Invasive Lobular Breast Cancer.. *Cell*, 2015, vol. 163 (2), 506-19
- **LIU, J. et al.** An Integrated TCGA Pan-Cancer Clinical Data Resource to Drive High-Quality Survival Outcome Analytics.. *Cell*, 2018, vol. 173 (2), 400-416, e11
- **SCHIANO LOMORIELLO, I. et al.** A self-sustaining endocytic-based loop promotes breast cancer plasticity leading to aggressiveness and pro-metastatic behavior.. *Nat Commun*, 2020, vol. 11 (1), 3020
- **RYSER, M.D. et al.** Cancer Outcomes in DCIS Patients Without Locoregional Treatment.. *J Natl Cancer Inst*, 2019, vol. 111 (9), 952-960
- **SZKLARCZYK, D. et al.** The STRING database in 2023: protein-protein association networks and functional enrichment analyses for any sequenced genome of interest.. *Nucleic Acids Res*, 2023, vol. 51 (D1), D638-D646
- **BEN-DAVID, U. et al.** Genetic and transcriptional evolution alters cancer cell line drug response.. *Nature*, 2018, vol. 560 (7718), 325-330
- **LIU, Y. et al.** Multi-omic measurements of heterogeneity in HeLa cells across laboratories.. *Nat Biotechnol*, 2019, vol. 37 (3), 314-322
- **MARTINO, F. et al.** Breast cancers as ecosystems: a metabolic perspective.. *Cell Mol Life Sci*, 2023, vol. 80 (9), 244
- **GONG, Y. et al.** Metabolic-Pathway-Based Subtyping of Triple-Negative Breast Cancer Reveals Potential Therapeutic Targets.. *Cell Metab*, 2021, vol. 33 (1), 51-64
- **YANG, H. et al.** ExtracellularATP promotes breast cancer chemoresistance via HIF-1alpha signaling.. *Cell Death Dis*, 2022, vol. 13 (3), 199
- **OEHLER, M.K. et al.** Tissue and plasma expression of the angiogenic peptide adrenomedullin in breast cancer.. *Br J Cancer*, 2003, vol. 89 (10), 1927-33
- **ZHU, L. et al.** Significant prognostic values of aquaporin mRNA expression in breast cancer.. *Cancer Manag Res*, 2019, vol. 11, 1503-1515

- **YE, L. et al.** Overexpression of CDCA7 predicts poor prognosis and induces EZH2-mediated progression of triple-negative breast cancer.. *Int J Cancer*, 2018, vol. 143 (10), 2602-2613
- **SRIDHAR, S. et al.** Increased expression of P-cadherin is an indicator of poor prognosis in breast cancer: a systematic review and meta-analysis.. *Breast Cancer Res Treat*, 2020, vol. 179 (2), 301-313
- **WANG, L. et al.** Investigating CENPW as a Novel Biomarker Correlated With the Development and Poor Prognosis of Breast Carcinoma.. *Front Genet*, 2022, vol. 13, 900111
- **CHEN, F. et al.** Ceruloplasmin correlates with immune infiltration and serves as a prognostic biomarker in breast cancer.. *Aging*, 2021, vol. 13 (16), 20438-20467
- **CHAN, N. et al.** Influencing the Tumor Microenvironment: A Phase II Study of Copper Depletion Using Tetrathiomolybdate in Patients with Breast Cancer at High Risk for Recurrence and in Preclinical Models of Lung Metastases.. *Clin Cancer Res*, 2017, vol. 23 (3), 666-676
- **ZHANG, J. et al.** Transcriptome-Based Network Analysis Unveils Eight Immune-Related Genes as Molecular Signatures in the Immunomodulatory Subtype of Triple-Negative Breast Cancer.. *Front Oncol*, 2020, vol. 10, 1787
- **DE ARAUJO, R.A. et al.** The elusive Luminal B breast cancer and the mysterious chemokines.. *J Cancer Res Clin Oncol*, 2023
- **TSUTSUMI, E. et al.** Cxcl10 Chemokine Induces Migration of ING4-Deficient Breast Cancer Cells via a Novel Cross Talk Mechanism between the Cxcr3 and Egfr Receptors.. *Mol Cell Biol*, 2022, vol. 42 (2), e0038221
- **LI, L.** ; **Y.P. GAN** ; **H. PENG**. RAMP2-AS1 inhibits CXCL11 expression to suppress malignant phenotype of breast cancer by recruiting DNMT1 and DNMT3B.. *Exp Cell Res*, 2022, vol. 416 (2), 113139
- **MOTYKA, J. et al.** Plasma Levels of CXC Motif Chemokine 1 (CXCL1) and Chemokine 8 (CXCL8) as Diagnostic Biomarkers in Luminal A and B Breast Cancer.. *J Clin Med*, 2022, vol. 11 (22)
- **HOZHABRI, H. et al.** A comprehensive bioinformatics analysis to identify potential prognostic biomarkers among CC and CXC chemokines in breast cancer.. *Sci Rep*, 2022, vol. 12 (1), 10374
- **WANG, R.X. et al.** Value of CXCL8-CXCR1/2 axis in neoadjuvant chemotherapy for triple-negative breast cancer patients: a retrospective pilot study.. *Breast Cancer Res Treat*, 2020, vol. 181 (3), 561-570
- **TIAINEN, L. et al.** Low Plasma IL-8 Levels During Chemotherapy Are Predictive of Excellent Long-Term Survival in Metastatic Breast Cancer.. *Clin Breast Cancer*, 2019, vol. 19 (4), e522-e533
- **FANG, Q.I. et al.** Increased CXCL8 Expression Is Negatively Correlated with the Overall Survival of Patients with ER-Negative Breast Cancer.. *Anticancer Res*, 2017, vol. 37 (9), 4845-4852
- **SAINSBURY, J.R. et al.** Epidermal-growth-factor receptor status as predictor of early recurrence of and death from breast cancer.. *Lancet*, 1987, vol. 1 (8547), 1398-402
- **LEHMANN, B.D. et al.** Identification of human triple-negative breast cancer subtypes and preclinical models for selection of targeted therapies.. *J Clin Invest*, 2011, vol. 121 (7), 2750-67
- **MASUDA, H. et al.** Role of epidermal growth factor receptor in breast cancer.. *Breast Cancer Res Treat*, 2012, vol. 136 (2), 331-45
- **BHARGAVA, R. et al.** EGFR gene amplification in breast cancer: correlation with epidermal growth factor receptor mRNA and protein expression and HER-2 status and absence of EGFR-activating mutations.. *Mod Pathol*, 2005, vol. 18 (8), 1027-33
- **SONG, K.H. et al.** GALNT14 promotes lung-specific breast cancer metastasis by modulating self-renewal and interaction with the lung microenvironment.. *Nat Commun*, 2016, vol. 7, 13796
- **NAGELKERKE, A. et al.** Hypoxic regulation and prognostic value of LAMP3 expression in breast cancer.. *Cancer*, 2011, vol. 117 (16), 3670-81
- **JACQUET, E. et al.** Aberrant activation of five embryonic stem cell-specific genes robustly predicts a high risk of relapse in breast cancers.. *BMC Genomics*, 2023, vol. 24 (1), 463
- **LIU, C. et al.** Malic Enzyme 1 Indicates Worse Prognosis in Breast Cancer and Promotes Metastasis by Manipulating Reactive Oxygen Species.. *Onco Targets Ther*, 2020, vol. 13, 8735-8747
- **LIAO, R. et al.** ME1 promotes basal-like breast cancer progression and associates with poor prognosis.. *Sci Rep*, 2018, vol. 8 (1), 16743
- **YUAN, Y. et al.** NDUFA4L2 promotes trastuzumab resistance in HER2-positive breast cancer.. *Ther Adv Med Oncol*, 2021, vol. 13, 17588359211027836
- **SULEMAN, M. et al.** PIR promotes tumorigenesis of breast cancer by upregulating cell cycle activator E2F1.. *Cell Cycle*, 2019, vol. 18 (21), 2914-2927
- **CHOI, B.H. et al.** Lineage-specific silencing of PSAT1 induces serine auxotrophy and sensitivity to dietary serine starvation in luminal breast tumors.. *Cell Rep*, 2022, vol. 38 (3), 110278
- **BARNABAS, G.D. et al.** Serine Biosynthesis Is a Metabolic Vulnerability in IDH2-Driven Breast Cancer Progression.. *Cancer Res*, 2021, vol. 81 (6), 1443-1456

- **DE MARCHI, T. et al.** Phosphoserine aminotransferase 1 is associated to poor outcome on tamoxifen therapy in recurrent breast cancer.. *Sci Rep*, 2017, vol. 7 (1), 2099
- **WANG, X. et al.** TIG1 promotes the development and progression of inflammatory breast cancer through activation of Axl kinase.. *Cancer Res*, 2013, vol. 73 (21), 6516-25
- **ZHANG, Y.L. et al.** Chromatin complexes subunit BAP18 promotes triple-negative breast cancer progression through transcriptional activation of oncogene S100A9.. *Cell Death Dis*, 2022, vol. 13 (4), 408
- **LI, Z. et al.** ESR1 mutant breast cancers show elevated basal cytokeratins and immune activation.. *Nat Commun*, 2022, vol. 13 (1), 2011
- **LI, J. et al.** S100A9-CXCL12 activation in BRCA1-mutant breast cancer promotes an immunosuppressive microenvironment associated with resistance to immunotherapy.. *Nat Commun*, 2022, vol. 13 (1), 1481
- **GOH, J.Y. et al.** Chromosome 1q21.3 amplification is a trackable biomarker and actionable target for breast cancer recurrence.. *Nat Med*, 2017, vol. 23 (11), 1319-1330
- **MA, J. et al.** SEC61G promotes breast cancer development and metastasis via modulating glycolysis and is transcriptionally regulated by E2F1.. *Cell Death Dis*, 2021, vol. 12 (6), 550
- **OLIEMULLER, E. et al.** SOX11 promotes epithelial/mesenchymal hybrid state and alters tropism of invasive breast cancer cells.. *Elife*, 2020, vol. 9
- **OLIEMULLER, E. et al.** SOX11 promotes invasive growth and ductal carcinoma in situ progression.. *J Pathol*, 2017, vol. 243 (2), 193-207
- **GOTHLIN EREMO, A. et al.** Evaluation of SPP1/osteopontin expression as predictor of recurrence in tamoxifen treated breast cancer.. *Sci Rep*, 2020, vol. 10 (1), 1451
- **WALASZEK, K. et al.** Breast cancer risk in premalignant lesions: osteopontin splice variants indicate prognosis.. *Br J Cancer*, 2018, vol. 119 (10), 1259-1266
- **INSUA-RODRIGUEZ, J. et al.** Stress signaling in breast cancer cells induces matrix components that promote chemoresistant metastasis.. *EMBO Mol Med*, 2018, vol. 10 (10)
- **SANGALETTI, S. et al.** Osteopontin shapes immunosuppression in the metastatic niche.. *Cancer Res*, 2014, vol. 74 (17), 4706-19
- **CHU, P.Y. et al.** Downregulation of ATP binding cassette subfamily a member 10 acts as a prognostic factor associated with immune infiltration in breast cancer.. *Aging*, 2022, vol. 14 (5), 2252-2267
- **JEONG, D. et al.** ELOVL2: a novel tumor suppressor attenuating tamoxifen resistance in breast cancer.. *Am J Cancer Res*, 2021, vol. 11 (6), 2568-2589
- **BRADBURY, R. ; W.G. JIANG ; Y.X. CUI.** MDM2 and PSMA Play Inhibitory Roles in Metastatic Breast Cancer Cells Through Regulation of Matrix Metalloproteinases.. *Anticancer Res*, 2016, vol. 36 (3), 1143-51
- **LI, H.N. et al.** Elevated expression of FREM1 in breast cancer indicates favorable prognosis and high-level immune infiltration status.. *Cancer Med*, 2020, vol. 9 (24), 9554-9570
- **COHEN-DVASHI, H. et al.** Navigator-3, a modulator of cell migration, may act as a suppressor of breast cancer progression.. *EMBO Mol Med*, 2015, vol. 7 (3), 299-314
- **LOU, W. et al.** Five miRNAs-mediated PIEZO2 downregulation, accompanied with activation of Hedgehog signaling pathway, predicts poor prognosis of breast cancer.. *Aging*, 2019, vol. 11 (9), 2628-2652
- **DI, S. et al.** Long non-coding RNA MAFG-AS1 promotes proliferation and metastasis of breast cancer by modulating STC2 pathway.. *Cell Death Discov*, 2022, vol. 8 (1), 249
- **NIU, X. et al.** Research progress of STC2 in breast cancer.. *Biophys Rep*, 2021, vol. 7 (3), 185-192
- **PARRIS, T.Z. et al.** Clinical implications of gene dosage and gene expression patterns in diploid breast carcinoma.. *Clin Cancer Res*, 2010, vol. 16 (15), 3860-74
- **LEI, P. et al.** Role of Glucose Metabolic Reprogramming in Breast Cancer Progression and Drug Resistance.. *Cancers*, 2023, vol. 15 (13)
- **ABDEL-WAHAB, A.F. ; W. MAHMOUD ; R.M. AL-HARIZY.** Targeting glucose metabolism to suppress cancer progression: prospective of anti-glycolytic cancer therapy.. *Pharmacol Res*, 2019, vol. 150, 104511
- **PICH-BAVASTRO, C. et al.** Activin A-mediated polarization of cancer-associated fibroblasts and macrophages confers resistance to checkpoint immunotherapy in skin cancer.. *Clin Cancer Res*, 2023
- **LI, B. et al.** Comprehensive analyses of tumor immunity: implications for cancer immunotherapy.. *Genome Biol*, 2016, vol. 17 (1), 174
- **LI, T. et al.** TIMER: A Web Server for Comprehensive Analysis of Tumor-Infiltrating Immune Cells.. *Cancer Res*, 2017, vol. 77 (21), e108-e110
- **RACLE, J. et al.** Simultaneous enumeration of cancer and immune cell types from bulk tumor gene expression data.. *Elife*, 2017, vol. 6
- **FINOTELLO, F. et al.** Molecular and pharmacological modulators of the tumor immune contexture revealed by deconvolution of RNA-seq data.. *Genome Med*, 2019, vol. 11 (1), 34
- **CHEN, B. et al.** Profiling Tumor Infiltrating Immune Cells with CIBERSORT.. *Methods Mol Biol*, 2018, vol. 1711, 243-259